# EUROPEAN PATENT SPECIFICATION

(11) **EP 0 749 444 B1**
(45) Date of publication and mention of the grant of the patent: **16.08.2001**
(21) Application number: 95903119.6
(22) Date of filing: 15.11.1994
(51) Int. Cl.: C07K 14/755, G01N 33/53, A61K 38/37

(54) **HYBRID HUMAN/ANIMAL FACTOR VIII**
HYBRIDER MENSCH/TIER FAKTOR VIII
FACTEUR VIII HYBRIDE HUMAIN/ANIMAL

(30) Priority: 11.03.1994 US 212133
(43) Date of publication of application: 27.12.1996
(73) Proprietor: EMORY UNIVERSITY, Atlanta, GA 30322 (US)
(72) Inventor: LOLLAR, John S., Decatur, GA 30033 (US); RUNGE, Marschall S., Atlanta, GA 30345 (US)
(74) Representative: Fisher, Adrian John
(86) International application number: PCT/US94/13200
(87) International publication number: WO 95/24427

(56) References cited:
- WO-A-93/20093
- WO-A-94/11503
- GENOMICS, vol.16, 1993 pages 374 - 379 B. ELDER ET AL 'Sequence of the murine factor VIII cDNA' cited in the application
- THROMBOSIS AND HAEMOSTASIS, vol.69, 1 March 1993 pages 240 - 246 M. SHIMA ET AL 'A factor VIII neutralizing monoclonal antibody and a human inhibitor alloantibody recognizing epitopes in the C2 domain inhibit factor VIII binding to von Willebtand factor and to phophatidylserine' cited in the application
- CHEMICAL ABSTRACTS, vol. 111, no. 25, 18 December 1989, Columbus, Ohio, US; abstract no. 230240, D. SCANDELLA ET AL 'Localization of epitopes for human factor VIII inhibitor antibodies by immunoblotting and antibody neutralization' page 570 ; & BLOOD, vol.74, no.5, 1989 pages 1618 - 1626
- CHEMICAL ABSTRACTS, vol. 119, no. 25, 20 December 1993, Columbus, Ohio, US; abstract no. 268801, D. SCANDELLA ET AL 'A recombinant factor VIII A2 domain polypeptide qantitatively neutrilizes human inhibitor antibodies that bind A2' page 782 ; & BLOOD, vol.82, no.6, 1993 pages 1767 - 1775
- CHEMICAL ABSTRACTS, vol. 121, no. 19, 7 November 1994, Columbus, Ohio, US; abstract no. 228287, J. GILLES AND J-M. SAINT-REMY 'Healthy subjects produce both anti-factor VIII and specific anti-idiotypic antibodies' page 850 ; & J. CLIN. INVEST., vol.94, no.4, 1994 pages 1496 - 1505

### SEQUENCE LISTING

(1) GENERAL INFORMATION:
   (i) APPLICANT: Emory University
   (ii) TITLE OF INVENTION: Hybrid Human/Animal Factor VIII
   (iii) NUMBER OF SEQUENCES: 12
   (iv) CORRESPONDENCE ADDRESS:
      (A) ADDRESSEE: Kilpatrick & Cody
      (B) STREET: 1100 Peachtree Street, Suite 2800
      (C) CITY: Atlanta
      (D) STATE: Georgia
      (E) COUNTRY: US
      (F) ZIP: 30309
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: PCT
      (B) FILING DATE: 15-NOV-1994
      (C) CLASSIFICATION:
   (viii) ATTORNEY/AGENT INFORMATION:
      (A) NAME: Pabst, Patrea L.
      (B) REGISTRATION NUMBER: 31,284
      (C) REFERENCE/DOCKET NUMBER: EMU106CIP(2)
   (ix) TELECOMMUNICATION INFORMATION:
      (A) TELEPHONE: 404-815-6508
      (B) TELEFAX: 404-815-6555
(2) INFORMATION FOR SEQ ID NO:1:
   (i) SEQUENCE CHARACTERISTICS:
      (A) LENGTH: 9009 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapien
      (F) TISSUE TYPE: Liver
   (ix) FEATURE:
      (A) NAME/KEY: misc_feature (Domain Structure)
      (B) LOCATION: 5125 ... 7053
      (D) OTHER INFORMATION: /note= "Equivalent to the A3-C1-C2 domain"
   (ix) FEATURE:
      (A) NAME/KEY: misc_feature (Domain Structure)
      (B) LOCATION: 1 ... 2277
      (D) OTHER INFORMATION: /note= "Equivalent to the A1-A2 domain."
   (ix) FEATURE:
      (A) NAME/KEY: Domain
      (B) LOCATION: 1..2277
      (D) OTHER INFORMATION: /note= "cDNA encoding human factor VIII."
   (xi) SEQUENCE DESCRIPTION: SEQ ID NO:1:
   CAGTGGGTAA GTTCCTTAAA TGCTCTGCAA AGAAATTGGG ACTTTTCATT AAATCAGAAA 60
   TTTTACTTTT TTCCCCTCCT GGGAGCTAAA GATATTTTAG AGAAGAATTA ACCTTTTGCT 120
   TCTCCAGTTG AACATTTGTA GCAATAAGTC ATGCAAATAG AGCTCTCCAC CTGCTTCTTT 180
   CTGTGCCTTT TGCGATTCTG CTTTAGTGCC ACCAGAAGAT ACTACCTGGG TGCAGTGGAA 240
   CTGTCATGGG ACTATATGCA AAGTGATCTC GGTGAGCTGC CTGTGGACGC AAGATTTCCT 300
   CCTAGAGTGC CAAAATCTTT TCCATTCAAC ACCTCAGTCG TGTACAAAAA GACTCTGTTT 360
   GTAGAATTCA CGGTTCACCT TTTCAACATC GCTAAGCCAA GGCCACCCTG GATGGGTCTG 420
   CTAGGTCCTA CCATCCAGGC TGAGGTTTAT GATACAGTGG TCATTACACT TAAGAACATG 480
   GCTTCCCATC CTGTCAGTCT TCATGCTGTT GGTGTATCCT ACTGGAAAGC TTCTGAGGGA 540
   GCTGAATATG ATGATCAGAC CAGTCAAAGG GAGAAAGAAG ATGATAAAGT CTTCCCTGGT 600
   GGAAGCCATA CATATGTCTG GCAGGTCCTG AAAGAGAATG GTCCAATGGC CTCTGACCCA 660
   CTGTGCCTTA CCTACTCATA TCTTTCTCAT GTGGACCTGG TAAAAGACTT GAATTCAGGC 720
   CTCATTGGAG CCCTACTAGT ATGTAGAGAA GGGAGTCTGG CCAAGGAAAA GACACAGACC 780
   TTGCACAAAT TTATACTACT TTTTGCTGTA TTTGATGAAG GGAAAAGTTG GCACTCAGAA 840
   ACAAAGAACT CCTTGATGCA GGATAGGGAT GCTGCATCTG CTCGGGCCTG GCCTAAAATG 900
   CACACAGTCA ATGGTTATGT AAACAGGTCT CTGCCAGGTC TGATTGGATG CCACAGGAAA   960
   TCAGTCTATT GGCATGTGAT TGGAATGGGC ACCACTCCTG AAGTGCACTC AATATTCCTC  1020
   GAAGGTCACA CATTTCTTGT GAGGAACCAT CGCCAGGCGT CCTTGGAAAT CTCGCCAATA  1080
   ACTTTCCTTA CTGCTCAAAC ACTCTTGATG GACCTTGGAC AGTTTCTACT GTTTTGTCAT  1140
   ATCTCTTCCC ACCAACATGA TGGCATGGAA GCTTATGTCA AAGTAGACAG CTGTCCAGAG  1200
   GAACCCCAAC TACGAATGAA AAATAATGAA GAAGCGGAAG ACTATGATGA TGATCTTACT  1260
   GATTCTGAAA TGGATGTGGT CAGGTTTGAT GATGACAACT CTCCTTCCTT TATCCAAATT  1320
   CGCTCAGTTG CCAAGAAGCA TCCTAAAACT TGGGTACATT ACATTGCTGC TGAAGAGGAG  1380
   GACTGGGACT ATGCTCCCTT AGTCCTCGCC CCCGATGACA GAAGTTATAA AAGTCAATAT  1440
   TTGAACAATG GCCCTCAGCG GATTGGTAGG AAGTACAAAA AAGTCCGATT TATGGCATAC  1500
   ACAGATGAAA CCTTTAAGAC TCGTGAAGCT ATTCAGCATG AATCAGGAAT CTTGGGACCT  1560
   TTACTTTATG GGGAAGTTGG AGACACACTG TTGATTATAT TTAAGAATCA AGCAAGCAGA  1620
   CCATATAACA TCTACCCTCA CGGAATCACT GATGTCCGTC CTTTGTATTC AAGGAGATTA  1680
   CCAAAAGGTG TAAAACATTT GAAGGATTTT CCAATTCTGC CAGGAGAAAT ATTCAAATAT  1740
   AAATGGACAG TGACTGTAGA AGATGGGCCA ACTAAATCAG ATCCTCGGTG CCTGACCCGC  1800
   TATTACTCTA GTTTCGTTAA TATGGAGAGA GATCTAGCTT CAGGACTCAT TGGCCCTCTC  1860
   CTCATCTGCT ACAAAGAATC TGTAGATCAA AGAGGAAACC AGATAATGTC AGACAAGAGG  1920
   AATGTCATCC TGTTTTCTGT ATTTGATGAG AACCGAAGCT GGTACCTCAC AGAGAATATA  1980
   CAACGCTTTC TCCCCAATCC AGCTGGAGTG CAGCTTGAGG ATCCAGAGTT CCAAGCCTCC     2040
   AACATCATGC ACAGCATCAA TGGCTATGTT TTTGATAGTT TGCAGTTGTC AGTTTGTTTG     2100
   CATGAGGTGG CATACTGGTA CATTCTAAGC ATTGGAGCAC AGACTGACTT CCTTTCTGTC     2160
   TTCTTCTCTG GATATACCTT CAAACACAAA ATGGTCTATG AAGACACACT CACCCTATTC     2220
   CCATTCTCAG GAGAAACTGT CTTCATGTCG ATGGAAAACC CAGGTCTATG GATTCTGGGG     2280
   TGCCACAACT CAGACTTTCG GAACAGAGGC ATGACCGCCT TACTGAAGGT TTCTAGTTGT     2340
   GACAAGAACA CTGGTGATTA TTACGAGGAC AGTTATGAAG ATATTTCAGC ATACTTGCTG     2400
   AGTAAAAACA ATGCCATTGA ACCAAGAAGC TTCTCCCAGA ATTCAAGACA CCCTAGCACT     2460
   AGGCAAAAGC AATTTAATGC CACCACAATT CCAGAAAATG ACATAGAGAA GACTGACCCT     2520
   TGGTTTGCAC ACAGAACACC TATGCCTAAA ATACAAAATG TCTCCTCTAG TGATTTGTTG     2580
   ATGCTCTTGC GACAGAGTCC TACTCCACAT GGGCTATCCT TATCTGATCT CCAAGAAGCC     2640
   AAATATGAGA CTTTTTCTGA TGATCCATCA CCTGGAGCAA TAGACAGTAA TAACAGCCTG     2700
   TCTGAAATGA CACACTTCAG GCCACAGCTC CATCACAGTG GGGACATGGT ATTTACCCCT     2760
   GAGTCAGGCC TCCAATTAAG ATTAAATGAG AAACTGGGGA CAACTGCAGC AACAGAGTTG     2820
   AAGAAACTTG ATTTCAAAGT TTCTAGTACA TCAAATAATC TGATTTCAAC AATTCCATCA     2880
   GACAATTTGG CAGCAGGTAC TGATAATACA AGTTCCTTAG GACCCCCAAG TATGCCAGTT     2940
   CATTATGATA GTCAATTAGA TACCACTCTA TTTGGCAAAA AGTCATCTCC CCTTACTGAG     3000
   TCTGGTGGAC CTCTGAGCTT GAGTGAAGAA AATAATGATT CAAAGTTGTT AGAATCAGGT     3060
   TTAATGAATA GCCAAGAAAG TTCATGGGGA AAAAATGTAT CGTCAACAGA GAGTGGTAGG 3120
   TTATTTAAAG GGAAAAGAGC TCATGGACCT GCTTTGTTGA CTAAAGATAA TGCCTTATTC 3180
   AAAGTTAGCA TCTCTTTGTT AAAGACAAAC AAAACTTCCA ATAATTCAGC AACTAATAGA 3240
   AAGACTCACA TTGATGGCCC ATCATTATTA ATTGAGAATA GTCCATCAGT CTGGCAAAAT 3300
   ATATTAGAAA GTGACACTGA GTTTAAAAAA GTGACACCTT TGATTCATGA CAGAATGCTT 3360
   ATGGACAAAA ATGCTACAGC TTTGAGGCTA AATCATATGT CAAATAAAAC TACTTCATCA 3420
   AAAAACATGG AAATGGTCCA ACAGAAAAAA GAGGGCCCCA TTCCACCAGA TGCACAAAAT 3480
   CCAGATATGT CGTTCTTTAA GATGCTATTC TTGCCAGAAT CAGCAAGGTG GATACAAAGG 3540
   ACTCATGGAA AGAACTCTCT GAACTCTGGG CAAGGCCCCA GTCCAAAGCA ATTAGTATCC 3600
   TTAGGACCAG AAAAATCTGT GGAAGGTCAG AATTTCTTGT CTGAGAAAAA CAAAGTGGTA 3660
   GTAGGAAAGG GTGAATTTAC AAAGGACGTA GGACTCAAAG AGATGGTTTT TCCAAGCAGC 3720
   AGAAACCTAT TTCTTACTAA CTTGGATAAT TTACATGAAA ATAATACACA CAATCAAGAA 3780
   AAAAAAATTC AGGAAGAAAT AGAAAAGAAG GAAACATTAA TCCAAGAGAA TGTAGTTTTG 3840
   CCTCAGATAC ATACAGTGAC TGGCACTAAG AATTTCATGA AGAACCTTTT CTTACTGAGC 3900
   ACTAGGCAAA ATGTAGAAGG TTCATATGAG GGGGCATATG CTCCAGTACT TCAAGATTTT 3960
   AGGTCATTAA ATGATTCAAC AAATAGAACA AAGAAACACA CAGCTCATTT CTCAAAAAAA 4020
   GGGGAGGAAG AAAACTTGGA AGGCTTGGGA AATCAAACCA AGCAAATTGT AGAGAAATAT 4080
   GCATGCACCA CAAGGATATC TCCTAATACA AGCCAGCAGA ATTTTGTCAC GCAACGTAGT 4140
   AAGAGAGCTT TGAAACAATT CAGACTCCCA CTAGAAGAAA CAGAACTTGA AAAAAGGATA 4200
   ATTGTGGATG ACACCTCAAC CCAGTGGTCC AAAAACATGA AACATTTGAC CCCGAGCACC 4260
   CTCACACAGA TAGACTACAA TGAGAAGGAG AAAGGGGCCA TTACTCAGTC TCCCTTATCA 4320
   GATTGCCTTA CGAGGAGTCA TAGCATCCCT CAAGCAAATA GATCTCCATT ACCCATTGCA 4380
   AAGGTATCAT CATTTCCATC TATTAGACCT ATATATCTGA CCAGGGTCCT ATTCCAAGAC 4440
   AACTCTTCTC ATCTTCCAGC AGCATCTTAT AGAAAGAAAG ATTCTGGGGT CCAAGAAAGC 4500
   AGTCATTTCT TACAAGGAGC CAAAAAAAAT AACCTTTCTT TAGCCATTCT AACCTTGGAG 4560
   ATGACTGGTG ATCAAAGAGA GGTTGGCTCC CTGGGGACAA GTGCCACAAA TTCAGTCACA 4620
   TACAAGAAAG TTGAGAACAC TGTTCTCCCG AAACCAGACT TGCCCAAAAC ATCTGGCAAA 4680
   GTTGAATTGC TTCCAAAAGT TCACATTTAT CAGAAGGACC TATTCCCTAC GGAAACTAGC 4740
   AATGGGTCTC CTGGCCATCT GGATCTCGTG GAAGGGAGCC TTCTTCAGGG AACAGAGGGA 4800
   GCGATTAAGT GGAATGAAGC AAACAGACCT GGAAAAGTTC CCTTTCTGAG AGTAGCAACA 4860
   GAAAGCTCTG CAAAGACTCC CTCCAAGCTA TTGGATCCTC TTGCTTGGGA TAACCACTAT 4920
   GGTACTCAGA TACCAAAAGA AGAGTGGAAA TCCCAAGAGA AGTCACCAGA AAAAACAGCT 4980
   TTTAAGAAAA AGGATACCAT TTTGTCCCTG AACGCTTGTG AAAGCAATCA TGCAATAGCA 5040
   GCAATAAATG AGGGACAAAA TAAGCCCGAA ATAGAAGTCA CCTGGGCAAA GCAAGGTAGG 5100
   ACTGAAAGGC TGTGCTCTCA AAACCCACCA GTCTTGAAAC GCCATCAACG GGAAATAACT 5160
   CGTACTACTC TTCAGTCAGA TCAAGAGGAA ATTGACTATG ATGATACCAT ATCAGTTGAA 5220
   ATGAAGAAGG AAGATTTTGA CATTTATGAT GAGGATGAAA ATCAGAGCCC CCGCAGCTTT 5280
   CAAAAGAAAA CACGACACTA TTTTATTGCT GCAGTGGAGA GGCTCTGGGA TTATGGGATG 5340
   AGTAGCTCCC CACATGTTCT AAGAAACAGG GCTCAGAGTG GCAGTGTCCC TCAGTTCAAG 5400
   AAAGTTGTTT TCCAGGAATT TACTGATGGC TCCTTTACTC AGCCCTTATA CCGTGGAGAA 5460
   CTAAATGAAC ATTTGGGACT CCTGGGGCCA TATATAAGAG CAGAAGTTGA AGATAATATC 5520
   ATGGTAACTT TCAGAAATCA GGCCTCTCGT CCCTATTCCT TCTATTCTAG CCTTATTTCT 5580
   TATGAGGAAG ATCAGAGGCA AGGAGCAGAA CCTAGAAAAA ACTTTGTCAA GCCTAATGAA 5640
   ACCAAAACTT ACTTTTGGAA AGTGCAACAT CATATGGCAC CCACTAAAGA TGAGTTTGAC 5700
   TGCAAAGCCT GGGCTTATTT CTCTGATGTT GACCTGGAAA AAGATGTGCA CTCAGGCCTG 5760
   ATTGGACCCC TTCTGGTCTG CCACACTAAC ACACTGAACC CTGCTCATGG GAGACAAGTG 5820
   ACAGTACAGG AATTTGCTCT GTTTTTCACC ATCTTTGATG AGACCAAAAG CTGGTACTTC 5880
   ACTGAAAATA TGGAAAGAAA CTGCAGGGCT CCCTGCAATA TCCAGATGGA AGATCCCACT 5940
   TTTAAAGAGA ATTATCGCTT CCATGCAATC AATGGCTACA TAATGGATAC ACTACCTGGC 6000
   TTAGTAATGG CTCAGGATCA AAGGATTCGA TGGTATCTGC TCAGCATGGG CAGCAATGAA 6060
   AACATCCATT CTATTCATTT CAGTGGACAT GTGTTCACTG TACGAAAAAA AGAGGAGTAT 6120
   AAAATGGCAC TGTACAATCT CTATCCAGGT GTTTTTGAGA CAGTGGAAAT GTTACCATCC 6180
   AAAGCTGGAA TTTGGCGGGT GGAATGCCTT ATTGGCGAGC ATCTACATGC TGGGATGAGC 6240
   ACACTTTTTC TGGTGTACAG CAATAAGTGT CAGACTCCCC TGGGAATGGC TTCTGGACAC 6300
   ATTAGAGATT TTCAGATTAC AGCTTCAGGA CAATATGGAC AGTGGGCCCC AAAGCTGGCC 6360
   AGACTTCATT ATTCCGGATC AATCAATGCC TGGAGCACCA AGGAGCCCTT TTCTTGGATC 6420
   AAGGTGGATC TGTTGGCACC AATGATTATT CACGGCATCA AGACCCAGGG TGCCCGTCAG 6480
   AAGTTCTCCA GCCTCTACAT CTCTCAGTTT ATCATCATGT ATAGTCTTGA TGGGAAGAAG 6540
   TGGCAGACTT ATCGAGGAAA TTCCACTGGA ACCTTAATGG TCTTCTTTGG CAATGTGGAT 6600
   TCATCTGGGA TAAAACACAA TATTTTTAAC CCTCCAATTA TTGCTCGATA CATCCGTTTG 6660
   CACCCAACTC ATTATAGCAT TCGCAGCACT CTTCGCATGG AGTTGATGGG CTGTGATTTA 6720
   AATAGTTGCA GCATGCCATT GGGAATGGAG AGTAAAGCAA TATCAGATGC ACAGATTACT 6780
   GCTTCATCCT ACTTTACCAA TATGTTTGCC ACCTGGTCTC CTTCAAAAGC TCGACTTCAC 6840
   CTCCAAGGGA GGAGTAATGC CTGGAGACCT CAGGTGAATA ATCCAAAAGA GTGGCTGCAA 6900
   GTGGACTTCC AGAAGACAAT GAAAGTCACA GGAGTAACTA CTCAGGGAGT AAAATCTCTG 6960
   CTTACCAGCA TGTATGTGAA GGAGTTCCTC ATCTCCAGCA GTCAAGATGG CCATCAGTGG 7020
   ACTCTCTTTT TTCAGAATGG CAAAGTAAAG GTTTTTCAGG GAAATCAAGA CTCCTTCACA 7080
   CCTGTGGTGA ACTCTCTAGA CCCACCGTTA CTGACTCGCT ACCTTCGAAT TCACCCCCAG 7140
   AGTTGGGTGC ACCAGATTGC CCTGAGGATG GAGGTTCTGG GCTGCGAGGC ACAGGACCTC 7200
   TACTGAGGGT GGCCACTGCA GCACCTGCCA CTGCCGTCAC CTCTCCCTCC TCAGCTCCAG 7260
   GGCAGTGTCC CTCCCTGGCT TGCCTTCTAC CTTTGTGCTA AATCCTAGCA GACACTGCCT   7320
   TGAAGCCTCC TGAATTAACT ATCATCAGTC CTGCATTTCT TTGGTGGGGG GCCAGGAGGG   7380
   TGCATCCAAT TTAACTTAAC TCTTACCTAT TTTCTGCAGC TGCTCCCAGA TTACTCCTTC   7440
   CTTCCAATAT AACTAGGCAA AAAGAAGTGA GGAGAAACCT GCATGAAAGC ATTCTTCCCT   7500
   GAAAAGTTAG GCCTCTCAGA GTCACCACTT CCTCTGTTGT AGAAAAACTA TGTGATGAAA   7560
   CTTTGAAAAA GATATTTATG ATGTTAACAT TTCAGGTTAA GCCTCATACG TTTAAAATAA   7620
   AACTCTCAGT TGTTTATTAT CCTGATCAAG CATGGAACAA AGCATGTTTC AGGATCAGAT   7680
   CAATACAATC TTGGAGTCAA AAGGCAAATC ATTTGGACAA TCTGCAAAAT GGAGAGAATA   7740
   CAATAACTAC TACAGTAAAG TCTGTTTCTG CTTCCTTACA CATAGATATA ATTATGTTAT   7800
   TTAGTCATTA TGAGGGGCAC ATTCTTATCT CCAAAACTAG CATTCTTAAA CTGAGAATTA   7860
   TAGATGGGGT TCAAGAATCC CTAAGTCCCC TGAAATTATA TAAGGCATTC TGTATAAATG   7920
   CAAATGTGCA TTTTTCTGAC GAGTGTCCAT AGATATAAAG CCATTGGTCT TAATTCTGAC   7980
   CAATAAAAAA ATAAGTCAGG AGGATGCAAT TGTTGAAAGC TTTGAAATAA AATAACATGT   8040
   CTTCTTGAAA TTTGTGATGG CCAAGAAAGA AAATGATGAT GACATTAGGC TTCTAAAGGA   8100
   CATACATTTA ATATTTCTGT GGAAATATGA GGAAAATCCA TGGTTATCTG AGATAGGAGA   8160
   TACAAACTTT GTAATTCTAA TAATGCACTC AGTTTACTCT CTCCCTCTAC TAATTTCCTG   8220
   CTGAAAATAA CACAACAAAA ATGTAACAGG GGAAATTATA TACCGTGACT GAAAACTAGA   8280
   GTCCTACTTA CATAGTTGAA ATATCAAGGA GGTCAGAAGA AAATTGGACT GGTGAAAACA   8340
   GAAAAAACAC TCCAGTCTGC CATATCACCA CACAATAGGA TCCCCCTTCT TGCCCTCCAC   8400
   CCCCATAAGA TTGTGAAGGG TTTACTGCTC CTTCCATCTG CCTGCACCCC TTCACTATGA   8460
   CTACACAGAA CTCTCCTGAT AGTAAAGGGG GCTGGAGGCA AGGATAAGTT ATAGAGCAGT   8520
   TGGAGGAAGC ATCCAAAGAC TGCAACCCAG GGCAAATGGA AAACAGGAGA TCCTAATATG   8580
   AAAGAAAAAT GGATCCCAAT CTGAGAAAAG GCAAAAGAAT GGCTACTTTT TTCTATGCTG   8640
   GAGTATTTTC TAATAATCCT GCTTGACCCT TATCTGACCT CTTTGGAAAC TATAACATAG   8700
   CTGTCACAGT ATAGTCACAA TCCACAAATG ATGCAGGTGC AAATGGTTTA TAGCCCTGTG   8760
   AAGTTCTTAA AGTTTAGAGG CTAACTTACA GAAATGAATA AGTTGTTTTG TTTTATAGCC   8820
   CGGTAGAGGA GTTAACCCCA AAGGTGATAT GGTTTTATTT CCTGTTATGT TTAACTTGAT   8880
   AATCTTATTT TGGCATTCTT TTCCCATTGA CTATATACAT CTCTATTTCT CAAATGTTCA   8940
   TGGAACTAGC TCTTTTATTT TCCTGCTGGT TTCTTCAGTA ATGAGTTAAA TAAAACATTG   9000
   ACACATACA                                                           9009
(2) INFORMATION FOR SEQ ID NO:2:
   (i) SEQUENCE CHARACTERISTICS:
      (A) LENGTH: 2332 amino acids
      (B) TYPE: amino acid
      (C) STRANDEDNESS: single
      (D) TOPOLOGY: linear
   (ii) MOLECULE TYPE: protein
   (iii) HYPOTHETICAL: YES
   (iv) ANTI-SENSE: NO
   (v) FRAGMENT TYPE: N-terminal
   (vi) ORIGINAL SOURCE:
      (A) ORGANISM: Homo sapien
      (F) TISSUE TYPE: Liver cDNA sequence
   (xi) SEQUENCE DESCRIPTION: SEQ ID NO:2:
   Ala Thr Arg Arg Tyr Tyr Leu Gly Ala Val Glu Leu Ser Trp Asp Tyr
   1               5                   10                  15
   Met Gln Ser Asp Leu Gly Glu Leu Pro Val Asp Ala Arg Phe Pro Pro
               20                  25                  30
   Arg Val Pro Lys Ser Phe Pro Phe Asn Thr Ser Val Val Tyr Lys Lys
           35                  40                  45
   Thr Leu Phe Val Glu Phe Thr Val His Leu Phe Asn Ile Ala Lys Pro
       50                  55                  60
   Arg Pro Pro Trp Met Gly Leu Leu Gly Pro Thr Ile Gln Ala Glu Val
   65                  70                  75                  80
   Tyr Asp Thr Val Val Ile Thr Leu Lys Asn Met Ala Ser His Pro Val
                   85                  90                  95
   Ser Leu His Ala Val Gly Val Ser Tyr Trp Lys Ala Ser Glu Gly Ala
               100                 105                 110
   Glu Tyr Asp Asp Gln Thr Ser Gln Arg Glu Lys Glu Asp Asp Lys Val
           115                 120                 125
   Phe Pro Gly Gly Ser His Thr Tyr Val Trp Gln Val Leu Lys Glu Asn
       130                 135                 140
   Gly Pro Met Ala Ser Asp Pro Leu Cys Leu Thr Tyr Ser Tyr Leu Ser
   145                 150                 155                 160
   His Val Asp Leu Val Lys Asp Leu Asn Ser Gly Leu Ile Gly Ala Leu
                   165                 170                 175
   Leu Val Cys Arg Glu Gly Ser Leu Ala Lys Glu Lys Thr Gln Thr Leu
               180                 185                 190
   His Lys Phe Ile Leu Leu Phe Ala Val Phe Asp Glu Gly Lys Ser Trp
           195                 200                 205
   His Ser Glu Thr Lys Asn Ser Leu Met Gln Asp Arg Asp Ala Ala Ser
       210                 215                 220
   Ala Arg Ala Trp Pro Lys Met His Thr Val Asn Gly Tyr Val Asn Arg
   225                 230                 235                 240
   Ser Leu Pro Gly Leu Ile Gly Cys His Arg Lys Ser Val Tyr Trp His
                   245                 250                 255
   Val Ile Gly Met Gly Thr Thr Pro Glu Val His Ser Ile Phe Leu Glu
               260                 265                 270
   Gly His Thr Phe Leu Val Arg Asn His Arg Gln Ala Ser Leu Glu Ile
           275                 280                 285
   Ser Pro Ile Thr Phe Leu Thr Ala Gln Thr Leu Leu Met Asp Leu Gly
       290                 295                 300
   Gln Phe Leu Leu Phe Cys His Ile Ser Ser His Gln His Asp Gly Met
   305 310 315 320
   Glu Ala Tyr Val Lys Val Asp Ser Cys Pro Glu Glu Pro Gln Leu Arg
   325 330 335
   Met Lys Asn Asn Glu Glu Ala Glu Asp Tyr Asp Asp Asp Leu Thr Asp
   340 345 350
   Ser Glu Met Asp Val Val Arg Phe Asp Asp Asp Asn Ser Pro Ser Phe
   355 360 365
   Ile Gln Ile Arg Ser Val Ala Lys Lys His Pro Lys Thr Trp Val His
   370 375 380
   Tyr Ile Ala Ala Glu Glu Glu Asp Trp Asp Tyr Ala Pro Leu Val Leu
   385 390 395 400
   Ala Pro Asp Asp Arg Ser Tyr Lys Ser Gln Tyr Leu Asn Asn Gly Pro
   405 410 415
   Gln Arg Ile Gly Arg Lys Tyr Lys Lys Val Arg Phe Met Ala Tyr Thr
   420 425 430
   Asp Glu Thr Phe Lys Thr Arg Glu Ala Ile Gln His Glu Ser Gly Ile
   435 440 445
   Leu Gly Pro Leu Leu Tyr Gly Glu Val Gly Asp Thr Leu Leu Ile Ile
   450 455 460
   Phe Lys Asn Gln Ala Ser Arg Pro Tyr Asn Ile Tyr Pro His Gly Ile
   465 470 475 480
   Thr Asp Val Arg Pro Leu Tyr Ser Arg Arg Leu Pro Lys Gly Val Lys
   485 490 495
   His Leu Lys Asp Phe Pro Ile Leu Pro Gly Glu Ile Phe Lys Tyr Lys
   500 505 510
   Trp Thr Val Thr Val Glu Asp Gly Pro Thr Lys Ser Asp Pro Arg Cys
   515 520 525
   Leu Thr Arg Tyr Tyr Ser Ser Phe Val Asn Met Glu Arg Asp Leu Ala
   530 535 540
   Ser Gly Leu Ile Gly Pro Leu Leu Ile Cys Tyr Lys Glu Ser Val Asp
   545 550 555 560
   Gln Arg Gly Asn Gln Ile Met Ser Asp Lys Arg Asn Val Ile Leu Phe
   565 570 575
   Ser Val Phe Asp Glu Asn Arg Ser Trp Tyr Leu Thr Glu Asn Ile Gln
   580 585 590
   Arg Phe Leu Pro Asn Pro Ala Gly Val Gln Leu Glu Asp Pro Glu Phe
   595 600 605
   Gln Ala Ser Asn Ile Met His Ser Ile Asn Gly Tyr Val Phe Asp Ser
   610 615 620
   Leu Gln Leu Ser Val Cys Leu His Glu Val Ala Tyr Trp Tyr Ile Leu
   625 630 635 640
   Ser Ile Gly Ala Gln Thr Asp Phe Leu Ser Val Phe Phe Ser Gly Tyr
   645 650 655
   Thr Phe Lys His Lys Met Val Tyr Glu Asp Thr Leu Thr Leu Phe Pro
   660 665 670
   Phe Ser Gly Glu Thr Val Phe Met Ser Met Glu Asn Pro Gly Leu Trp
   675 680 685
   Ile Leu Gly Cys His Asn Ser Asp Phe Arg Asn Arg Gly Met Thr Ala
   690 695 700
   Leu Leu Lys Val Ser Ser Cys Asp Lys Asn Thr Gly Asp Tyr Tyr Glu
   705 710 715 720
   Asp Ser Tyr Glu Asp Ile Ser Ala Tyr Leu Leu Ser Lys Asn Asn Ala
   725 730 735
   Ile Glu Pro Arg Ser Phe Ser Gln Asn Ser Arg His Pro Ser Thr Arg
   740 745 750
   Gln Lys Gln Phe Asn Ala Thr Thr Ile Pro Glu Asn Asp Ile Glu Lys
   755 760 765
   Thr Asp Pro Trp Phe Ala His Arg Thr Pro Met Pro Lys Ile Gln Asn
   770 775 780
   Val Ser Ser Ser Asp Leu Leu Met Leu Leu Arg Gln Ser Pro Thr Pro
   785 790 795 800
   His Gly Leu Ser Leu Ser Asp Leu Gln Glu Ala Lys Tyr Glu Thr Phe
   805 810 815
   Ser Asp Asp Pro Ser Pro Gly Ala Ile Asp Ser Asn Asn Ser Leu Ser
   820 825 830
   Glu Met Thr His Phe Arg Pro Gln Leu His His Ser Gly Asp Met Val
   835 840 845
   Phe Thr Pro Glu Ser Gly Leu Gln Leu Arg Leu Asn Glu Lys Leu Gly
   850 855 860
   Thr Thr Ala Ala Thr Glu Leu Lys Lys Leu Asp Phe Lys Val Ser Ser
   865 870 875 880
   Thr Ser Asn Asn Leu Ile Ser Thr Ile Pro Ser Asp Asn Leu Ala Ala
   885 890 895
   Gly Thr Asp Asn Thr Ser Ser Leu Gly Pro Pro Ser Met Pro Val His
   900 905 910
   Tyr Asp Ser Gln Leu Asp Thr Thr Leu Phe Gly Lys Lys Ser Ser Pro
   915 920 925
   Leu Thr Glu Ser Gly Gly Pro Leu Ser Leu Ser Glu Glu Asn Asn Asp
   930 935 940
   Ser Lys Leu Leu Glu Ser Gly Leu Met Asn Ser Gln Glu Ser Ser Trp
   945 950 955 960
   Gly Lys Asn Val Ser Ser Thr Glu Ser Gly Arg Leu Phe Lys Gly Lys
   965 970 975
   Arg Ala His Gly Pro Ala Leu Leu Thr Lys Asp Asn Ala Leu Phe Lys
   980 985 990
   Val Ser Ile Ser Leu Leu Lys Thr Asn Lys Thr Ser Asn Asn Ser Ala
   995 1000 1005
   Thr Asn Arg Lys Thr His Ile Asp Gly Pro Ser Leu Leu Ile Glu Asn
   1010 1015 1020
   Ser Pro Ser Val Trp Gln Asn Ile Leu Glu Ser Asp Thr Glu Phe Lys
   1025 1030 1035 1040
   Lys Val Thr Pro Leu Ile His Asp Arg Met Leu Met Asp Lys Asn Ala
   1045 1050 1055
   Thr Ala Leu Arg Leu Asn His Met Ser Asn Lys Thr Thr Ser Ser Lys
               1060                1065                1070
   Asn Met Glu Met Val Gln Gln Lys Lys Glu Gly Pro Ile Pro Pro Asp
           1075                1080                1085
   Ala Gln Asn Pro Asp Met Ser Phe Phe Lys Met Leu Phe Leu Pro Glu
       1090                1095                1100
   Ser Ala Arg Trp Ile Gln Arg Thr His Gly Lys Asn Ser Leu Asn Ser
   1105                1110                1115                1120
   Gly Gln Gly Pro Ser Pro Lys Gln Leu Val Ser Leu Gly Pro Glu Lys
                   1125                1130                1135
   Ser Val Glu Gly Gln Asn Phe Leu Ser Glu Lys Asn Lys Val Val Val
               1140                1145                1150
   Gly Lys Gly Glu Phe Thr Lys Asp Val Gly Leu Lys Glu Met Val Phe
           1155                1160                1165
   Pro Ser Ser Arg Asn Leu Phe Leu Thr Asn Leu Asp Asn Leu His Glu
       1170                1175                1180
   Asn Asn Thr His Asn Gln Glu Lys Lys Ile Gln Glu Glu Ile Glu Lys
   1185                1190                1195                1200
   Lys Glu Thr Leu Ile Gln Glu Asn Val Val Leu Pro Gln Ile His Thr
                   1205                1210                1215
   Val Thr Gly Thr Lys Asn Phe Met Lys Asn Leu Phe Leu Leu Ser Thr
               1220                1225                1230
   Arg Gln Asn Val Glu Gly Ser Tyr Glu Gly Ala Tyr Ala Pro Val Leu
           1235                1240                1245
   Gln Asp Phe Arg Ser Leu Asn Asp Ser Thr Asn Arg Thr Lys Lys His
   1250 1255 1260
   Thr Ala His Phe Ser Lys Lys Gly Glu Glu Glu Asn Leu Glu Gly Leu
   1265 1270 1275 1280
   Gly Asn Gln Thr Lys Gln Ile Val Glu Lys Tyr Ala Cys Thr Thr Arg
   1285 1290 1295
   Ile Ser Pro Asn Thr Ser Gln Gln Asn Phe Val Thr Gln Arg Ser Lys
   1300 1305 1310
   Arg Ala Leu Lys Gln Phe Arg Leu Pro Leu Glu Glu Thr Glu Leu Glu
   1315 1320 1325
   Lys Arg Ile Ile Val Asp Asp Thr Ser Thr Gln Trp Ser Lys Asn Met
   1330 1335 1340
   Lys His Leu Thr Pro Ser Thr Leu Thr Gln Ile Asp Tyr Asn Glu Lys
   1345 1350 1355 1360
   Glu Lys Gly Ala Ile Thr Gln Ser Pro Leu Ser Asp Cys Leu Thr Arg
   1365 1370 1375
   Ser His Ser Ile Pro Gln Ala Asn Arg Ser Pro Leu Pro Ile Ala Lys
   1380 1385 1390
   Val Ser Ser Phe Pro Ser Ile Arg Pro Ile Tyr Leu Thr Arg Val Leu
   1395 1400 1405
   Phe Gln Asp Asn Ser Ser His Leu Pro Ala Ala Ser Tyr Arg Lys Lys
   1410 1415 1420
   Asp Ser Gly Val Gln Glu Ser Ser His Phe Leu Gln Gly Ala Lys Lys
   1425 1430 1435 1440
   Asn Asn Leu Ser Leu Ala Ile Leu Thr Leu Glu Met Thr Gly Asp Gln
                   1445                1450                1455
   Arg Glu Val Gly Ser Leu Gly Thr Ser Ala Thr Asn Ser Val Thr Tyr
               1460                1465                1470
   Lys Lys Val Glu Asn Thr Val Leu Pro Lys Pro Asp Leu Pro Lys Thr
           1475                1480                1485
   Ser Gly Lys Val Glu Leu Leu Pro Lys Val His Ile Tyr Gln Lys Asp
       1490                1495                1500
   Leu Phe Pro Thr Glu Thr Ser Asn Gly Ser Pro Gly His Leu Asp Leu
   1505                1510                1515                1520
   Val Glu Gly Ser Leu Leu Gln Gly Thr Glu Gly Ala Ile Lys Trp Asn
                   1525                1530                1535
   Glu Ala Asn Arg Pro Gly Lys Val Pro Phe Leu Arg Val Ala Thr Glu
               1540                1545                1550
   Ser Ser Ala Lys Thr Pro Ser Lys Leu Leu Asp Pro Leu Ala Trp Asp
           1555                1560                1565
   Asn His Tyr Gly Thr Gln Ile Pro Lys Glu Glu Trp Lys Ser Gln Glu
       1570                1575                1580
   Lys Ser Pro Glu Lys Thr Ala Phe Lys Lys Lys Asp Thr Ile Leu Ser
   1585                1590                1595                1600
   Leu Asn Ala Cys Glu Ser Asn His Ala Ile Ala Ala Ile Asn Glu Gly
                   1605                1610                1615
   Gln Asn Lys Pro Glu Ile Glu Val Thr Trp Ala Lys Gln Gly Arg Thr
               1620                1625                1630
   Glu Arg Leu Cys Ser Gln Asn Pro Pro Val Leu Lys Arg His Gln Arg
   1635 1640 1645
   Glu Ile Thr Arg Thr Thr Leu Gln Ser Asp Gln Glu Glu Ile Asp Tyr
   1650 1655 1660
   Asp Asp Thr Ile Ser Val Glu Met Lys Lys Glu Asp Phe Asp Ile Tyr
   1665 1670 1675 1680
   Asp Glu Asp Glu Asn Gln Ser Pro Arg Ser Phe Gln Lys Lys Thr Arg
   1685 1690 1695
   His Tyr Phe Ile Ala Ala Val Glu Arg Leu Trp Asp Tyr Gly Met Ser
   1700 1705 1710
   Ser Ser Pro His Val Leu Arg Asn Arg Ala Gln Ser Gly Ser Val Pro
   1715 1720 1725
   Gln Phe Lys Lys Val Val Phe Gln Glu Phe Thr Asp Gly Ser Phe Thr
   1730 1735 1740
   Gln Pro Leu Tyr Arg Gly Glu Leu Asn Glu His Leu Gly Leu Leu Gly
   1745 1750 1755 1760
   Pro Tyr Ile Arg Ala Glu Val Glu Asp Asn Ile Met Val Thr Phe Arg
   1765 1770 1775
   Asn Gln Ala Ser Arg Pro Tyr Ser Phe Tyr Ser Ser Leu Ile Ser Tyr
   1780 1785 1790
   Glu Glu Asp Gln Arg Gln Gly Ala Glu Pro Arg Lys Asn Phe Val Lys
   1795 1800 1805
   Pro Asn Glu Thr Lys Thr Tyr Phe Trp Lys Val Gln His His Met Ala
   1810 1815 1820
   Pro Thr Lys Asp Glu Phe Asp Cys Lys Ala Trp Ala Tyr Phe Ser Asp
   1825                1830                1835                1840
   Val Asp Leu Glu Lys Asp Val His Ser Gly Leu Ile Gly Pro Leu Leu
                   1845                1850                1855
   Val Cys His Thr Asn Thr Leu Asn Pro Ala His Gly Arg Gln Val Thr
               1860                1865                1870
   Val Gln Glu Phe Ala Leu Phe Phe Thr Ile Phe Asp Glu Thr Lys Ser
           1875                1880                1885
   Trp Tyr Phe Thr Glu Asn Met Glu Arg Asn Cys Arg Ala Pro Cys Asn
       1890                1895                1900
   Ile Gln Met Glu Asp Pro Thr Phe Lys Glu Asn Tyr Arg Phe His Ala
   1905                1910                1915                1920
   Ile Asn Gly Tyr Ile Met Asp Thr Leu Pro Gly Leu Val Met Ala Gln
                   1925                1930                1935
   Asp Gln Arg Ile Arg Trp Tyr Leu Leu Ser Met Gly Ser Asn Glu Asn
               1940                1945                1950
   Ile His Ser Ile His Phe Ser Gly His Val Phe Thr Val Arg Lys Lys
           1955                1960                1965
   Glu Glu Tyr Lys Met Ala Leu Tyr Asn Leu Tyr Pro Gly Val Phe Glu
       1970                1975                1980
   Thr Val Glu Met Leu Pro Ser Lys Ala Gly Ile Trp Arg Val Glu Cys
   1985                1990                1995                2000
   Leu Ile Gly Glu His Leu His Ala Gly Met Ser Thr Leu Phe Leu Val
                   2005                2010                2015
   Tyr Ser Asn Lys Cys Gln Thr Pro Leu Gly Met Ala Ser Gly His Ile
   2020 2025 2030
   Arg Asp Phe Gln Ile Thr Ala Ser Gly Gln Tyr Gly Gln Trp Ala Pro
   2035 2040 2045
   Lys Leu Ala Arg Leu His Tyr Ser Gly Ser Ile Asn Ala Trp Ser Thr
   2050 2055 2060
   Lys Glu Pro Phe Ser Trp Ile Lys Val Asp Leu Leu Ala Pro Met Ile
   2065 2070 2075 2080
   Ile His Gly Ile Lys Thr Gln Gly Ala Arg Gln Lys Phe Ser Ser Leu
   2085 2090 2095
   Tyr Ile Ser Gln Phe Ile Ile Met Tyr Ser Leu Asp Gly Lys Lys Trp
   2100 2105 2110
   Gln Thr Tyr Arg Gly Asn Ser Thr Gly Thr Leu Met Val Phe Phe Gly
   2115 2120 2125
   Asn Val Asp Ser Ser Gly Ile Lys His Asn Ile Phe Asn Pro Pro Ile
   2130 2135 2140
   Ile Ala Arg Tyr Ile Arg Leu His Pro Thr His Tyr Ser Ile Arg Ser
   2145 2150 2155 2160
   Thr Leu Arg Met Glu Leu Met Gly Cys Asp Leu Asn Ser Cys Ser Met
   2165 2170 2175
   Pro Leu Gly Met Glu Ser Lys Ala Ile Ser Asp Ala Gln Ile Thr Ala
   2180 2185 2190
   Ser Ser Tyr Phe Thr Asn Met Phe Ala Thr Trp Ser Pro Ser Lys Ala
   2195 2200 2205
   Arg Leu His Leu Gln Gly Arg Ser Asn Ala Trp Arg Pro Gln Val Asn
       2210                2215                2220
   Asn Pro Lys Glu Trp Leu Gln Val Asp Phe Gln Lys Thr Met Lys Val
   2225                2230                2235                2240
   Thr Gly Val Thr Thr Gln Gly Val Lys Ser Leu Leu Thr Ser Met Tyr
                   2245                2250                2255
   Val Lys Glu Phe Leu Ile Ser Ser Ser Gln Asp Gly His Gln Trp Thr
               2260                2265                2270
   Leu Phe Phe Gln Asn Gly Lys Val Lys Val Phe Gln Gly Asn Gln Asp
           2275                2280                2285
   Ser Phe Thr Pro Val Val Asn Ser Leu Asp Pro Pro Leu Leu Thr Arg
       2290                2295                2300
   Tyr Leu Arg Ile His Pro Gln Ser Trp Val His Gln Ile Ala Leu Arg
   2305                2310                2315                2320
   Met Glu Val Leu Gly Cys Glu Ala Gln Asp Leu Tyr
                   2325                2330
(2) INFORMATION FOR SEQ ID NO:3:
   (i) SEQUENCE CHARACTERISTICS:
      (A) LENGTH: 1130 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Porcine
      (F) TISSUE TYPE: Blood
   (ix) FEATURE:
      (A) NAME/KEY: Region
      (B) LOCATION: 1..1130
      (D) OTHER INFORMATION: /note= "cDNA encoding A2 domain of porcine factor VIII."
   (xi) SEQUENCE DESCRIPTION: SEQ ID NO:3:
   TAAGCACCCT AAGACGTGGG TGCACTACAT CTCTGCAGAG GAGGAGGACT GGGACTACGC     60
   CCCCGCGGTC CCCAGCCCCA GTGACAGAAG TTATAAAAGT CTCTACTTGA ACAGTGGTCC    120
   TCAGCGAATT GGTAGGAAAT ACAAAAAAGC TCGATTCGTC GCTTACACGG ATGTAACATT    180
   TAAGACTCGT AAAGCTATTC CGTATGAATC AGGAATCCTG GGACCTTTAC TTTATGGAGA    240
   AGTTGGAGAC ACACTTTTGA TTATATTTAA GAATAAAGCG AGCCGACCAT ATAACATCTA    300
   CCCTCATGGA ATCACTGATG TCAGCGCTTT GCACCCAGGG AGACTTCTAA AAGGTTGGAA    360
   ACATTTGAAA GACATGCCAA TTCTGCCAGG AGAGACTTTC AAGTATAAAT GGACAGTGAC    420
   TGTGGAAGAT GGGCCAACCA AGTCCGATCC TCGGTGCCTG ACCCGCTACT ACTCGAGCTC    480
   CATTAATCTA GAGAAAGATC TGGCTTCGGG ACTCATTGGC CCTCTCCTCA TCTGCTACAA    540
   AGAATCTGTA GACCAAAGAG GAAACCAGAT GATGTCAGAC AAGAGAAACG TCATCCTGTT    600
   TTCTGTATTC GATGAGAATC AAAGCTGGTA CCTCGCAGAG AATATTCAGC GCTTCCTCCC    660
   CAATCCGGAT GGATTACAGC CCCAGGATCC AGAGTTCCAA GCTTCTAACA TCATGCACAG    720
   CATCAATGGC TATGTTTTTG ATAGCTTGCA GCTGTCGGTT TGTTTGCACG AGGTGGCATA    780
   CTGGTACATT CTAAGTGTTG GAGCACAGAC GGACTTCCTC TCCGTCTTCT TCTCTGGCTA    840
   CACCTTCAAA CACAAAATGG TCTATGAAGA CACACTCACC CTGTTCCCCT TCTCAGGAGA    900
   AACGGTCTTC ATGTCAATGG AAAACCCAGG TCTCTGGGTC CTAGGGTGCC ACAACTCAGA    960
   CTTGCGGAAC AGAGGGATGA CAGCCTTACT GAAGGTGTAT AGTTGTGACA GGGACATTGG   1020
   TGATTATTAT GACAACACTT ATGAAGATAT TCCAGGCTTC TTGCTGAGTG GAAAGAATGT   1080
   CATTGAACCC AGAAGCTTTG CCCAGAATTC AAGACCCCCT AGTGCGAGCA              1130
(2) INFORMATION FOR SEQ ID NO:4:
   (i) SEQUENCE CHARACTERISTICS:
      (A) LENGTH: 368 amino acids
      (B) TYPE: amino acid
      (C) STRANDEDNESS: single
      (D) TOPOLOGY: linear
   (ii) MOLECULE TYPE: protein
   (iii) HYPOTHETICAL: YES
   (iv) ANTI-SENSE: NO
   (v) FRAGMENT TYPE: N-terminal
   (vi) ORIGINAL SOURCE:
      (A) ORGANISM: Porcine
      (F) TISSUE TYPE: Spleen
   (ix) FEATURE:
      (A) NAME/KEY: Protein
      (B) LOCATION: 1..368
      (D) OTHER INFORMATION: /note= "Predicted amino acid sequence of the porcine factor VIII A2 domain, defined as residues homologous to human factor VIII amino acid sequence 373-740.
         (Residues 1-4 are from known porcine amino acid sequence.)
   (xi) SEQUENCE DESCRIPTION: SEQ ID NO:4:
   Ser Val Ala Lys Lys His Pro Lys Thr Trp Val His Tyr Ile Ser Ala
   1               5                   10                  15
   Glu Glu Glu Asp Trp Asp Tyr Ala Pro Ala Val Pro Ser Pro Ser Asp
               20                  25                  30
   Arg Ser Tyr Lys Ser Leu Tyr Leu Asn Ser Gly Pro Gln Arg Ile Gly
           35                  40                  45
   Arg Lys Tyr Lys Lys Ala Arg Phe Val Ala Tyr Thr Asp Val Thr Phe
       50                  55                  60
   Lys Thr Arg Lys Ala Ile Pro Tyr Glu Ser Gly Ile Leu Gly Pro Leu
   65                  70                  75                  80
   Leu Tyr Gly Glu Val Gly Asp Thr Leu Leu Ile Ile Phe Lys Asn Lys
                   85                  90                  95
   Ala Ser Arg Pro Tyr Asn Ile Tyr Pro His Gly Ile Thr Asp Val Ser
               100                 105                 110
   Ala Leu His Pro Gly Arg Leu Leu Lys Gly Trp Lys His Leu Lys Asp
           115                 120                 125
   Met Pro Ile Leu Pro Gly Glu Thr Phe Lys Tyr Lys Trp Thr Val Thr
       130                 135                 140
   Val Glu Asp Gly Pro Thr Lys Ser Asp Pro Arg Cys Leu Thr Arg Tyr
   145                 150                 155                 160
   Tyr Ser Ser Ser Ile Asn Leu Glu Lys Asp Leu Ala Ser Gly Leu Ile
                   165                 170                 175
   Gly Pro Leu Leu Ile Cys Tyr Lys Glu Ser Val Asp Gln Arg Gly Asn
               180                 185                 190
   Gln Met Met Ser Asp Lys Arg Asn Val Ile Leu Phe Ser Val Phe Asp
           195                 200                 205
   Glu Asn Gln Ser Trp Tyr Leu Ala Glu Asn Ile Gln Arg Phe Leu Pro
       210                 215                 220
   Asn Pro Asp Gly Leu Gln Pro Gln Asp Pro Glu Phe Gln Ala Ser Asn
   225                 230                 235                 240
   Ile Met His Ser Ile Asn Gly Tyr Val Phe Asp Ser Leu Gln Leu Ser
                   245                 250                 255
   Val Cys Leu His Glu Val Ala Tyr Trp Tyr Ile Leu Ser Val Gly Ala
               260                 265                 270
   Gln Thr Asp Phe Leu Ser Val Phe Phe Ser Gly Tyr Thr Phe Lys His
           275                 280                 285
   Lys Met Val Tyr Glu Asp Thr Leu Thr Leu Phe Pro Phe Ser Gly Glu
       290                 295                 300
   Thr Val Phe Met Ser Met Glu Asn Pro Gly Leu Trp Val Leu Gly Cys
   305                 310                 315                 320
   His Asn Ser Asp Leu Arg Asn Arg Gly Met Thr Ala Leu Leu Lys Val
                   325                 330                 335
   Tyr Ser Cys Asp Arg Asp Ile Gly Asp Tyr Tyr Asp Asn Thr Tyr Glu
               340                 345                 350
   Asp Ile Pro Gly Phe Leu Leu Ser Gly Lys Asn Val Ile Glu Pro Arg
           355                 360                 365
(2) INFORMATION FOR SEQ ID NO:5:
   (i) SEQUENCE CHARACTERISTICS:
      (A) LENGTH: 7493 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: Mus musculus
   (ix) FEATURE:
      (A) NAME/KEY: repeat_unit
      (B) LOCATION: 1..407
      (D) OTHER INFORMATION: /rpt_type= "terminal" /note= "5'UTR"
   (ix) FEATURE:
      (A) NAME/KEY: misc_feature
      (B) LOCATION: 7471..7476
      (D) OTHER INFORMATION: /function= "PolyA_signal"
   (ix) FEATURE:
      (A) NAME/KEY: repeat_unit
      (B) LOCATION: 7368..7493
      (D) OTHER INFORMATION: /rpt_type= "terminal" /note= "3'UTR"
   (ix) FEATURE:
      (A) NAME/KEY: misc_feature
      (B) LOCATION: 408..7367
      (D) OTHER INFORMATION: /product= "Coagulation Factor VIII"
   (x) PUBLICATION INFORMATION:
      (A) AUTHORS: Elder, F.
         Lakich, D.
         Gitschier, J.
      (B) TITLE: Sequence of the Murine Factor VIII cDNA.
      (C) JOURNAL: Genomics
      (D) VOLUME: 16
      (F) PAGES: 374-379
      (G) DATE: 1993
      (K) RELEVANT RESIDUES IN SEQ ID NO:5: FROM 1 TO 7476
   (xi) SEQUENCE DESCRIPTION: SEQ ID NO:5:
   TCTAGAGTTT CTTTGCTACA GGTACCAAGG AACAGTCTTT TAGAATAGGC TAGGAATTTA     60
   AATACACCTG AACGCCCCTC CTCAGTATTC TGTTCCTTTT CTTAAGGATT CAAACTTGTT    120
   AGGATGCACC CAGCAGGAAA TGGGTTAAGC CTTAGCTCAG CCACTCTTCC TATTCCAGTT    180
   TTCCTGTGCC TGCTTCCTAC TACCCAAAAG GAAGTAATCC TTCAGATCTG TTTTGTGCTA    240
   ATGCTACTTT CACTCACAGT AGATAAACTT CCAGAAAATC CTCTGCAAAA TATTTAGGAC    300
   TTTTTACTAA ATCATTACAT TTCTTTTTGT TCTTAAAAGC TAAAGTTATT TTAGAGAAGA    360
   GTTAAATTTT CATTTCTTTA GTTGAACATT TTCTAGTAAT AAAAGCCATG CAAATAGCAC    420
   TCTTCGCTTG CTTCTTTCTG AGCCTTTTCA ATTTCTGCTC TAGTGCCATC AGAAGATACT    480
   ACCTTGGTGC AGTGGAATTG TCCTGGAACT ATATTCAGAG TGATCTGCTC AGTGTGCTGC    540
   ATACAGACTC AAGATTTCTT CCTAGAATGT CAACATCTTT TCCATTCAAC ACCTCCATCA    600
   TGTATAAAAA GACTGTGTTT GTAGAGTACA AGGACCAGCT TTTCAACATT GCCAAGCCCA    660
   GGCCACCCTG GATGGGTTTG CTAGGTCCTA CCATTTGGAC TGAGGTTCAT GACACAGTGG    720
   TCATTACACT TAAAAACATG GCTTCTCATC CTGTCAGTCT TCATGCTGTT GGTGTGTCCT    780
   ACTGGAAAGC TTCTGAGGGA GATGAATATG AAGATCAGAC AAGCCAAATG GAGAAGGAAG    840
   ATGATAAAGT TTTCCCTGGT GAAAGTCATA CTTATGTTTG GCAAGTCCTG AAAGAGAATG    900
   GTCCAATGGC CTCTGACCCT CCATGTCTCA CTTACTCATA TATGTCTCAT GTGGATCTGG    960
   TGAAAGATTT GAATTCAGGC CTCATTGGAG CTCTGCTAGT ATGTAAAGAA GGCAGTCTCT   1020
   CCAAAGAAAG AACACAGATG TTGTACCAAT TTGTACTGCT TTTTGCTGTA TTTGATGAAG   1080
   GGAAGAGCTG GCACTCAGAA ACAAACGACT CTTATACACA GTCTATGGAT TCTGCATCTG   1140
   CTAGAGACTG GCCTAAAATG CACACAGTCA ATGGCTATGT AAACAGGTCT CTTCCAGGTC   1200
   TGATTGGATG CCATAGGAAA TCAGTCTACT GGCACGTGAT TGGAATGGGC ACCACTCCTG 1260
   AAATACACTC AATATTCCTC GAAGGTCACA CATTTTTTGT GAGGAACCAC CGTCAAGCTT 1320
   CATTGGAGAT ATCACCAATA ACTTTCCTTA CTGCTCAAAC ACTCTTGATA GATCTTGGGC 1380
   AGTTCCTACT ATTTTGTCAT ATCTCTTCCC ATAAACATGA TGGCATGGAA GCTTATGTCA 1440
   AAGTAGATAG CTGCCCTGAG GAATCCCAAT GGCAAAAGAA AAATAATAAT GAGGAAATGG 1500
   AAGATTATGA TGATGATCTT TATTCAGAAA TGGATATGTT CACATTGGAT TATGACAGCT 1560
   CTCCTTTTAT CCAAATTCGC TCGGTTGCTA AAAAGTACCC TAAAACTTGG ATACATTATA 1620
   TTTCTGCTGA GGAGGAAGAC TGGGACTATG CACCTTCAGT TCCTACCTCG GATAATGGAA 1680
   GTTATAAAAG CCAGTATCTG AGCAATGGTC CTCATCGGAT TGGTAGGAAA TATAAAAAAG 1740
   TCAGATTTAT AGCATACACA GATGAAACCT TTAAGACTCG TGAAACTATT CAGCATGAAT 1800
   CAGGACTCTT GGGACCTTTA CTTTATGGAG AAGTTGGAGA CACACTGTTG ATTATTTTTA 1860
   AGAATCAAGC AAGCCGACCA TATAACATTT ACCCTCATGG AATCACTGAT GTCAGTCCTC 1920
   TACATGCAAG GAGATTGCCA AGAGGTATAA AGCACGTGAA GGATTTGCCA ATTCATCCAG 1980
   GAGAGATATT CAAGTACAAG TGGACAGTTA CAGTAGAAGA TGGACCAACT AAATCAGATC 2040
   CACGGTGCCT GACCCGCTAT TATTCAAGTT TCATTAACCC TGAGAGAGAT CTAGCTTCAG 2100
   GACTGATTGG CCCTCTTCTC ATCTGCTACA AAGAATCTGT AGATCAAAGG GGAAACCAGA 2160
   TGATGTCAGA CAAAAGAAAT GTCATCCTGT TTTCTATATT TGATGAGAAC CAAAGCTGGT 2220
   ACATCACAGA GAACATGCAA CGCTTCCTCC CCAATGCAGC TAAAACACAG CCCCAGGACC 2280
   CTGGGTTCCA GGCCTCCAAC ATCATGCACA GCATCAATGG CTATGTTTTT GATAGCTTGG 2340
   AGTTGACAGT TTGTTTGCAT GAGGTGGCAT ACTGGCACAT TCTCAGTGTT GGAGCACAGA 2400
   CAGACTTCTT ATCTATCTTC TTCTCTGGAT ATACTTTCAA ACACAAAATG GTCTATGAAG 2460
   ATACACTTAC CCTGTTCCCA TTCTCAGGAG AAACTGTCTT TATGTCGATG GAAAACCCAG 2520
   GTCTATGGGT CTTGGGGTGT CATAATTCAG ACTTTCGGAA GAGAGGTATG ACAGCATTGC 2580
   TGAAAGTTTC TAGTTGTGAC AAGAGCACTA GTGATTATTA TGAAGAAATA TATGAAGATA 2640
   TTCCAACACA GTTGGTGAAT GAGAACAATG TCATTGATCC CAGAAGCTTC TTCCAGAATA 2700
   CAAATCATCC TAATACTAGG AAAAAGAAAT TCAAAGATTC CACAATTCCA AAAAATGATA 2760
   TGGAGAAGAT TGAGCCTCAG TTTGAAGAGA TAGCAGAGAT GCTTAAAGTA CAGAGTGTCT 2820
   CAGTTAGTGA CATGTTGATG CTCTTGGGAC AGAGTCATCC TACTCCACAT GGCTTATTTT 2880
   TATCAGATGG CCAAGAAGCC ATCTATGAGG CTATTCATGA TGATCATTCA CCAAATGCAA 2940
   TAGACAGCAA TGAAGGCCCA TCTAAAGTGA CCCAACTCAG GCCAGAATCC CATCACAGTG 3000
   AGAAAATAGT ATTTACTCCT CAGCCCGGCC TCCAGTTAAG ATCCAATAAA AGTTTGGAGA 3060
   CAACTATAGA AGTAAAGTGG AAGAAACTTG GTTTGCAAGT TTCTAGTTTG CCAAGTAATC 3120
   TAATGACTAC AACAATTCTG TCAGACAATT TGAAAGCAAC TTTTGAAAAG ACAGATTCTT 3180
   CAGGATTTCC AGATATGCCA GTTCACTCTA GTAGTAAATT AAGTACTACT GCATTTGGTA 3240
   AGAAAGCATA TTCCCTTGTT GGGTCTCATG TACCTTTAAA CGCGAGTGAA GAAAATAGTG 3300
   ATTCCAACAT ATTGGATTCA ACTTTAATGT ATAGTCAAGA AAGTTTACCA AGAGATAATA 3360
   TATTATCAAT AGAGAATGAT AGATTACTCA GAGAGAAGAG GTTTCATGGA ATTGCTTTAT 3420
   TGACCAAAGA TAATACTTTA TTCAAAGACA ATGTCTCCTT AATGAAAACA AACAAAACAT 3480
   ATAATCATTC AACAACTAAT GAAAAACTAC ACACTGAGAG CCCAACATCA ATTGAGAATA 3540
   GTACAACAGA CTTGCAAGAT GCCATATTAA AGGTCAATAG TGAGATTCAA GAAGTAACAG 3600
   CTTTGATTCA TGATGGAACA CTTTTAGGCA AAAATTCTAC ATATTTGAGA CTAAACCATA 3660
   TGCTAAATAG AACTACCTCA ACAAAAAATA AAGACATATT TCATAGAAAA GATGAAGATC 3720
   CTATTCCACA AGATGAAGAG AATACAATCA TGCCATTTTC CAAGATGTTG TTCTTGTCAG 3780
   AATCTTCAAA TTGGTTTAAA AAGACCAATG GAAATAATTC CTTGAACTCT GAGCAAGAAC 3840
   ATAGTCCAAA GCAATTAGTA TATTTAATGT TTAAAAAATA TGTAAAAAAT CAAAGTTTCT 3900
   TGTCAGAGAA AAATAAAGTC ACAGTAGAAC AGGATGGATT TACAAAGAAC ATAGGACTTA 3960
   AAGACATGGC TTTTCCACAT AATATGAGCA TATTTCTTAC CACTTTGTCT AACGTACATG 4020
   AAAATGGTAG GCACAATCAA GAAAAAAATA TTCAGGAAGA GATAGAGAAG GAAGCACTAA 4080
   TTGAAGAGAA AGTAGTTTTG CCCCAGGTGC ACGAAGCAAC TGGCTCTAAG AATTTCTTGA 4140
   AAGACATATT GATACTAGGC ACTAGGCAAA ATATAAGTTT ATATGAAGTA CATGTACCAG 4200
   TACTTCAAAA CATCACATCA ATAAACAATT CAACAAATAC AGTACAGATT CACATGGAGC 4260
   ATTTCTTTAA AAGAAGGAAG GACAAGGAAA CAAATTCAGA AGGCTTGGTA AATAAAACCA 4320
   GAGAAATGGT AAAAAACTAT CCAAGCCAGA AGAATATTAC TACTCAACGT AGTAAACGGG 4380
   CTTTGGGACA ATTCAGACTG TCAACTCAAT GGCTTAAAAC CATAAACTGT TCAACACAGT 4440
   GTATCATTAA ACAGATAGAC CACAGCAAGG AAATGAAAAA GTTCATTACT AAATCTTCCT 4500
   TATCAGATTC TTCTGTGATT AAAAGCACCA CTCAGACAAA TAGTTCTGAC TCACACATTG 4560
   TAAAAACATC AGCATTTCCA CCAATAGATC TCAAAAGGAG TCCATTCCAA AACAAATTTT 4620
   CTCATGTTCA AGCATCATCC TACATTTATG ACTTTAAGAC AAAAAGTTCA AGAATTCAAG 4680
   AAAGCAATAA TTTCTTAAAA GAAACCAAAA TAAATAACCC TTCTTTAGCC ATTCTACCAT 4740
   GGAATATGTT CATAGATCAA GGAAAATTTA CCTCCCCAGG GAAAAGTAAC ACAAACTCAG 4800
   TCACATATAA GAAACGTGAG AACATTATTT TCTTGAAACC AACTTTGCCT GAAGAATCTG 4860
   GCAAAATTGA ATTGCTTCCT CAAGTTTCCA TTCAAGAGGA AGAAATTTTA CCTACAGAAA 4920
   CTAGCCATGG ATCTCCTGGA CACTTGAATC TCATGAAAGA GGTCTTTCTT CAGAAAATAC 4980
   AGGGGCCTAC TAAATGGAAT AAAGCAAAGA GGCATGGAGA AAGTATAAAA GGTAAAACAG 5040
   AGAGCTCTAA AAATACTCGC TCAAAACTGC TAAATCATCA TGCTTGGGAT TATCATTATG 5100
   CTGCACAGAT ACCAAAAGAT ATGTGGAAAT CCAAAGAGAA GTCACCAGAA ATTATATCCA 5160
   TTAAGCAAGA GGACACCATT TTGTCTCTGA GGCCTCATGG AAACAGTCAT TCAATAGGGG 5220
   CAAATGAGAA ACAAAATTGG CCTCAAAGAG AAACCACTTG GGTAAAGCAA GGCCAAACTC 5280
   AAAGGACATG CTCTCAAATC CCACCAGTGT TGAAACGACA TCAAAGGGAA CTTAGTGCTT 5340
   TTCAATCAGA ACAAGAAGCA ACTGACTATG ATGATGCCAT CACCATTGAA ACAATCGAGG 5400
   ATTTTGACAT TTACAGTGAG GACATAAAGC AAGGTCCCCG CAGCTTTCAA CAGAAAACAA 5460
   GGCACTATTT TATTGCAGCT GTGGAACGAC TCTGGGACTA TGGGATGAGT ACATCTCATG 5520
   TTCTACGAAA TAGGTATCAA AGTGACAATG TACCTCAGTT CAAGAAAGTA GTTTTCCAGG 5580
   AATTTACTGA TGGCTCCTTT AGTCAGCCCT TATATCGTGG AGAATTAAAT GAACACCTGG 5640
   GGTTGTTGGG CCCATATATA AGAGCAGAAG TTGAAGACAA CATTATGGTA ACTTTCAAAA 5700
   ACCAGGCCTC CCGTCCCTAC TCCTTCTATT CTAGCCTCAT TTCTTATAAA GAAGATCAGA 5760
   GAGGAGAAGA ACCTAGAAGA AACTTTGTCA AGCCTAATGA AACCAAAATT TATTTTTGGA 5820
   AAGTACAACA TCATATGGCA CCCACAGAAG ATGAGTTTGA CTGCAAGGCC TGGGCTTATT 5880
   TCTCTGATGT TGATCTTGAA AGAGATATGC ACTCGGGATT AATTGGACCC CTTCTGATTT 5940
   GCCACGCGAA CACACTGAAT CCTGCTCATG GGAGACAAGT GTCAGTACAG GAATTTGCTC 6000
   TGCTTTTCAC TATCTTTGAT GAGACCAAGA GCTGGTACTT CACTGAAAAC GTGAAAAGGA 6060
   ACTGCAAGAC ACCCTGCAAT TTCCAGATGG AAGACCCCAC TTTGAAAGAG AATTATCGCT 6120
   TCCATGCAAT CAATGGTTAT GTAATGGATA CCCTACCAGG CTTAGTAATG GCTCAAGATC 6180
   AAAGGATTCG ATGGTATCTT CTCAGCATGG GCAACAATGA GAACATCCAA TCTATTCATT 6240
   TCAGTGGACA TGTTTTCACT GTACGGAAAA AAGAGGAGTA TAAAATGGCA GTGTACAACC 6300
   TCTACCCAGG TGTTTTTGAG ACTCTGGAAA TGATACCATC CAGAGCTGGA ATATGGCGAG 6360
   TAGAATGCCT TATTGGCGAG CACTTACAGG CTGGGATGAG CACTCTTTTT CTGGTGTACA 6420
   GCAAGCAGTG TCAGATTCCT CTTGGAATGG CTTCTGGAAG CATCCGTGAT TTCCAGATTA 6480
   CAGCTTCAGG ACATTATGGA CAGTGGGCCC CAAACCTGGC AAGACTTCAT TATTCCGGAT 6540
   CAATCAATGC CTGGAGTACC AAGGAGCCCT TTTCTTGGAT CAAGGTAGAT CTGTTGGCAC 6600
   CAATGATTGT TCATGGCATC AAGACTCAGG GTGCTCGTCA GAAATTTTCC AGCCTTTATA    6660
   TCTCTCAATT TATCATCATG TATAGCCTGG ATGGGAAGAA GTGGCTGAGT TATCAAGGAA    6720
   ATTCCACTGG AACCTTAATG GTTTTCTTTG GCAATGTGGA CTCATCTGGG ATTAAGCATA    6780
   ATAGTTTTAA TCCTCCAATT ATTGCTCGAT ATATCCGTTT GCACCCCACT CATTCTAGCA    6840
   TCCGTAGTAC TCTTCGCATG GAGTTGATGG GCTGTGATTT AAACAGTTGC AGCATACCAT    6900
   TGGGAATGGA AAGTAAAGTA ATATCAGATA CACAAATCAC TGCCTCATCC TACTTCACCA    6960
   ACATGTTTGC TACTTGGTCT CCTTCACAAG CTCGACTTCA CCTCCAGGGA AGGACTAATG    7020
   CCTGGCGACC TCAGGTGAAT GATCCAAAAC AATGGTTGCA AGTGGACTTA CAAAAGACAA    7080
   TGAAAGTCAC TGGAATAATA ACCCAGGGAG TGAAATCTCT CTTTACCAGC ATGTTTGTGA    7140
   AAGAGTTCCT TATTTCCAGC AGTCAAGATG GCCATCACTG GACTCAAATT TTATACAATG    7200
   GCAAGGTAAA GGTTTTTCAG GGGAATCAGG ACTCATCCAC ACCTATGATG AATTCTCTAG    7260
   ACCCACCATT ACTCACTCGC TATCTTCGAA TTCACCCCCA GATCTGGGAG CACCAAATTG    7320
   CTCTGAGGCT TGAGATTCTA GGATGTGAGG CCCAGCAGCA ATACTGAGGT AGCCTCTGCA    7380
   TCACCTGCTT ATTCCCCTTC CTCAGCTCAA AGATTGTCTT AATGTTTTAT TGCTGTGAAG    7440
   AGACACTATG ACCATGGCAA CTCTTTATAA AATAAAGCAT TTAATCAGGG CTT           7493
(2) INFORMATION FOR SEQ ID NO:6:
   (i) SEQUENCE CHARACTERISTICS:
      (A) LENGTH: 2319 amino acids
      (B) TYPE: amino acid
      (C) STRANDEDNESS: single
      (D) TOPOLOGY: linear
   (ii) MOLECULE TYPE: protein
   (iii) HYPOTHETICAL: YES
   (iv) ANTI-SENSE: NO
   (v) FRAGMENT TYPE: N-terminal
   (vi) ORIGINAL SOURCE:
      (A) ORGANISM: Mus musculus
   (x) PUBLICATION INFORMATION:
      (A) AUTHORS: Elder, F.
         Lakich, D.
         Gitschier, J.
      (B) TITLE: Sequence of the Murine Factor VIII cDNA.
      (C) JOURNAL: Genomics
      (D) VOLUME: 16
      (F) PAGES: 374-379
      (G) DATE: 1993
      (K) RELEVANT RESIDUES IN SEQ ID NO:6: FROM 1 TO 2319
   (xi) SEQUENCE DESCRIPTION: SEQ ID NO:6:
   Met Gln Ile Ala Leu Phe Ala Cys Phe Phe Leu Ser Leu Phe Asn Phe
   1               5                   10                  15
   Cys Ser Ser Ala Ile Arg Arg Tyr Tyr Leu Gly Ala Val Glu Leu Ser
               20                  25                  30
   Trp Asn Tyr Ile Gln Ser Asp Leu Leu Ser Val Leu His Thr Asp Ser
           35                  40                  45
   Arg Phe Leu Pro Arg Met Ser Thr Ser Phe Pro Phe Asn Thr Ser Ile
       50                  55                  60
   Met Tyr Lys Lys Thr Val Phe Val Glu Tyr Lys Asp Gln Leu Phe Asn
   65                  70                  75                  80
   Ile Ala Lys Pro Arg Pro Pro Trp Met Gly Leu Leu Gly Pro Thr Ile
                   85                  90                  95
   Trp Thr Glu Val His Asp Thr Val Val Ile Thr Leu Lys Asn Met Ala
               100                 105                 110
   Ser His Pro Val Ser Leu His Ala Val Gly Val Ser Tyr Trp Lys Ala
           115                 120                 125
   Ser Glu Gly Asp Glu Tyr Glu Asp Gln Thr Ser Gln Met Glu Lys Glu
       130                 135                 140
   Asp Asp Lys Val Phe Pro Gly Glu Ser His Thr Tyr Val Trp Gln Val
   145                 150                 155                 160
   Leu Lys Glu Asn Gly Pro Met Ala Ser Asp Pro Pro Cys Leu Thr Tyr
                   165                 170                 175
   Ser Tyr Met Ser His Val Asp Leu Val Lys Asp Leu Asn Ser Gly Leu
               180                 185                 190
   Ile Gly Ala Leu Leu Val Cys Lys Glu Gly Ser Leu Ser Lys Glu Arg
           195                 200                 205
   Thr Gln Met Leu Tyr Gln Phe Val Leu Leu Phe Ala Val Phe Asp Glu
       210                 215                 220
   Gly Lys Ser Trp His Ser Glu Thr Asn Asp Ser Tyr Thr Gln Ser Met
   225                 230                 235                 240
   Asp Ser Ala Ser Ala Arg Asp Trp Pro Lys Met His Thr Val Asn Gly
   245 250 255
   Tyr Val Asn Arg Ser Leu Pro Gly Leu Ile Gly Cys His Arg Lys Ser
   260 265 270
   Val Tyr Trp His Val Ile Gly Met Gly Thr Thr Pro Glu Ile His Ser
   275 280 285
   Ile Phe Leu Glu Gly His Thr Phe Phe Val Arg Asn His Arg Gln Ala
   290 295 300
   Ser Leu Glu Ile Ser Pro Ile Thr Phe Leu Thr Ala Gln Thr Leu Leu
   305 310 315 320
   Ile Asp Leu Gly Gln Phe Leu Leu Phe Cys His Ile Ser Ser His Lys
   325 330 335
   His Asp Gly Met Glu Ala Tyr Val Lys Val Asp Ser Cys Pro Glu Glu
   340 345 350
   Ser Gln Trp Gln Lys Lys Asn Asn Asn Glu Glu Met Glu Asp Tyr Asp
   355 360 365
   Asp Asp Leu Tyr Ser Glu Met Asp Met Phe Thr Leu Asp Tyr Asp Ser
   370 375 380
   Ser Pro Phe Ile Gln Ile Arg Ser Val Ala Lys Lys Tyr Pro Lys Thr
   385 390 395 400
   Trp Ile His Tyr Ile Ser Ala Glu Glu Glu Asp Trp Asp Tyr Ala Pro
   405 410 415
   Ser Val Pro Thr Ser Asp Asn Gly Ser Tyr Lys Ser Gln Tyr Leu Ser
   420 425 430
   Asn Gly Pro His Arg Ile Gly Arg Lys Tyr Lys Lys Val Arg Phe Ile
           435                 440                 445
   Ala Tyr Thr Asp Glu Thr Phe Lys Thr Arg Glu Thr Ile Gln His Glu
       450                 455                 460
   Ser Gly Leu Leu Gly Pro Leu Leu Tyr Gly Glu Val Gly Asp Thr Leu
   465                 470                 475                 480
   Leu Ile Ile Phe Lys Asn Gln Ala Ser Arg Pro Tyr Asn Ile Tyr Pro
                   485                 490                 495
   His Gly Ile Thr Asp Val Ser Pro Leu His Ala Arg Arg Leu Pro Arg
               500                 505                 510
   Gly Ile Lys His Val Lys Asp Leu Pro Ile His Pro Gly Glu Ile Phe
           515                 520                 525
   Lys Tyr Lys Trp Thr Val Thr Val Glu Asp Gly Pro Thr Lys Ser Asp
       530                 535                 540
   Pro Arg Cys Leu Thr Arg Tyr Tyr Ser Ser Phe Ile Asn Pro Glu Arg
   545                 550                 555                 560
   Asp Leu Ala Ser Gly Leu Ile Gly Pro Leu Leu Ile Cys Tyr Lys Glu
                   565                 570                 575
   Ser Val Asp Gln Arg Gly Asn Gln Met Met Ser Asp Lys Arg Asn Val
               580                 585                 590
   Ile Leu Phe Ser Ile Phe Asp Glu Asn Gln Ser Trp Tyr Ile Thr Glu
           595                 600                 605
   Asn Met Gln Arg Phe Leu Pro Asn Ala Ala Lys Thr Gln Pro Gln Asp
       610                 615                 620
   Pro Gly Phe Gln Ala Ser Asn Ile Met His Ser Ile Asn Gly Tyr Val
   625 630 635 640
   Phe Asp Ser Leu Glu Leu Thr Val Cys Leu His Glu Val Ala Tyr Trp
   645 650 655
   His Ile Leu Ser Val Gly Ala Gln Thr Asp Phe Leu Ser Ile Phe Phe
   660 665 670
   Ser Gly Tyr Thr Phe Lys His Lys Met Val Tyr Glu Asp Thr Leu Thr
   675 680 685
   Leu Phe Pro Phe Ser Gly Glu Thr Val Phe Met Ser Met Glu Asn Pro
   690 695 700
   Gly Leu Trp Val Leu Gly Cys His Asn Ser Asp Phe Arg Lys Arg Gly
   705 710 715 720
   Met Thr Ala Leu Leu Lys Val Ser Ser Cys Asp Lys Ser Thr Ser Asp
   725 730 735
   Tyr Tyr Glu Glu Ile Tyr Glu Asp Ile Pro Thr Gln Leu Val Asn Glu
   740 745 750
   Asn Asn Val Ile Asp Pro Arg Ser Phe Phe Gln Asn Thr Asn His Pro
   755 760 765
   Asn Thr Arg Lys Lys Lys Phe Lys Asp Ser Thr Ile Pro Lys Asn Asp
   770 775 780
   Met Glu Lys Ile Glu Pro Gln Phe Glu Glu Ile Ala Glu Met Leu Lys
   785 790 795 800
   Val Gln Ser Val Ser Val Ser Asp Met Leu Met Leu Leu Gly Gln Ser
   805 810 815
   His Pro Thr Pro His Gly Leu Phe Leu Ser Asp Gly Gln Glu Ala Ile
               820                 825                 830
   Tyr Glu Ala Ile His Asp Asp His Ser Pro Asn Ala Ile Asp Ser Asn
           835                 840                 845
   Glu Gly Pro Ser Lys Val Thr Gln Leu Arg Pro Glu Ser His His Ser
       850                 855                 860
   Glu Lys Ile Val Phe Thr Pro Gln Pro Gly Leu Gln Leu Arg Ser Asn
   865                 870                 875                 880
   Lys Ser Leu Glu Thr Thr Ile Glu Val Lys Trp Lys Lys Leu Gly Leu
                   885                 890                 895
   Gln Val Ser Ser Leu Pro Ser Asn Leu Met Thr Thr Thr Ile Leu Ser
               900                 905                 910
   Asp Asn Leu Lys Ala Thr Phe Glu Lys Thr Asp Ser Ser Gly Phe Pro
           915                 920                 925
   Asp Met Pro Val His Ser Ser Ser Lys Leu Ser Thr Thr Ala Phe Gly
       930                 935                 940
   Lys Lys Ala Tyr Ser Leu Val Gly Ser His Val Pro Leu Asn Ala Ser
   945                 950                 955                 960
   Glu Glu Asn Ser Asp Ser Asn Ile Leu Asp Ser Thr Leu Met Tyr Ser
                   965                 970                 975
   Gln Glu Ser Leu Pro Arg Asp Asn Ile Leu Ser Ile Glu Asn Asp Arg
               980                 985                 990
   Leu Leu Arg Glu Lys Arg Phe His Gly Ile Ala Leu Leu Thr Lys Asp
           995                 1000                1005
   Asn Thr Leu Phe Lys Asp Asn Val Ser Leu Met Lys Thr Asn Lys Thr
       1010                1015                1020
   Tyr Asn His Ser Thr Thr Asn Glu Lys Leu His Thr Glu Ser Pro Thr
   1025                1030                1035                1040
   Ser Ile Glu Asn Ser Thr Thr Asp Leu Gln Asp Ala Ile Leu Lys Val
                   1045                1050                1055
   Asn Ser Glu Ile Gln Glu Val Thr Ala Leu Ile His Asp Gly Thr Leu
               1060                1065                1070
   Leu Gly Lys Asn Ser Thr Tyr Leu Arg Leu Asn His Met Leu Asn Arg
           1075                1080                1085
   Thr Thr Ser Thr Lys Asn Lys Asp Ile Phe His Arg Lys Asp Glu Asp
       1090                1095                1100
   Pro Ile Pro Gln Asp Glu Glu Asn Thr Ile Met Pro Phe Ser Lys Met
   1105                1110                1115                1120
   Leu Phe Leu Ser Glu Ser Ser Asn Trp Phe Lys Lys Thr Asn Gly Asn
                   1125                1130                1135
   Asn Ser Leu Asn Ser Glu Gln Glu His Ser Pro Lys Gln Leu Val Tyr
               1140                1145                1150
   Leu Met Phe Lys Lys Tyr Val Lys Asn Gln Ser Phe Leu Ser Glu Lys
           1155                1160                1165
   Asn Lys Val Thr Val Glu Gln Asp Gly Phe Thr Lys Asn Ile Gly Leu
       1170                1175                1180
   Lys Asp Met Ala Phe Pro His Asn Met Ser Ile Phe Leu Thr Thr Leu
   1185                1190                1195                1200
   Ser Asn Val His Glu Asn Gly Arg His Asn Gln Glu Lys Asn Ile Gln
   1205 1210 1215
   Glu Glu Ile Glu Lys Glu Ala Leu Ile Glu Glu Lys Val Val Leu Pro
   1220 1225 1230
   Gln Val His Glu Ala Thr Gly Ser Lys Asn Phe Leu Lys Asp Ile Leu
   1235 1240 1245
   Ile Leu Gly Thr Arg Gln Asn Ile Ser Leu Tyr Glu Val His Val Pro
   1250 1255 1260
   Val Leu Gln Asn Ile Thr Ser Ile Asn Asn Ser Thr Asn Thr Val Gln
   1265 1270 1275 1280
   Ile His Met Glu His Phe Phe Lys Arg Arg Lys Asp Lys Glu Thr Asn
   1285 1290 1295
   Ser Glu Gly Leu Val Asn Lys Thr Arg Glu Met Val Lys Asn Tyr Pro
   1300 1305 1310
   Ser Gln Lys Asn Ile Thr Thr Gln Arg Ser Lys Arg Ala Leu Gly Gln
   1315 1320 1325
   Phe Arg Leu Ser Thr Gln Trp Leu Lys Thr Ile Asn Cys Ser Thr Gln
   1330 1335 1340
   Cys Ile Ile Lys Gln Ile Asp His Ser Lys Glu Met Lys Lys Phe Ile
   1345 1350 1355 1360
   Thr Lys Ser Ser Leu Ser Asp Ser Ser Val Ile Lys Ser Thr Thr Gln
   1365 1370 1375
   Thr Asn Ser Ser Asp Ser His Ile Val Lys Thr Ser Ala Phe Pro Pro
   1380 1385 1390
   Ile Asp Leu Lys Arg Ser Pro Phe Gln Asn Lys Phe Ser His Val Gln
   1395 1400 1405
   Ala Ser Ser Tyr Ile Tyr Asp Phe Lys Thr Lys Ser Ser Arg Ile Gln
   1410 1415 1420
   Glu Ser Asn Asn Phe Leu Lys Glu Thr Lys Ile Asn Asn Pro Ser Leu
   1425 1430 1435 1440
   Ala Ile Leu Pro Trp Asn Met Phe Ile Asp Gln Gly Lys Phe Thr Ser
   1445 1450 1455
   Pro Gly Lys Ser Asn Thr Asn Ser Val Thr Tyr Lys Lys Arg Glu Asn
   1460 1465 1470
   Ile Ile Phe Leu Lys Pro Thr Leu Pro Glu Glu Ser Gly Lys Ile Glu
   1475 1480 1485
   Leu Leu Pro Gln Val Ser Ile Gln Glu Glu Glu Ile Leu Pro Thr Glu
   1490 1495 1500
   Thr Ser His Gly Ser Pro Gly His Leu Asn Leu Met Lys Glu Val Phe
   1505 1510 1515 1520
   Leu Gln Lys Ile Gln Gly Pro Thr Lys Trp Asn Lys Ala Lys Arg His
   1525 1530 1535
   Gly Glu Ser Ile Lys Gly Lys Thr Glu Ser Ser Lys Asn Thr Arg Ser
   1540 1545 1550
   Lys Leu Leu Asn His His Ala Trp Asp Tyr His Tyr Ala Ala Gln Ile
   1555 1560 1565
   Pro Lys Asp Met Trp Lys Ser Lys Glu Lys Ser Pro Glu Ile Ile Ser
   1570 1575 1580
   Ile Lys Gln Glu Asp Thr Ile Leu Ser Leu Arg Pro His Gly Asn Ser
   1585 1590 1595 1600
   His Ser Ile Gly Ala Asn Glu Lys Gln Asn Trp Pro Gln Arg Glu Thr
   1605 1610 1615
   Thr Trp Val Lys Gln Gly Gln Thr Gln Arg Thr Cys Ser Gln Ile Pro
   1620 1625 1630
   Pro Val Leu Lys Arg His Gln Arg Glu Leu Ser Ala Phe Gln Ser Glu
   1635 1640 1645
   Gln Glu Ala Thr Asp Tyr Asp Asp Ala Ile Thr Ile Glu Thr Ile Glu
   1650 1655 1660
   Asp Phe Asp Ile Tyr Ser Glu Asp Ile Lys Gln Gly Pro Arg Ser Phe
   1665 1670 1675 1680
   Gln Gln Lys Thr Arg His Tyr Phe Ile Ala Ala Val Glu Arg Leu Trp
   1685 1690 1695
   Asp Tyr Gly Met Ser Thr Ser His Val Leu Arg Asn Arg Tyr Gln Ser
   1700 1705 1710
   Asp Asn Val Pro Gln Phe Lys Lys Val Val Phe Gln Glu Phe Thr Asp
   1715 1720 1725
   Gly Ser Phe Ser Gln Pro Leu Tyr Arg Gly Glu Leu Asn Glu His Leu
   1730 1735 1740
   Gly Leu Leu Gly Pro Tyr Ile Arg Ala Glu Val Glu Asp Asn Ile Met
   1745 1750 1755 1760
   Val Thr Phe Lys Asn Gln Ala Ser Arg Pro Tyr Ser Phe Tyr Ser Ser
   1765 1770 1775
   Leu Ile Ser Tyr Lys Glu Asp Gln Arg Gly Glu Glu Pro Arg Arg Asn
   1780 1785 1790
   Phe Val Lys Pro Asn Glu Thr Lys Ile Tyr Phe Trp Lys Val Gln His
   1795 1800 1805
   His Met Ala Pro Thr Glu Asp Glu Phe Asp Cys Lys Ala Trp Ala Tyr
   1810 1815 1820
   Phe Ser Asp Val Asp Leu Glu Arg Asp Met His Ser Gly Leu Ile Gly
   1825 1830 1835 1840
   Pro Leu Leu Ile Cys His Ala Asn Thr Leu Asn Pro Ala His Gly Arg
   1845 1850 1855
   Gln Val Ser Val Gln Glu Phe Ala Leu Leu Phe Thr Ile Phe Asp Glu
   1860 1865 1870
   Thr Lys Ser Trp Tyr Phe Thr Glu Asn Val Lys Arg Asn Cys Lys Thr
   1875 1880 1885
   Pro Cys Asn Phe Gln Met Glu Asp Pro Thr Leu Lys Glu Asn Tyr Arg
   1890 1895 1900
   Phe His Ala Ile Asn Gly Tyr Val Met Asp Thr Leu Pro Gly Leu Val
   1905 1910 1915 1920
   Met Ala Gln Asp Gln Arg Ile Arg Trp Tyr Leu Leu Ser Met Gly Asn
   1925 1930 1935
   Asn Glu Asn Ile Gln Ser Ile His Phe Ser Gly His Val Phe Thr Val
   1940 1945 1950
   Arg Lys Lys Glu Glu Tyr Lys Met Ala Val Tyr Asn Leu Tyr Pro Gly
   1955 1960 1965
   Val Phe Glu Thr Leu Glu Met Ile Pro Ser Arg Ala Gly Ile Trp Arg
   1970 1975 1980
   Val Glu Cys Leu Ile Gly Glu His Leu Gln Ala Gly Met Ser Thr Leu
   1985 1990 1995 2000
   Phe Leu Val Tyr Ser Lys Gln Cys Gln Ile Pro Leu Gly Met Ala Ser
   2005 2010 2015
   Gly Ser Ile Arg Asp Phe Gln Ile Thr Ala Ser Gly His Tyr Gly Gln
   2020 2025 2030
   Trp Ala Pro Asn Leu Ala Arg Leu His Tyr Ser Gly Ser Ile Asn Ala
   2035 2040 2045
   Trp Ser Thr Lys Glu Pro Phe Ser Trp Ile Lys Val Asp Leu Leu Ala
   2050 2055 2060
   Pro Met Ile Val His Gly Ile Lys Thr Gln Gly Ala Arg Gln Lys Phe
   2065 2070 2075 2080
   Ser Ser Leu Tyr Ile Ser Gln Phe Ile Ile Met Tyr Ser Leu Asp Gly
   2085 2090 2095
   Lys Lys Trp Leu Ser Tyr Gln Gly Asn Ser Thr Gly Thr Leu Met Val
   2100 2105 2110
   Phe Phe Gly Asn Val Asp Ser Ser Gly Ile Lys His Asn Ser Phe Asn
   2115 2120 2125
   Pro Pro Ile Ile Ala Arg Tyr Ile Arg Leu His Pro Thr His Ser Ser
   2130 2135 2140
   Ile Arg Ser Thr Leu Arg Met Glu Leu Met Gly Cys Asp Leu Asn Ser
   2145 2150 2155 2160
   Cys Ser Ile Pro Leu Gly Met Glu Ser Lys Val Ile Ser Asp Thr Gln
                   2165                2170                2175
   Ile Thr Ala Ser Ser Tyr Phe Thr Asn Met Phe Ala Thr Trp Ser Pro
               2180                2185                2190
   Ser Gln Ala Arg Leu His Leu Gln Gly Arg Thr Asn Ala Trp Arg Pro
           2195                2200                2205
   Gln Val Asn Asp Pro Lys Gln Trp Leu Gln Val Asp Leu Gln Lys Thr
       2210                2215                2220
   Met Lys Val Thr Gly Ile Ile Thr Gln Gly Val Lys Ser Leu Phe Thr
   2225                2230                2235                2240
   Ser Met Phe Val Lys Glu Phe Leu Ile Ser Ser Ser Gln Asp Gly His
                   2245                2250                2255
   His Trp Thr Gln Ile Leu Tyr Asn Gly Lys Val Lys Val Phe Gln Gly
               2260                2265                2270
   Asn Gln Asp Ser Ser Thr Pro Met Met Asn Ser Leu Asp Pro Pro Leu
           2275                2280                2285
   Leu Thr Arg Tyr Leu Arg Ile His Pro Gln Ile Trp Glu His Gln Ile
       2290                2295                2300
   Ala Leu Arg Leu Glu Ile Leu Gly Cys Glu Ala Gln Gln Gln Tyr
   2305                2310                2315
(2) INFORMATION FOR SEQ ID NO:7:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: Other nucleic acid
   (iii) HYPOTHETICAL: NO
   (iv) ANTI-SENSE: NO
   (xi) SEQUENCE DESCRIPTION: SEQ ID NO:7:
   CCTTCCTTTA TCCAAATACG TAGATCAAGA GGAAATTGAC        40
(2) INFORMATION FOR SEQ ID NO:8:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: Other nucleic acid
   (iii) HYPOTHETICAL: NO
   (iv) ANTI-SENSE: NO
   (xi) SEQUENCE DESCRIPTION: SEQ ID NO:8:
   GTAGCGTTGC CAAGAAGCAC CCTAAGACG 29
(2) INFORMATION FOR SEQ ID NO:9:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: Other nucleic acid
   (iii) HYPOTHETICAL: NO
   (iv) ANTI-SENSE: NO
   (xi) SEQUENCE DESCRIPTION: SEQ ID NO:9:
   GAAGAGTAGT ACGAGTTATT TCTCTGGGTT CAATGAC 37
(2) INFORMATION FOR SEQ ID NO:10:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: Other nucleic acid
   (iii) HYPOTHETICAL: NO
   (iv) ANTI-SENSE: NO
   (xi) SEQUENCE DESCRIPTION: SEQ ID NO:10:
   CCTTTATCCA AATACGTAGC GTTTGCCAAG AAG 33
(2) INFORMATION FOR SEQ ID NO:11:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: Other nucleic acid
   (iii) HYPOTHETICAL: NO
   (iv) ANTI-SENSE: NO
   (xi) SEQUENCE DESCRIPTION: SEQ ID NO:11:
   AARCAYCCNA ARACNTGGG                                    19
(2) INFORMATION FOR SEQ ID NO:12:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: Other nucleic acid
   (iii) HYPOTHETICAL: NO
   (iv) ANTI-SENSE: NO
   (xi) SEQUENCE DESCRIPTION: SEQ ID NO:12:
   GCTCGCACTA GGGGGTCTTG AATTC                             25

## Description

The government has rights in this invention arising from National Institutes of Health Grant Nos. HL40921, HL46215, and HL36094 that partially funded the research leading to this invention.

### Background of the Invention

This invention relates generally to a hybrid factor VIII having human and animal factor VIII amino acid sequence and methods of preparation and use thereof.

Blood clotting begins when platelets adhere to the cut wall of an injured blood vessel at a lesion site. Subsequently, in a cascade of enzymatically regulated reactions, soluble fibrinogen molecules are converted by the enzyme thrombin to insoluble strands of fibrin that hold the platelets together in a thrombus. At each step in the cascade, a protein precursor is converted to a protease that cleaves the next protein precursor in the series. Cofactors are required at most of the steps. In its active form, the protein factor VIII is a cofactor that is required for the activation of factor X by the protease, activated factor IX.

Factor VIII or antihemophilic factor was noticed in plasma and named in the 1930s. In the 1940s, a deficiency in factor VIII was associated with the clotting disorder hemophilia A. Factor VIII was found to be X-linked and was hypothesized to be a protein. Work involving bovine, human, and porcine plasma identified factor VIII as a protein in the 1980s, though its definitive cellular source remains uncertain.

Precisely how factor VIII functions in blood coagulation is unknown. It is known that factor VIII is activated to factor VIIIa proteolytically by thrombin or factor Xa. In combination with calcium and phospholipid, factor VIIIa makes factor IXa a more efficient activator of factor X by an unknown mechanism.

People deficient in factor VIII or having antibodies against factor VIII who are not treated with factor VIII suffer uncontrolled internal bleeding that may cause a range of serious symptoms, from inflammatory reactions in joints to early death. Severe hemophiliacs, who number about 10,000 in the United States, can be treated with infusion of factor VIII, which will restore the blood's normal clotting ability if administered with sufficient frequency and concentration. The classic definition of factor VIII, in fact, is that substance present in normal blood plasma that corrects the clotting defect in plasma derived from individuals with hemophilia A.

Several preparations of human plasma-derived factor VIII of varying degrees of purity are available commercially for the treatment of hemophilia A. These include a partially-purified factor VIII derived from the pooled blood of many donors that is heat- and detergent-treated for viruses but contains a significant level of antigenic proteins; a monoclonal antibody-purified factor VIII that has lower levels of antigenic impurities and viral contamination; and recombinant human factor VIII, clinical trials for which are underway. Additionally, a preparation of partially-purified porcine factor VIII is available to treat patients with inhibitors to human factor VIII, i.e., those who have circulating antibody molecules that bind and neutralize human factor VIII.

Hemophiliacs require daily replacement of factor VIII to prevent the deforming hemophilic arthropathy that occurs after many years of recurrent hemorrhages into the joints. However, supplies of factor VIII concentrates have never been plentiful enough for treating hemophiliacs adequately because of problems in commercial production and therapeutic use. For example, the commonly used plasma-derived is difficult to isolate and purify, is immunogenic, and requires treatment to remove the risk of infectivity from AIDS and hepatitis viruses. Recombinant human factor VIII may lessen the latter two problems. Porcine factor VIII may also present an alternative, since human factor VIII is unstable at physiologic concentrations and pH, is present in blood at an extremely low concentration (0.2 *µ*g/ml plasma), and its specific clotting activity is low, compared with porcine factor VIII.

Since many inhibitors of human factor VIII react less strongly with porcine factor VIII, porcine factor VIII is currently used to correct factor VIII deficiency in patients under conditions in which they do not respond to infusions of human factor VIII. A limitation of porcine factor VIII is the development of inhibitory antibodies to it after one or more infusions.

The problems associated with the commonly used, commercially available, plasma-derived factor VIII have stimulated significant interest in the development of a better factor VIII product. There is a need for a more potent factor VIII molecule so that more units of clotting activity can be delivered per molecule; a factor VIII molecule that is stable at a selected pH and physiologic concentration; a factor VIII molecule that is less apt to produce inhibitory antibodies; and a factor VIII molecule that evades immune detection in patients who have already acquired antibodies to human factor VIII.

WO-A-9320093 describes the discovery of hybrid human/porcine factor VIII molecules having coagulant activity, in which elements of the factor VIII molecule of one species are substituted for corresponding elements of the factor VIII molecule of the other species. WO-A-9411503 describes hybrid human/animal factor VIII molecules, in which elements of the factor VIII molecule of one species are substituted for corresponding elements of the factor VIII molecule of the other species.

It is therefore an object of the present invention to provide a factor VIII that corrects hemophilia in a patient deficient in factor VIII or having inhibitors of human factor VIII.

It is a further object of the present invention to provide methods for treatment of hemophiliacs.

It is still another object of the present invention to provide a factor VIII that is stable at a selected pH and physiologic concentration.

### Summary of the Invention

A hybrid factor VIII with coagulant activity including in one embodiment factor VIII amino acid sequence derived from human and pig or other non-human mammal (referred to herein as "animal"); or in a second embodiment including factor VIII amino acid sequence derived from human or animal or both and amino acid sequence not derived from factor VIII, preferably substituted in an antigenic region of the factor VIII, is described. This hybrid factor VIII molecule is produced by isolation and recombination of human and animal factor VIII subunits or domains; or by genetic engineering of the human and animal factor VIII genes.

In the preferred embodiment, recombinant DNA methods are used to substitute elements of animal factor VIII for the corresponding elements of human factor VIII, resulting in hybrid human/animal factor VIII molecules. In another embodiment, recombinant DNA methods are used to replace one or more amino acids in the human or animal factor VIII or in a hybrid of two species with amino acids that do not have sequence identity to factor VIII, preferably a sequence of amino acids that is non-immunoreactive with naturally occurring inhibitory antibodies to factor VIII. An example of an amino acid sequence that can be used to replace particularly immunogenic epitopes is a sequence of alanine residues.

In another embodiment, subunits of factor VIII are isolated and purified from human or animal plasma, and hybrid human/animal factor VIII is produced either by mixture of animal heavy chain subunits with human light chain subunits or by mixture of human heavy chain subunits with animal light chain subunits, thereby producing human light chain/animal heavy chain and human heavy chain/animal light chain hybrid molecules. These hybrid molecules are isolated by ion exchange chromatography.

Alternatively, one or more domains or partial domains of factor VIII are isolated and purified from human or animal plasma, and hybrid human/animal factor VIII is produced by mixture of domains or partial domains from one species with domains or partial domains of the second species. Hybrid molecules can be isolated by ion exchange chromatography.

Methods for preparing highly purified hybrid factor VIII are described having the steps of: (a) isolation of subunits of plasma-derived human factor VIII and subunits of plasma-derived animal factor VIII, followed by reconstitution of coagulant activity by mixture of human and animal subunits, followed by isolation of hybrid human/animal factor VIII by ion exchange chromatography; (b) isolation of domains or partial domains of plasma-derived human factor VIII and domains or partial domains of plasma-derived animal factor VIII, followed by reconstitution of coagulant activity by mixture of human and animal domains, followed by isolation of hybrid human/animal factor VIII by ion exchange chromatography; (c) construction of domains or partial domains of animal factor VIII by recombinant DNA technology, followed by exchange of domains of animal and human factor VIII to produce hybrid human/animal factor VIII with coagulant activity; (d) creation of hybrid human/animal factor VIII by replacement of specific amino acid residues of human factor VIII with the animal factor VIII amino acid residues having sequence identity to the replaced human amino acids by site-directed mutagenesis; or (e) creation of a hybrid factor VIII molecule having human or animal amino acid sequence or both, in which specific amino acid residues of the factor VIII are replaced with amino acid residues not having sequence identity to factor VIII by site-directed mutagenesis.

Some species of hybrid factor VIII have specific activity greater than human factor VIII and equal to or slightly higher than porcine factor VIII. Some species of hybrid factor VIII have immunoreactivity with inhibitory antibodies to factor VIII equal to or less than human or porcine factor VIII.

### Brief Description of the Drawings

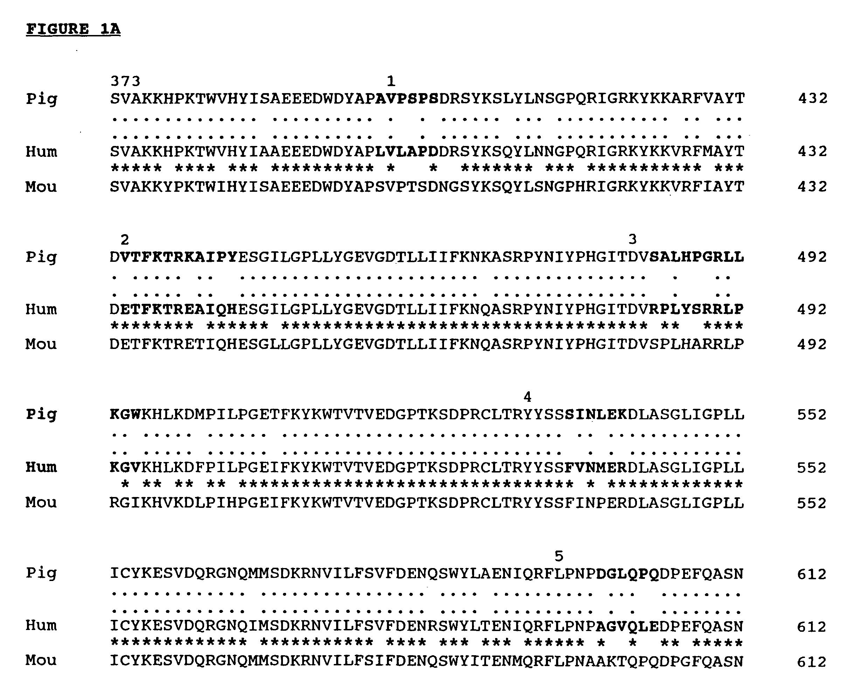

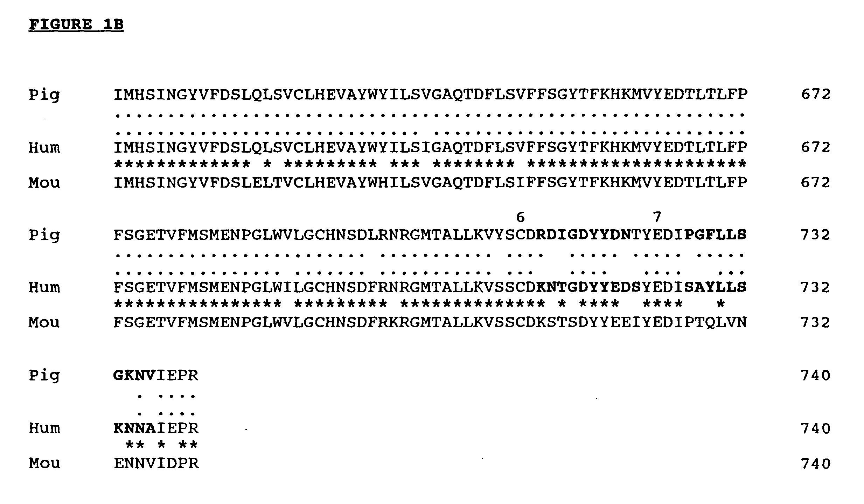

Figure 1A and 1B is an amino acid sequence alignment of human, mouse, and porcine factor VIII A2 domains, in which residue numbering begins at position 373 with respect to the full length sequence of human factor VIII (SEQ ID NO: 2).

### Detailed Description of the Invention

### - Definitions

Unless otherwise specified or indicated, as used herein, "hybrid factor VIII" or "hybrid protein" denotes any functional factor VIII protein molecule with (1) amino acid sequence derived from both human and porcine (human/porcine) or other non-human mammalian (human/non-porcine mammalian) factor VIII; (2) amino acid sequence derived from two different non-human mammalian species (animal-1/animal-2, porcine/non-human, non-porcine mammal), such as pig and mouse; and (3) amino acid sequence derived from hybrid, human, or animal factor VIII into which amino acid sequence having no known sequence identity to factor VIII is substituted. As used herein, "mammalian factor VIII" includes factor VIII with amino acid sequence derived from any non-human mammal, unless otherwise specified. "Animal", as used herein, refers to pig and other non-human mammals. Hybrid human/porcine factor VIII has coagulation activity in a human factor VIII assay. This activity, as well as that of other hybrid factor VIII, may be less than, equal to, or greater than that of either plasma-derived or recombinant human factor VIII. In some embodiments, this hybrid factor VIII is not cross-reactive or is less cross-reactive with all naturally occurring inhibitory factor VIII antibodies than human or porcine factor VIII.

This hybrid factor VIII can be made (1) by substitution of isolated, plasma-derived animal subunits or human subunits (heavy or light chains) for corresponding human subunits or animal subunits; (2) by substitution of human domains or animal domains (A1, A2, A3, B, C1, and C2) for corresponding animal domains or human domains; (3) by substitution of parts of human domains or animal domains for parts of animal domains or human domains; (4) by substitution of one or more human or animal specific amino acid residue(s) for the corresponding animal or human specific amino acid residue(s); or (5) by substitution of one or more specific amino acid residue(s) in human, animal, or hybrid factor VIII with amino acid sequence that has no known sequence identity to factor VIII. A fusion protein is the product of a hybrid gene in which the coding sequence for one protein is extensively altered, for example, by fusing part of it to the coding sequence for a second protein from a different gene to produce a hybrid gene that encodes the fusion protein. As used herein, a fusion protein is a subset of the hybrid protein described in this application.

"Corresponding amino acids" are those present at a site in a factor VIII molecule that have the same structure and/or function as a site in another factor VIII molecule, although the amino acid residue number may not be identical.

"Specific activity," as used herein, refers to the activity that will correct the coagulation defect of human factor VIII deficient plasma. Specific activity is measured in units of clotting activity per milligram total factor VIII protein in a standard assay in which the clotting time of human factor VIII deficient plasma is compared to that of normal human plasma. One unit of factor VIII activity is the activity present in one milliliter of normal human plasma. In the assay, the shorter the time for clot formation, the greater the activity of the factor VIII being assayed.

The human factor VIII cDNA nucleotide sequence is shown in SEQ ID NO:1. The human factor VIII predicted amino acid sequence is shown in SEQ ID NO:2. In a factor VIII molecule, a "domain" as used herein is a continuous sequence of amino acids that are defined by internal amino acid sequence identity and sites of proteolytic cleavage by thrombin. Unless otherwise specified, factor VIII domains include the following amino acid residues, when the sequences are aligned with the human amino acid sequence (SEQ ID NO:2): A1, residues 1-372; A2, residues 373-740; B, residues 741-1648; A3, residues 1690-2032; C1, residues 2033-2172; C2, residues 2173-2332. The A3-C1-C2 sequence includes residues 1690-2332. The remaining sequence, residues 1649-1689, is usually referred to as the factor VIII light chain activation peptide. A "partial domain" as used herein is a continuous sequence of amino acids containing part of a domain.

As used herein, a "hybrid human/animal factor VIII equivalent" or "hybrid factor VIII equivalent" is an active factor VIII molecule wherein (1) one or more specific amino acid residues in the human, animal, or hybrid factor VIII that forms an epitope which is immunoreactive with endogenous factor VIII inhibitory antibodies is substituted with one or more amino acid residues that have no known identity to human or animal factor VIII sequence, and that do not form an epitope immunoreactive with endogenous factor VIII inhibitory antibodies; and/or (2) a one or more specific amino acid residues in the human, animal, or hybrid factor VIII that is critical to coagulant activity is substituted with one or more specific amino acid residues that have no known identity to human or animal factor VIII sequence that also have coagulant activity. The resulting hybrid factor VIII equivalent molecule has less reactivity with factor VIII inhibitory antibodies than the unsubstituted human factor VIII and has coagulant activity.

"Factor VIII deficiency," as used herein, includes deficiency in clotting activity caused by production of a defective factor VIII, by inadequate or no production of factor VIII, or by partial or total inhibition of factor VIII by inhibitors. Hemophilia A is a type of factor VIII deficiency resulting from a defect in an X-linked gene and the absence or deficiency of the factor VIII protein it encodes.

"Subunits" of human or animal factor VIII, as used herein, are the heavy and light chains of the protein. The heavy chain of factor VIII contains three "domains," A1, A2, and B. The light chain of factor VIII also contains three "domains," A3, C1, and C2.

As used herein, "diagnostic assays" include assays that in some manner utilize the antigen-antibody interaction to detect and/or quantify the amount of a particular antibody that is present in a test sample to assist in the selection of medical therapies. There are many such assays known to those of skill in the art. As used herein, however, the hybrid human\animal DNA and protein expressed therefrom, in whole or in part, can be substituted for the corresponding reagents in the otherwise known assays, whereby the modified assays may be used to detect and/or quantify antibodies to factor VIII. It is the use of these reagents, the hybrid human/animal DNA and protein expressed therefrom or the hybrid human/animal equivalent factor VIII DNA and protein expressed therefrom, that permits modification of known assays for detection of antibodies to human or animal factor VIII or to hybrid human/animal factor VIII. Such assays include, but are not limited to ELISAs, immunodiffusion assays, and immunoblots. Suitable methods for practicing any of these assays are known to those of skill in the art. As used herein, the hybrid human/animal or equivalent factor VIII or portion thereof that includes at least one epitope of the protein, can be used as the diagnostic reagent.

The terms "epitope", "antigenic site", "immunogenic site", and "antigenic determinant", as used herein, are used synonymously and are defined as a portion of the hybrid factor VIII protein that is specifically recognized by an antibody. It can consist of any number of amino acid residues, and it can be dependent upon the primary, secondary, or tertiary structure of the protein. In accordance with this disclosure, a factor VIII protein or equivalent that includes at least one epitope may be used as a reagent in the diagnostic assays.

### - General Description of Methods

Hybrid human/animal and equivalent factor VIII molecules, some of which have greater coagulant activity in a standard clotting assay when compared to highly-purified human factor VIII, and some of which have less immunoreactivity to inhibitory antibodies to human or porcine factor VIII, can be constructed as follows.

Five types of hybrid human/porcine or equivalent factor VIII molecules and the methods for preparing them are disclosed herein: those obtained (1) by substituting a porcine subunit (i.e., heavy chain or light chain) for the corresponding human subunit; (2) by substituting one or more porcine domain(s) (i.e., A1, A2, A3, B, C1, and C2) for the corresponding human domain(s); (3) by substituting part of one or more porcine domain(s) for the corresponding part of one or more domain(s) of the human domain; (4) by substituting one or more specific amino acid residue(s) in human factor VIII with the corresponding residue(s) from the porcine sequence; and (5) by substituting one or more specific amino acids in human, porcine, or hybrid human/porcine factor VIII with amino acid residue{s} having no known sequence identity to factor VIII. Five types of hybrid factor VIII molecules that have human factor VIII amino acid sequence and non-porcine mammalian factor VIII amino acid sequence, or as in the fifth category, human, non-porcine mammalian, or hybrid factor VIII, can also be prepared by the same methods.

Hybrid human/animal and equivalent factor VIII proteins listed above under groups (1)-(3) are made by isolation of subunits, domains, or parts of domains of plasma-derived factor VIII, followed by reconstitution and purification. Hybrid human/animal and equivalent factor VIII proteins described under groups (3)-(5) above are made by recombinant DNA methods. The hybrid molecule may contain a greater or lesser percentage of human than animal sequence, depending on the origin of the various regions, as described in more detail below.

It is shown below that hybrid human/porcine factor VIII consisting of porcine heavy chain/human light chain and corresponding to the first type of hybrid listed above has greater specific coagulant activity in a standard clotting assay as compared to human factor VIII. The hybrid human/animal or equivalent factor VIII with coagulant activity, whether the activity is higher, equal to, or lower than that of human factor VIII, can be useful in treating patients with inhibitors, since these inhibitors can react less with hybrid human/animal or equivalent factor VIII than with either human or porcine factor VIII.

### Preparation of hybrid human/animal factor VIII molecules from isolated human and animal factor VIII subunits by reconstitution:

Hybrid human/animal factor VIII molecules are prepared and isolated, and their procoagulant activity is characterized. One method, modified from procedures reported by Fay, P.J., *et al*., 265 J. Biol. Chem. 6197 (1990); and Lollar, J.S., *et al*., 263 J. Biol. Chem. 10451 (1988), involves the isolation of subunits (heavy and light chains) of human and animal factor VIII, followed by recombination of human heavy chain and animal light chain or by recombination of human light chain and animal heavy chain.

Isolation of both human and animal individual subunits involves dissociation of the light chain/heavy chain dimer by chelation of calcium with ethylenediaminetetraacetic acid (EDTA), followed by monoS™ HPLC (Pharmacia-LKB, Piscataway, NJ). Hybrid human/animal factor VIII molecules are reconstituted from isolated subunits in the presence of calcium. Hybrid human light chain/animal heavy chain or animal light chain/human heavy chain factor VIII is isolated from unreacted heavy chains by monoS™ HPLC by procedures for the isolation of porcine factor VIII, such as described by Lollar, J.S., et al., 71 Blood 137-143 (1988).

These methods, described in detail in the examples below, result in hybrid human light chain/porcine heavy chain molecules with greater than six times the procoagulant activity of human factor VIII. Other hybrid human/non-porcine mammalian factor VIII molecules can be prepared, isolated, and characterized for activity by the same methods.

### Preparation of hybrid human/animal factor VIII molecules from isolated human and animal factor VIII domains by reconstitution:

Hybrid human/animal factor VIII molecules with domain substitutions are prepared and isolated, and their procoagulant activity is characterized. One method involves the isolation of one or more domains of human and one or more domains of animal factor VIII, followed by recombination of human and animal domains to form hybrid human/animal factor VIII with coagulant activity, as described by Lollar, P., *et al*., 267(33) J. Biol. Chem. 23652-23657 (Nov. 25, 1992).

Plasma-derived animal and human A1/A3-C1-C2 dimers are isolated by dissociation of the A2 domain from factor VIIIa in the presence of NaOH, after which the mixture is diluted and the dimer is eluted using monoS™ HPLC (Pharmacia-LKB, Piscataway, NJ). The A2 domain is isolated from factor VIIIa as a minor component in the monoS™ HPLC. Hybrid human/animal factor VIII molecules are reconstituted by mixing equal volumes of the A2 domain of one species and the A1/A3-C1-C2 dimer of the other species. Hybrid factor VIII with one or more domain substitutions is isolated from the mixture of unreacted dimers and A2 domains by monoS™ HPLC by procedures for the isolation of porcine factor VIII, as described by Lollar, J.S., et al., 71 Blood 137-143 (1988).

These methods, described in detail in the examples below, result in hybrid factor VIII molecules with procoagulant activity.

### Preparation of hybrid factor VIII molecules by recombinant engineering of the sequences encoding human, animal, and hybrid factor VIII subunits, domains, or parts of domains:

### Substitution of subunits, domains, parts of domains:

The human factor VIII gene was isolated and expressed in mammalian cells, as reported by Toole, J.J., *et al*., 312 Nature 342-347 (1984) (Genetics Institute); Gitschier, J., *et al*., 312 Nature 326-330 (1984) (Genentech); Wood, W.I., *et al*., 312 Nature 330-337 (1984) (Genentech); Vehar, G.A., *et al*., 312 Nature 337-342 (1984) (Genentech), and the amino acid sequence was deduced from cDNA. U.S. Patent No. 4,965,199 to Capon *et al*. discloses a recombinant DNA method for producing factor VIII in mammalian host cells and purification of human factor VIII. Factor VIII expression in CHO (Chinese hamster ovary) cells and BHKC (baby hamster kidney cells) has been reported.

The cDNA sequence encoding human factor VIII and predicted amino acid sequence are shown in SEQ ID NO:1 and SEQ ID NO:2, respectively.

Recombinant hybrid factor VIII is prepared starting with human cDNA (Biogen, Inc.) encoding the factor VIII sequence corresponding to domains A1-A2-A3-C1-C2. The factor VIII encoded by this cDNA lacks the entire B domain and corresponds to amino acid residues 1-740 and 1649-2332 of single chain human factor VIII (see SEQ ID NO:2), according to the numbering system of Wood *et al*., 312 Nature 330-337 (1984). The B domain is deleted, since it does not appear to be necessary for biological function.

Porcine factor VIII has been isolated and purified from plasma (Fass, D.N., *et al*., 59 Blood 594 (1982)). The amino acid sequence of the B and part of the A2 domains of porcine factor VIII are reported by Toole, J.J., *et al*., 83 Proc. Nat'l. Acad. Sci. U.S.A. 5939-5942 (1986).

Both porcine and human factor VIII are isolated from plasma as a two subunit protein. The subunits, known as the heavy chain and light chain, are held together by a non-covalent bond that requires calcium or other divalent metal ions. The heavy chain of factor VIII contains three domains, A1, A2, and B, which are linked covalently. The light chain of factor VIII also contains three domains, designated A3, C1, and C2. The B domain has no known function and can be removed from the molecule proteolytically or by recombinant DNA technology methods without significant alteration in any measurable parameter of factor VIII. Human recombinant factor VIII has a similar structure and function to plasma-derived factor VIII, though it is not glycosylated unless expressed in mammalian cells.

Both human and porcine activated factor VIII (factor VIIIa) have three subunits due to cleavage of the heavy chain between the A1 and A2 domains. This structure is designated A1/A2/A3-C1-C2. Human factor VIIIa is not stable under the conditions that stabilize porcine factor VIIIa. This is because of the weaker association of the A2 subunit of human factor VIIIa. Dissociation of the A2 subunit of human and porcine factor VIIIa is associated with loss of activity in the factor VIIIa molecule.

The complete A2 domain of porcine factor VIII cDNA (SEQ ID NO:3), having sequence identity to residues 373-740 in SEQ ID NO:1, in mature human factor VIII, was sequenced. The predicted amino acid sequence is shown in SEQ ID NO:4.

Although only the A2 and B domains of porcine factor VIII have been sequenced entirely, the remainder of the porcine factor VIII molecule can be sequenced by standard cloning techniques, such as those described in Weis, J.H., "Construction of recombinant DNA libraries," in Current Protocols in Molecular Biology, F.M. Ausubel *et al*., eds. (1991), so that full length hybrids can be constructed.

Individual subunits, domains, or parts of domains of porcine or human factor VIII cDNA can be cloned and substituted for the corresponding human or porcine subunits, domains, or parts of domains by established mutagenesis techniques. For example, Lubin, I.M., et *al*., 269(12) J. Biol Chem. 8639-8641 (March 1994) describes techniques for substituting the porcine A2 domain for the human domain. These hybrid factor VIII cDNA molecules can be cloned into expression vectors for ultimate expression of active hybrid human/porcine factor VIII protein molecules by established techniques, as described by Selden, R.F., "Introduction of DNA into mammalian cells," in Current Protocols in Molecular Biology, F.M. Ausubel *et al*., eds (1991).

In a preferred embodiment, a hybrid human/porcine cDNA encoding factor VIII, in which the porcine sequence encodes a domain or part domain, such the A2 domain or part domain, is inserted in a mammalian expression vector, such as ReNeo, to form a construct that is used to stably transfect cells in culture, such as baby hamster kidney cells, using methods that are routine in the art, such as liposome-mediated transfection (Lipofectin™, Life Technologies, Inc.). Expression of recombinant hybrid factor VIII protein can be confirmed, for example, by sequencing, Northern and Western blotting, or polymerase chain reaction (PCR). Hybrid factor VIII protein in the culture media in which the transfected cells expressing the protein are maintained can be precipitated, pelleted, washed, resuspended in an appropriate buffer, and the recombinant hybrid factor VIII protein purified by standard techniques, including immunoaffinity chromatography. In one embodiment, the factor VIII is expressed as a fusion protein from a recombinant molecule in which a molecule encoding a protein that enhances stability, secretion, detection, or isolation is inserted in place adjacent to the factor VIII encoding sequence. The purified hybrid factor VIII can be assayed for immunoreactivity and coagulation activity by standard assays including, for example, the plasma-free factor VIII assay, the one-stage clotting assay, and the enzyme-linked immunosorbent assay using purified recombinant human factor VIII as a standard.

Other vectors, including both plasmid and eukaryotic viral vectors, may be used to express a recombinant gene construct in eukaryotic cells depending on the preference and judgment of the skilled practitioner (see, for example, Sambrook et al., Chapter 16). Other vectors and expression systems, including bacterial, yeast, and insect cell systems, can be used but are not preferred due to differences in, or lack of, glycosylation.

Recombinant hybrid factor VIII protein can be expressed in a variety of cells commonly used for culture and recombinant mammalian protein expression. A preferred cell line, available from the American Type Culture Collection, Rockville, MD, is baby hamster kidney cells, which are cultured using routine procedure and media.

The same methods can be used to prepare other recombinant hybrid factor VIII protein, such as human/non-porcine mammalian. Starting with primers from the known human DNA sequence, the murine and part of the porcine factor VIII cDNA have been cloned. Factor VIII sequences of other species for use in preparing a hybrid human/animal factor VIII molecule can be obtained using the known human DNA sequence as a starting point. Other techniques that can be employed include PCR amplification methods with animal tissue DNA, and use of a cDNA library from the animal to clone out the factor VIII sequence.

As an example, hybrid human/mouse factor VIII protein can be made as follows. DNA clones corresponding to the mouse homolog of the human factor VIII gene have been isolated and sequenced and the amino acid sequence of mouse factor VIII predicted, as described in Elder, G., *et al*., 16(2) Genomics 374-379 (May 1993), which also includes a comparison of the predicted amino acid sequences of mouse, human, and part of porcine factor VIII molecules. The mouse factor VIII cDNA sequence and predicted amino acid sequence are shown in SEQ ID NO:5 and SEQ ID NO:8, respectively. In a preferred embodiment, the RNA amplification with transcript sequencing (RAWTS) methods described in Sarkar, G., and S.S. Sommer, 244 Science 331-334 (1989), can be used. Briefly, the steps are (1) cDNA synthesis with oligo(dT) or an mRNA-specific oligonucleotide primer; (2) polymerase chain reaction (PCR) in which one or both oligonucleotides contains a phage promoter attached to a sequence complementary to the region to be amplified; (3) transcription with a phage promoter; and (4) reverse transcriptase-mediated dideoxy sequencing of the transcript, which is primed with a nested (internal) oligonucleotide. In addition to revealing sequence information, this method can generate an *in vitro* translation product by incorporating a translation initiation signal into the appropriate PCR primer; and can be used to obtain novel mRNA sequence information from other species.

### Substitution of amino acid(s):

The A2 domain is necessary for the procoagulant activity of the factor VIII molecule. According to Lollar, P., *et al*., 267 J. Biol. Chem. 23652-23657 (1992), the difference in coagulant activity between human and porcine factor VIII appears to be based on a difference in amino acid sequence between one or more residues in the A2 domain. Further, the A2 and the C2 domains in the human factor VIII molecule are thought to harbor the epitopes to which most, if not all, inhibitory antibodies react, according to Hoyer, L.W., and D. Scandella, 31 Semin. Hematol. 1-5 (1994). Recombinant hybrid factor VIII molecules can be made by substitution of amino acid sequence from animal A2, C2, and/or other domains into human factor VIII or amino acid sequence from the human A2, C2, and/or other domains into animal factor VIII, selecting in either case amino acid sequence that differs between the animal and human molecules. Hybrid molecules can also be made in which amino acid sequence from more than one animal is substituted in the human factor VIII molecule, or in which human and other animal amino acid sequence is inserted into an animal factor VIII molecule. Hybrid equivalent molecules can also be made, in which human, animal, or hybrid factor VIII contain one or more amino acids that have no known sequence identity to factor VIII. These hybrid molecules can then be assayed by standard procedures for coagulant activity and for reactivity with inhibitory antibodies to factor VIII for identification of hybrid factor VIII molecules with enhanced coagulant activity and/or decreased antibody immunoreactivity. Hybrid molecules may also be identified that have reduced coagulant activity compared to human but still have decreased antibody reactivity. The methods described herein to prepare hybrid human/porcine factor VIII with substitution of amino acids can be used to prepare recombinant hybrid human/non-porcine mammalian factor VIII protein, and hybrid animal-1/animal-2 factor VIII with amino acid sequence substitutions.

### Hybrid factor VIII molecules with altered coagulant activity.

Hybrid human/porcine factor VIII can be prepared in which human factor VIII amino acid sequence having procoagulant activity in the A2 domain is replaced with the corresponding porcine amino acid sequence, also having procoagulant activity. The sequence to be replaced is selected and prepared as follows. Both human and porcine A2 domains have 368 residues (SEQ ID NOs:2 and 6, respectively). As shown in Figure 1A-1B, which compares the alignment of the amino acid sequences of the human and porcine factor VIII A2 domains (residue numbering starts at position 373 with respect to the full length amino acid sequence of human factor VIII, SEQ ID NO:2), 50 of these residues are different and 318 are identical; i.e., there is an 86 percent sequence identity when human and porcine factor VIII A2 domains are aligned. Therefore, there is a large but finite number of combinations that will result in hybrid human/porcine factor VIII molecules with enhanced coagulant activity, based on these 50 differences.

For preparation of a hybrid human/porcine factor VIII molecule, the initial target candidates for mutagenesis, which were revealed upon comparison of the human and porcine A2 amino acid sequences (SEQ ID NOs:2 and 6, respectively) within the human A2 domain, are shown in Table I.

**TABLE I.**

| **HUMAN AMINO ACID SEQUENCE TARGET CANDIDATES FOR MUTAGENESIS (SEQ ID NO:2)** | | | |
|---|---|---|---|
| Sequence | Residues | Mismatches | Charge Changes |
| 398-403 | 6 | 4 | 1 |
| 434-444 | 10 | 4 | 3 |
| 484-496 | 13 | 7 | 3 |
| 598-603 | 6 | 4 | 2 |
| 536-541 | 6 | 4 | 0 |
| 713-722 | 10 | 6 | 2 |
| 727-737 | 11 | 6 | 2 |

Table I and the bold letters of Figure 1A-1B illustrate seven sequences in the human and pig A2 domain amino acid sequences (SEQ ID NOs:2 and 6, respectively) that constitute only 17 percent of the A2 domain but include 70 percent of the sequence differences between human and porcine A2 domains. Hybrids are made by selecting porcine sequence based on the sequence differences and substituting it into the human A2 domain.

Directed mutagenesis techniques are used to identify hybrid protein with coagulant activity that can be enhanced, equal to, or reduced, compared to human factor VIII, but preferably is enhanced. Specific human sequences are replaced with porcine sequences, preferably using the splicing by overlap extension method (SOE), as described by Ho, S.N., *et al*., 77 *Gene* 51-59 (1994), and in Examples 7 and 8. In another embodiment, oligonucleotide-directed mutagenesis can be used, as was done to loop out the amino acid sequence for part of the human A2 domain (see Example 7). As functional analysis of the hybrids reveals coagulant activity, the sequence can be further dissected and mapped for procoagulant sequence by point mutation analysis, using standard site-directed mutagenesis techniques. Amino acid sequence substitutions in the A2 domain are described in Examples 7 and 8.

### Hybrid factor VIII molecules with reduced immunoreactivity.

The approach described in the previous section for substitution of amino acids in the factor VIII molecule can also be used to identify one or more critical region(s) in the A2, C2, and/or other domains to which inhibitory antibodies are directed and to prepare an effective procoagulant hybrid molecule with no immunoreactivity or reduced immunoreactivity, as demonstrated in example 8, by replacement of one or more epitopes in the human factor VIII with corresponding porcine amino acid sequence.

Usually, porcine factor VIII has limited or no reaction with inhibitory antibodies. Over 90 percent of inhibitory antibodies to human factor VIII are directed against either the A2 or C2 domains or both. Hybrid human/porcine factor VIII molecules having decreased or no reactivity with inhibitory antibodies based on amino acid substitution in the A2 domain are prepared as follows. The porcine A2 domain is cloned by standard cloning techniques, as described above and in Examples 6, 7, and 8, and then cut and spliced within the A2 domain using routine procedures, such as using restriction sites to cut the cDNA or splicing by overlap extension (SOE). The resulting constructs of known porcine amino acid sequence are substituted into the human A2 domain to form a hybrid factor VIII construct, which is inserted into a mammalian expression vector, preferably ReNeo, stably transfected into cultured cells, preferably baby hamster kidney cells, and expressed, as described above. The hybrid factor VIII is assayed for immunoreactivity, for example with anti-A2 antibodies by the routine Bethesda assay or by plasma-free chromogenic substrate assay. The Bethesda unit (BU) is the standard method for measuring inhibitor titers. If the Bethesda titer is not measurable (<0.7 BU/mg IgG) in the hybrid, then a human A2 epitope was eliminated in the region of substituted corresponding porcine sequence. The epitope is progressively narrowed, and the specific A2 epitope can thus be determined to produce a hybrid human/porcine molecule with as little porcine sequence as possible.

Hybrid human/porcine factor VIII molecules having decreased or no reactivity with inhibitory antibodies based on substitution of amino acid sequence in the C2 or other domain, with or without substitution in the A2 domain, can be prepared. The procedures can be the same or similar to those described herein for amino acid substitution in the A2 domain, including cloning the porcine C2 or other domain, for example by using RT-PCR or by probing a porcine liver cDNA library with human C2 or other domain DNA; restriction site techniques and/or successive SOE to map and simultaneously replace epitopes in the C2 or other domain; expression in cultured cells; and routine assay for immunoreactivity. For the assays, antibodies specific to the C2 domain, such as the inhibitory autoantibody IgG described by Scandella, D., et *al*., Thromb. Haemostasis 67:665-671 (1992) and Lubin et al. (1994), are available, for example from Dr. Dorothea Scandella, American Red Cross, Rockville, MD.

The C2 domain consists of amino acid residues 2173-2332 (SEQ ID NO:2). Within this 154 amino acid region, inhibitor activity appears to be directed to a 65 amino acid region between residues 2248 and 2312, according to Shima, M., *et al*., 69 Thromb. Haemostas. 240-246 (1993). If the C2 sequence of human and porcine factor VIII is approximately 85 percent identical in this region, as it is elsewhere in the functionally active regions of factor VIII, there will be approximately ten differences between human and porcine factor VIII C2 amino acid sequence, which can be used as initial targets to construct hybrids with substituted C2 sequence.

It is likely that clinically significant factor VIII epitopes are confined to the A2 and C2 domains. However, if antibodies to other regions (A1, A3, B, or C1 domains) of factor VIII are identified, they can be mapped and eliminated by using hybrid human/porcine factor VIII molecules with the same approach.

### Preparation of hybrid factor VIII molecules using human and non-porcine mammalian factor VIII amino acid sequence.

The methods used to prepare hybrid human/porcine factor VIII with substitution of specific amino acids can be used to prepare recombinant hybrid human/non-porcine mammalian factor VIII protein that has, compared to human factor VIII, altered or the same coagulant activity and/or equal or reduced immunoreactivity, based on substitution of one or more amino acids in the A2, C2, and/or other domains.

Similar comparisons of amino acid sequence identity can be made between human and other non-porcine mammalian factor VIII proteins to determine the amino acid sequences in which procoagulant activity and anti-A2 and anti-C2 immunoreactivity or immunoreactivity in other domains reside. Similar methods can then be used to prepare other hybrid human/animal factor VIII molecules. As described above, functional analysis of each hybrid will reveal those with decreased reactivity to inhibitory antibodies and/or increased coagulant activity, and the sequence can be further dissected by point mutation analysis.

For example, hybrid human/mouse factor VIII molecules can be prepared as described above. The amino acid sequence alignment of the A2 domain of human (SEQ ID NO:2) and mouse (SEQ ID NO:6) is shown in Figure 1A-1B. As reported by Elder et al., the factor VIII protein encoded by the mouse cDNA (SEQ ID NO:5) has 2319 amino acids, with 74% sequence identity overall to the human sequence (SEQ ID NO:2) (87 percent identity when the B domain is excluded from the comparison), and is 32 amino acids shorter than human factor VIII. The amino acid sequences in the mouse A and C domains (SEQ ID NO:6) are highly conserved, with 84-93 percent sequence identity to the human sequence (SEQ ID NO:2), while the B and the two short acidic domains have 42-70 percent sequence identity. Specifically, the A1, A2, and A3 mouse amino acid sequences (SEQ ID NO:6) are 85, 85, and 90 percent identical to the corresponding human amino acid sequences (SEQ ID NO:2). The C1 and C2 mouse amino acid sequences are 93 and 84 percent identical to the corresponding human amino acid sequences. In the predicted mouse factor VIII amino acid sequence (SEQ ID NO:6), the A1, A2, and A3 domains include amino acids 1-330, 380-711, and 1664-1987, respectively, using amino acid sequence identity for numbering purposes.

The thrombin/factor Xa and all but one activated protein C cleavage sites are conserved in mouse factor VIII. The tyrosine residue for von Willebrand factor binding is also conserved.

According to Elder *et al*., the nucleotide sequence (SEQ ID NO:5) of mouse factor VIII contains 7519 bases and has 67 percent identity overall with the human nucleotide sequence (SEQ ID NO:1). The 6957 base pairs of murine coding sequence have 82 percent sequence identity with the 7053 base pairs of coding sequence in human factor VIII. When the B domain is not included in the comparison, there is an 88 percent nucleotide sequence identity.

Elder et *al*. report that human and mouse factor VIII molecules are 74 percent identical overall, and that 95 percent of the human residues that lead to hemophilia when altered are identical in the mouse. These data support the application of the same techniques used to identify amino acid sequence with coagulant activity and/or immunoreactivity to antibodies in the porcine factor VIII molecule to the mouse or other animal factor VIII to identify similar amino acid sequences and prepare hybrid molecules.

In another embodiment, cross-reactivity, in which human plasma reacts with porcine factor VIII, can be reduced by preparation of hybrid porcine/animal factor VIII. First, a determination of whether human A2 specific inhibitors react with factor VIII from other mammals is made, using the routine Bethesda assay and the particular mammalian plasma as the standard. Inhibitor titers are usually measured in plasma, so purified animal factor VIII is not necessary. If A2 inhibitors do not react with the animal factor VIII, such as murine factor VIII, the sequence of which is known, then corresponding animal sequence can be substituted into the porcine epitope region, as identified by using human/porcine hybrids. Once the animal sequence is known, site directed mutagenesis techniques, such as oligonucleotide-mediated mutagenesis described by Kunkel, T.A., et *al*., 204 Meth. Enzymol. 125-139 (1991), can be used to prepare the hybrid porcine/animal factor VIII molecule. If other animal plasmas are less reactive with A2 inhibitors than murine or porcine factor VIII, the animal sequence corresponding to the porcine epitope can be determined by routine procedures, such as RT-PCR, and a hybrid human/animal or porcine/animal factor VIII constructed by site directed mutagenesis. Also, hybrid human/animal or porcine/non-porcine mammalian factor VIII can be prepared that has corresponding amino acid sequence substitution from one or more other animals.

After identification of clinically significant epitopes, recombinant hybrid factor VIII molecules will be expressed that have less than or equal cross-reactivity with human factor VIII when tested *in vitro* against a broad survey of inhibitor plasmas. Preferably these molecules will be combined A2/C2 hybrids in which immunoreactive amino acid sequence in these domains is replaced by porcine or other animal sequence. Additional mutagenesis in these regions may be done to reduce cross-reactivity. Reduced cross-reactivity, although desirable, is not necessary to produce a product that may have advantages over the existing porcine factor VIII concentrate, which produces side effects due to contaminant porcine proteins and may produce untoward effects due to the immunogenicity of porcine factor VIII sequences. A hybrid human/animal or porcine/animal factor VIII molecule will not contain foreign porcine proteins. Additionally, the extensive epitope mapping accomplished in the porcine A2 domain indicates that greater than 95% of the therapeutic hybrid human/porcine factor VIII sequence will be human.

### Preparation of hybrid human/animal or porcine/animal factor VIII equivalents:

The methods described above and in the examples can also be used to prepare procoagulant hybrid human/animal, non-porcine animal-1/animal-2, or porcine/non-porcine mammalian factor VIII equivalent molecules. One or more specific amino acid residues in human or animal factor VIII or hybrid factor VIII that function as an antigenic site which is immunoreactive with endogenous factor VIII inhibitory antibodies can be identified as described, and then can be substituted with one or more specific amino acid residues that has no known identity to human or animal factor VIII sequence and that does not form an antigenic site immunoreactive with endogenous factor VIII inhibitory antibodies. One or more antigenic sites can be substituted to form an active hybrid factor VIII equivalent molecule. The resulting active hybrid factor VIII equivalent molecule has equal or less reactivity with factor VIII inhibitory antibodies than the unsubstituted human or animal or hybrid human/animal factor VIII.

Alternatively or additionally, active hybrid factor VIII equivalent molecules can be prepared, using the methods described above and in the examples, in which one or more specific amino acid residues in human or animal factor VIII or hybrid human/animal factor VIII that are critical to the coagulant activity can be identified as described, and then can be substituted with one or more amino acid residues having no known identity to human or animal factor VIII sequence that also provides coagulant activity. One or more specific amino acids that have coagulant activity can be replaced to form an active hybrid factor VIII equivalent molecule. The resulting procoagulant hybrid factor VIII equivalent molecule has coagulant activity that may be less than, equal to, or greater than that of the unsubstituted factor VIII molecule. Preferably, the hybrid factor VIII equivalent molecule has coagulant activity that is superior to that of human factor VIII.

Suitable specific amino acid residues that can be substituted for those sequences of amino acids critical to coagulant and/or antigenic activity in human or animal factor VIII or hybrid human/animal factor VIII include any specific amino acids not having sequence identity to animal or human factor VIII amino acid sequence that has coagulant activity and/or has less or equal reactivity with endogenous inhibitory antibodies to factor VIII.

Hybrid factor VIII equivalent molecules can have substitutions of one or more specific amino acid sequences for coagulant activity and/or one or more specific amino acid sequences for antigenic sites. Hybrid factor VIII equivalent molecules described herein also include those molecules in which amino acid residues not critical to coagulant activity or antigenic activity are substituted with amino acid residues having no known identity to animal factor VIII sequence.

In one embodiment, a hybrid factor VIII equivalent molecule, preferably a hybrid human/porcine molecule, can be prepared in which cross-reactivity with inhibitor plasmas is reduced as follows. One or more epitopes are identified, as described above, and then replaced by alanine residues, using, for example, the alanine scanning mutagenesis method described by Cunningham, B.C., and J.A. Wells, 244 Science 1081-1085 (1989). Since the human A2 epitope has been narrowed to 26 or fewer amino acids, as described in Example 8, alanine scanning mutagenesis can be performed on a limited number of hybrid proteins to determine which are active, non-cross-reactive hybrid factor VIII based on A2 amino acid substitutions.

### - Diagnostic Assays

The hybrid human/animal or equivalent factor VIII cDNA and/or protein expressed therefrom, in whole or in part, can be used in assays as diagnostic reagents for the detection of inhibitory antibodies to human or animal factor VIII or to hybrid human/animal factor VIII in substrates, including, for example, samples of serum and body fluids of human patients with factor VIII deficiency. These antibody assays include assays such as ELISA assays, immunoblots, radioimmunoassays, immunodiffusion assays, and assay of factor VIII biological activity (e.g., by coagulation assay). Techniques for preparing these reagents and methods for use thereof are known to those skilled in the art. For example, an immunoassay for detection of inhibitory antibodies in a patient serum sample can include reacting the test sample with a sufficient amount of the hybrid human/animal factor VIII that contains at least one antigenic site, wherein the amount is sufficient to form a detectable complex with the inhibitory antibodies in the sample.

Nucleic acid and amino acid probes can be prepared based on the sequence of the hybrid human/animal factor VIII molecule. These can be labeled using dyes or enzymatic, fluorescent, chemiluminescent, or radioactive labels that are commercially available. The amino acid probes can be used, for example, to screen sera or other body fluids where the presence of inhibitors to human, animal, or hybrid human/animal factor VIII is suspected. Levels of inhibitors can be quantitated in patients and compared to healthy controls, and can be used, for example, to determine whether a patient with a factor VIII deficiency can be treated with a hybrid human/animal factor VIII.

### - Pharmaceutical Compositions

Pharmaceutical compositions containing hybrid human/animal, porcine/non-porcine mammalian, animal-1/animal-2, or human/animal equivalent factor VIII, alone or in combination with appropriate pharmaceutical stabilization compounds, delivery vehicles, and/or carrier vehicles, are prepared according to known methods, as described in Remington's *Pharmaceutical Sciences* by E.W. Martin.

In one preferred embodiment, the preferred carriers or delivery vehicles for intravenous infusion are physiological saline or phosphate buffered saline.

In another preferred embodiment, suitable stabilization compounds, delivery vehicles, and carrier vehicles include but are not limited to other human or animal proteins such as albumin.

Phospholipid vesicles or liposomal suspensions are also preferred as pharmaceutically acceptable carriers or delivery vehicles. These can be prepared according to methods known to those skilled in the art and can contain, for example, phosphatidylserine/phosphatidylcholine or other compositions of phospholipids or detergents that together impart a negative charge to the surface, since factor VIII binds to negatively charged phospholipid membranes. Liposomes may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the hybrid factor VIII is then introduced into the container. The container is then swirled by hand to free lipid material from the sides of the container and to disperse lipid aggregates, thereby forming the liposomal suspension.

The hybrid factor VIII can be combined with other suitable stabilization compounds, delivery vehicles, and/or carrier vehicles, including vitamin K dependent clotting factors, tissue factor, and von Willebrand factor (vWf) or a fragment of vWf that contains the factor VIII binding site, and polysaccharides such as sucrose.

Hybrid factor VIII can also be delivered by gene therapy in the same way that human factor VIII can be delivered, using delivery means such as retroviral vectors. This method consists of incorporation of factor VIII cDNA into human cells that are transplanted directly into a factor VIII deficient patient or that are placed in an implantable device, permeable to the factor VIII molecules but impermeable to cells, that is then transplanted. The preferred method will be retroviral-mediated gene transfer. In this method, an exogenous gene (e.g., a factor VIII cDNA) is cloned into the genome of a modified retrovirus. The gene is inserted into the genome of the host cell by viral machinery where it will be expressed by the cell. The retroviral vector is modified so that it will not produce virus, preventing viral infection of the host. The general principles for this type of therapy are known to those skilled in the art and have been reviewed in the literature (e.g., Kohn, D.B., and P.W. Kantoff, 29 Transfusion 812-820, 1989).

Hybrid factor VIII can be stored bound to vWf to increase the half-life and shelf-life of the hybrid molecule. Additionally, lyophilization of factor VIII can improve the yields of active molecules in the presence of vWf. Current methods for storage of human and animal factor VIII used by commercial suppliers can be employed for storage of hybrid factor VIII. These methods include: (1) lyophilization of factor VIII in a partially-purified state (as a factor VIII "concentrate" that is infused without further purification); (2) immunoaffinity-purification of factor VIII by the Zimmerman method and lyophilization in the presence of albumin, which stabilizes the factor VIII; (3) lyophilization of recombinant factor VIII in the presence of albumin.

Additionally, hybrid factor VIII has been indefinitely stable at 4° C in 0.6 M NaCl, 20 mM MES, and 5 mM CaCl₂ at pH 6.0 and also can be stored frozen in these buffers and thawed with minimal loss of activity.

### - Methods of Treatment

Hybrid factor VIII is used to treat uncontrolled bleeding due to factor VIII deficiency (e.g., intraarticular, intracranial, or gastrointestinal hemorrhage) in hemophiliacs with and without inhibitory antibodies and in patients with acquired factor VIII deficiency due to the development of inhibitory antibodies. The active materials are preferably administered intravenously.

Additionally, hybrid factor VIII can be administered by transplant of cells genetically engineered to produce the hybrid or by implantation of a device containing such cells, as described above.

In a preferred embodiment, pharmaceutical compositions of hybrid factor VIII alone or in combination with stabilizers, delivery vehicles, and/or carriers are infused into patients intravenously according to the same procedure that is used for infusion of human or animal factor VIII.

The treatment dosages of hybrid factor VIII composition that must be administered to a patient in need of such treatment will vary depending on the severity of the factor VIII deficiency. Generally, dosage level is adjusted in frequency, duration, and units in keeping with the severity and duration of each patient's bleeding episode. Accordingly, the hybrid factor VIII is included in the pharmaceutically acceptable carrier, delivery vehicle, or stabilizer in an amount sufficient to deliver to a patient a therapeutically effective amount of the hybrid to stop bleeding, as measured by standard clotting assays.

Factor VIII is classically defined as that substance present in normal blood plasma that corrects the clotting defect in plasma derived from individuals with hemophilia A. The coagulant activity *in vitro* of purified and partially-purified forms of factor VIII is used to calculate the dose of factor VIII for infusions in human patients and is a reliable indicator of activity recovered from patient plasma and of correction of the *in vivo* bleeding defect. There are no reported discrepancies between standard assay of novel factor VIII molecules *in vitro* and their behavior in the dog infusion model or in human patients, according to Lusher, J.M., *et al.,* 328 New. Engl. J. Med. 453-459 (1993); Pittman, D.D., *et al*., 79 Blood 389-397 (1992), and Brinkhous *et al*., 82 Proc. Natl. Acad. Sci. 8752-8755 (1985).

Usually, the desired plasma factor VIII level to be achieved in the patient through administration of the hybrid factor VIII is in the range of 30-100% of normal. In a preferred mode of administration of the hybrid factor VIII, the composition is given intravenously at a preferred dosage in the range from about 20 to 50 units/kg body weight; the interval frequency is in the range from about 8 to 24 hours (in severely affected hemophiliacs); and the duration of treatment in days is in the range from 1 to 10 days or until the bleeding episode is resolved. *See*, *e*.*g*., Roberts, H.R., and M.R. Jones, "Hemophilia and Related Conditions - Congenital Deficiencies of Prothrombin (Factor II, Factor V, and Factors VII to XII)," Ch. 153, 1453-1474, 1460, in Hematology, Williams, W. J., et *al.,* ed. (1990). Patients with inhibitors may require more hybrid factor VIII, or patients may require less hybrid factor VIII because of its higher specific activity than human factor VIII or decreased antibody reactivity. As in treatment with human or porcine factor VIII, the amount of hybrid factor VIII infused is defined by the one-stage factor VIII coagulation assay and, in selected instances, *in vivo* recovery is determined by measuring the factor VIII in the patient's plasma after infusion. It is to be understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition.

Treatment can take the form of a single intravenous administration of the composition or periodic or continuous administration over an extended period of time, as required. Alternatively, hybrid factor VIII can be administered subcutaneously or orally with liposomes in one or several doses at varying intervals of time.

Hybrid factor VIII can also be used to treat uncontrolled bleeding due to factor VIII deficiency in hemophiliacs who have developed antibodies to human factor VIII. In this case, coagulant activity that is superior to that of human or animal factor VIII alone is not necessary. Coagulant activity that is inferior to that of human factor VIII (i.e., less than 3,000 units/mg) will be useful if that activity is not neutralized by antibodies in the patient's plasma.

The hybrid factor VIII molecule and the methods for isolation, characterization, making, and using it generally described above will be further understood with reference to the following non-limiting examples.

### Example 1: Assay of porcine factor VIII and hybrid human/porcine factor VIII

Porcine factor VIII has more coagulant activity than human factor VIII, based on specific activity of the molecule. These results are shown in Table III in Example 4. This conclusion is based on the use of appropriate standard curves that allow human and porcine factor VIII to be fairly compared. Coagulation assays are based on the ability of factor VIII to shorten the clotting time of plasma derived from a patient with hemophilia A. Two types of assays were employed: the one-stage and the two-stage assay.

In the one-stage assay, 0.1 ml hemophilia A plasma (George King Biomedical, Inc.) was incubated with 0.1 ml activated partial thromboplastin reagent (APTT) (Organon Teknika) and 0.01 ml sample or standard, consisting of diluted, citrated normal human plasma, for 5 min at 37°C in a water bath. Incubation was followed by addition of 0.1 ml 20 mM CaCl₂, and the time for development of a fibrin clot was determined by visual inspection.

A unit of factor VIII is defined as the amount present in 1 ml of citrated normal human plasma. With human plasma as the standard, porcine and human factor VIII activity were compared directly. Dilutions of the plasma standard or purified proteins were made into 0.15 M NaCl, 0.02 M HEPES, pH 7.4. The standard curve was constructed based on 3 or 4 dilutions of plasma, the highest dilution being 1/50, and on log₁₀ clotting time plotted against log₁₀ plasma concentration, which results in a linear plot. The units of factor VIII in an unknown sample were determined by interpolation from the standard curve.

The one-stage assay relies on endogenous activation of factor VIII by activators formed in the hemophilia A plasma, whereas the two-stage assay measures the procoagulant activity of preactivated factor VIII. In the two-stage assay, samples containing factor VIII that had been reacted with thrombin were added to a mixture of activated partial thromboplastin and human hemophilia A plasma that had been preincubated for 5 min at 37°C. The resulting clotting times were then converted to units/ml, based on the same human standard curve described above. The relative activity in the two-stage assay was higher than in the one-stage assay because the factor VIII had been preactivated.

### Example 2: Characterization of the functional difference between human and porcine factor VIII.

The isolation of porcine and human plasma-derived factor VIII and human recombinant factor VIII have been described in the literature in Fulcher, C. A., and T. S. Zimmerman, 79 Proc. Nat'l. Acad. Sci. U.S.A. 1648-1652 (1982); Toole, J.J., *et al*., 312 Nature 342-347 (1984) (Genetics Institute); Gitschier, J., *et al*., 312 Nature 326-330 (1984) (Genentech); Wood, W.I., *et al*., 312 Nature 330-337 (1984) (Genentech); Vehar, G.A., *et al*., 312 Nature 337-342 (1984) (Genentech); Fass, D.N., *et al*., 59 Blood 594 (1982); Toole, J.J., et *al*., 83 Proc. Nat'l. Acad. Sci. U.S.A. 5939-5942 (1986). This can be accomplished in several ways. All these preparations are similar in subunit composition, although this is the first description of the functional difference between human and porcine factor VIII, not noted previously in part due to the lack of use of a common standard by which to compare them.

For comparison of human recombinant and porcine factor VIII, preparations of highly-purified human recombinant factor VIII (Cutter Laboratories, Berkeley, CA) and porcine factor VIII (immunopurified as described in Fass, D.N., *et al.,* 59 Blood 594 (1982)) were subjected to high-pressure liquid chromatography (HPLC) over a Mono Q™ (Pharmacia-LKB, Piscataway, NJ) anion-exchange column (Pharmacia, Inc.). The purposes of the Mono Q™ HPLC step were elimination of minor impurities and exchange of human and porcine factor VIII into a common buffer for comparative purposes. Vials containing 1000-2000 units of factor VIII were reconstituted with 5 ml H₂O. Hepes (2 M at pH 7.4) was then added to a final concentration of 0.02 M. Factor VIII was applied to a Mono Q™ HR 5/5 column equilibrated in 0.15 M NaCl, 0.02 M Hepes, 5 mM CaCl₂, at pH 7.4 (Buffer A plus 0.15 M NaCl); washed with 10 ml Buffer A + 0.15 M NaCl; and eluted with a 20 ml linear gradient, 0.15 M to 0.90 M NaCl in Buffer A at a flow rate of 1 ml/min.

For comparison of human factor VIII (derived from plasma and purified by Mono Q™ HPLC) and porcine factor VIII, immunoaffinity-purified, plasma-derived porcine factor VIII was diluted 1:4 with 0.04 M Hepes, 5 mM CaCl₂, 0.01% Tween-80, at pH 7.4, and subjected to Mono Q™ HPLC under the same conditions described in the previous paragraph for human factor VIII. These procedures for the isolation of human and porcine factor VIII are standard for those skilled in the art.

Column fractions were assayed for factor VIII activity by a one-stage coagulation assay. The average results of the assays, expressed in units of activity per A₂₈₀ of material, are given in Table II, and indicate that porcine factor VIII has at least six times greater activity than human factor VIII when the one-stage assay is used.

**TABLE II:**

| **COMPARISON OF HUMAN AND PORCINE FACTOR VIII COAGULANT ACTIVITY** | |
|---|---|
| | Activity (U/A₂₈₀) |
| Porcine | 21,300 |
| Human plasma-derived | 3,600 |
| Human recombinant | 2,400 |

### Example 3: Comparison of the stability of human and porcine factor VIll

The results of the one-stage assay for factor VIII reflect activation of factor VIII to factor VIIIa in the sample and possibly loss of formed factor VIIIa activity. A direct comparison of the stability of human and porcine factor VIII was made. Samples from Mono Q™ HPLC (Pharmacia, Inc., Piscataway, N.J.) were diluted to the same concentration and buffer composition and reacted with thrombin. At various times, samples were removed for two-stage coagulation assay. Typically, peak activity (at 2 min) was 10-fold greater for porcine than human factor VIIIa, and the activities of both porcine and human factor VIIIa subsequently decreased, with human factor VIIIa activity decreasing more rapidly.

Generally, attempts to isolate stable human factor VIIIa are not successful even when conditions that produce stable porcine factor VIIIa are used. To demonstrate this, Mono Q™ HPLC-purified human factor VIII was activated with thrombin and subjected to Mono S™ cation-exchange (Pharmacia, Inc.) HPLC under conditions that produce stable porcine factor VIIIa, as described by Lollar, J.S., and C.G. Parker, 28 Biochemistry 666 (1989).

Human factor VIII, 43 *µ*g/ml (0.2 *µ*M) in 0.2 M NaCl, 0.01 M Hepes, 2.5 mM CaCl₂, at pH 7.4, in 10 ml total volume, was reacted with thrombin (0.036 *µ*M) for 10 min, at which time FPR-CH₂Cl D-phenyl-prolyl-arginyl-chloromethyl ketone was added to a concentration of 0.2 *µ*M for irreversible inactivation of thrombin. The mixture then was diluted 1:1 with 40 mM 2-(N-morpholino)ethane sulfonic acid (MES), 5 mM CaCl₂, at pH 6.0, and loaded at 2 ml/min onto a Mono S™ HR 5/5 HPLC column (Pharmacia, Inc.) equilibrated in 5 mM MES, 5 mM CaCl₂, at pH 6.0 (Buffer B) plus 0.1 M NaCl. Factor VIIIa was eluted without column washing with a 20 ml gradient from 0.1 M NaCl to 0.9 M NaCl in Buffer B at 1 ml/min.

The fraction with coagulant activity in the two-stage assay eluted as a single peak under these conditions. The specific activity of the peak fraction was approximately 7,500 U/A₂₈₀. Sodium dodecyl sulfate-polyacrylamide gel electrophoresis (SDS-PAGE) of the Mono S™ factor VIIIa peak, followed by silver staining of the protein, revealed two bands corresponding to a heterodimeric (A3-C1-C2/A1) derivative of factor VIII. Although the A2 fragment was not identified by silver staining under these conditions because of its low concentration, it was identified as a trace constituent by ¹²⁵I-labeling.

In contrast to the results with human factor VIII, porcine factor VIIIa isolated by Mono S™ HPLC under the same conditions had a specific activity 1.6 x 10⁶ U/A₂₈₀. Analysis of porcine factor VIIIa by SDS-PAGE revealed 3 fragments corresponding to A1, A2, and A3-C1-C2 subunits, demonstrating that porcine factor VIIIa possesses three subunits.

The results of Mono S™ HPLC of human thrombin-activated factor VIII preparations at pH 6.0 indicate that human factor VIIIa is labile under conditions that yield stable porcine factor VIIIa. However, although trace amounts of A2 fragment were identified in the peak fraction, determination of whether the coagulant activity resulted from small amounts of heterotrimeric factor VIIIa or from heterodimeric factor Villa that has a low specific activity was not possible from this method alone.

A way to isolate human factor VIIIa before it loses its A2 subunit is desirable to resolve this question. To this end, isolation was accomplished in a procedure that involves reduction of the pH of the Mono S™ buffers to pH 5. Mono Q™-purified human factor VIII (0.5 mg) was diluted with H₂O to give a final composition of 0.25 mg/ml (1 *µ*M) factor VIII in 0.25 M NaCl, 0.01 M Hepes, 2.5 mM CaCl₂, 0.005% Tween-80, at pH 7.4 (total volume 7.0 ml). Thrombin was added to a final concentration of 0.072 *µ*M and allowed to react for 3 min. Thrombin was then inactivated with FPR-CH₂Cl (0.2 *µ*M). The mixture then was diluted 1:1 with 40 mM sodium acetate, 5 mM CaCl₂, 0.01% Tween-80, at pH 5.0, and loaded at 2 ml/min onto a Mono S™ HR 5/5 HPLC column equilibrated in 0.01 M sodium acetate, 5 mM CaCl₂, 0.01% Tween-80, at pH 5.0, plus 0.1 M NaCl. Factor VIIIa was eluted without column washing with a 20 ml gradient from 0.1 M NaCl to 1.0 M NaCl in the same buffer at 1 ml/min. This resulted in recovery of coagulant activity in a peak that contained detectable amounts of the A2 fragment as shown by SDS-PAGE and silver staining. The specific activity of the peak fraction was ten-fold greater than that recovered at pH 6.0 (75,000 U/A₂₈₀ vs. 7,500 U/A₂₈₀). However, in contrast to porcine factor VIIIa isolated at pH 6.0, which is indefinitely stable at 4°C, human factor VIIIa activity decreased steadily over a period of several hours after elution from Mono S™. Additionally, the specific activity of factor VIIIa purified at pH 5.0 and assayed immediately is only 5% that of porcine factor VIIIa, indicating that substantial dissociation occurred prior to assay.

These results demonstrate that both human and porcine factor VIIIa are composed of three subunits (A1, A2, and A3-C1-C2). Dissociation of the A2 subunit is responsible for the loss of activity of both human and porcine factor VIIIa under certain conditions, such as physiological ionic strength, pH, and concentration. The relative stability of porcine factor VIIIa under certain conditions is because of stronger association of the A2 subunit.

### Example 4: Preparation of hybrid human/porcine factor VIII by reconstitution with subunits.

Porcine factor VIII light chains and factor VIII heavy chains were isolated as follows. A 0.5 M solution of EDTA at pH 7.4 was added to Mono Q™-purified porcine factor VIII to a final concentration of 0.05 M and was allowed to stand at room temperature for 18-24 h. An equal volume of 10 mM histidine-Cl, 10 mM EDTA, 0.02% v/v Tween 80, at pH 6.0 (Buffer B), was added, and the solution was applied at 1 ml/min to a Mono S™ HR 5/5 column previously equilibrated in Buffer A plus 0.25 M NaCl. Factor VIII heavy chains did not bind the resin, as judged by SDS-PAGE. Factor VIII light chain was eluted with a linear, 20 ml, 0.1-0.7 M NaCl gradient in Buffer A at 1 ml/min and was homogeneous by SDS-PAGE. Factor VIII heavy chains were isolated by mono Q™ HPLC (Pharmacia, Inc., Piscataway, N.J.) in the following way. Factor VIII heavy chains do not adsorb to mono S™ during the purification of factor VIII light chains. The fall-through material that contained factor VIII heavy chains was adjusted to pH 7.2 by addition of 0.5 M Hepes buffer, pH 7.4, and applied to a mono Q™ HR5/5 HPLC column (Pharmacia, Inc.) equilibrated in 0.1 M NaCl, 0.02 M Hepes, 0.01% Tween-80, pH 7.4. The column was washed with 10 ml of this buffer, and factor VIII heavy chains were eluted with a 20 ml 0.1-1.0 M NaCl gradient in this buffer. Human light chains and heavy chains were isolated in the same manner.

Human and porcine light and heavy chains were reconstituted according to the following steps. Ten *µ*l human or porcine factor VIII light chain, 100 *µ*g/ml, was mixed in 1 M NaCl, 0.02 M Hepes, 5 mM CaCl₂, 0.01% Tween-80, pH 7.4, with (1) 25 *µ*l heterologous heavy chain, 60 *µ*g/ml, in the same buffer; (2) 10 *µ*l 0.02 M Hepes, 0.01% Tween-80, pH 7.4; (3) 5 *µ* l 0.6 M CaCl₂, for 14 hr at room temperature. The mixture was diluted 1/4 with 0.02 M MES, 0.01% Tween-80, 5 mM CaCl₂, pH 6, and applied to Mono S™ Hr5/5 equilibrated in 0.1 M NaCl, 0.02 M MES, 0.01% Tween-80, 5mM CaCl₂, pH 6.0. A 20 ml gradient was run from 0.1 - 1.0 M NaCl in the same buffer at 1 ml/min, and 0.5 ml fractions were collected. Absorbance was read at 280 nm of fractions, and fractions were assayed with absorbance for factor VIII activity by the one-stage clotting assay. Heavy chains were present in excess, because free light chain (not associated with heavy chain) also binds Mono S™; excess heavy chains ensure that free light chains are not part of the preparation. Reconstitution experiments followed by Mono S™ HPLC purification were performed with all four possible combinations of chains: human light chain/human heavy chain, human light chain/porcine heavy chain, porcine light chain/porcine heavy chain, porcine light chain/human heavy chain. Table III shows that human light chain/porcine heavy chain factor VIII has activity comparable to native porcine factor VIII (Table II), indicating that structural elements in the porcine heavy chain are responsible for the increased coagulant activity of porcine factor VIII compared to human factor VIII.

**TABLE III:**

| **COMPARISON OF HYBRID HUMAN/PORCINE FACTOR VIII COAGULANT ACTIVITY WITH HUMAN AND PORCINE FACTOR VIII** | |
|---|---|
| | Activity (U/A₂₈₀) |
| Porcine light chain/porcine heavy chain | 30,600 |
| Human light chain/porcine heavy chain | 44,100 |
| Porcine light chain/human heavy chain | 1,100 |
| Human light chain/human heavy chain | 1,000 |

### Example 5: Preparation of active hybrid human/porcine factor VIII by reconstitution with domains.

The porcine A1/A3-C1-C2 dimer, the porcine A2 domain, the human A1/A3-C1-C2 dimer, and the human A2 domain were each isolated from porcine or human blood, according to the method described in Lollar, P., et *al*., 267(33) J. Biol. Chem. 23652-23657 (Nov. 25, 1992). For example, to isolate the porcine A1/A3-C1-C2 dimer, porcine factor VIIIa (140 *µ*g) at pH 6.0 was raised to pH 8.0 by addition of 5 N NaOH for 30 minutes, producing dissociation of the A2 domain and 95 percent inactivation by clotting assay. The mixture was diluted 1:8 with buffer B (20 mM HEPES, 5 mM CaCl₂, 0.01 % Tween 80, pH 7.4) and applied to a monoS column equilibrated in buffer B. The A1/A3-C1-C2 dimer eluted as a single sharp peak at approximately 0.4 M NaCl by using a 0.1-1.0 M NaCl gradient in buffer B. To isolate the porcine A2 domain, porcine factor VIIIa was made according to the method of Lollar, P., and C.G. Parker, 28 Biochem. 666-674 (1989), starting with 0.64 mg of factor VIII. Free porcine A2 domain was isolated as a minor component (50 *µ* g) at 0.3 M NaCl in the monoS™ chromatogram.

Hybrid human/porcine factor VIII molecules were reconstituted from the dimers and domains as follows. The concentrations and buffer conditions for the purified components were as follows: porcine A2, 0.63 *µ*M in buffer A (5 mM MES; 5 mM CaCl₂, 0.01% Tween 80, pH 6.0) plus 0.3 M NaCl; porcine A1/A3-C1-C2, 0.27 *µ*M in buffer B plus 0.4 M NaCl, pH 7.4; human A2, 1 *µ*M in 0.3 M NaCl, 10 mM histidine-HCl, 5 mM CaCl₂, 0.01 % Tween 20, pH 6.0; human A1/A3-C1-C2, 0.18 *µ*M in 0.5 M NaCl, 10 mM histidine-Cl, 2.5 mM CaCl₂, 0.1 % Tween 20, pH 6.0. Reconstitution experiments were done by mixing equal volumes of A2 domain and A1/A3-C1-C2 dimer. In mixing experiments with porcine A1/A3-C1-C2 dimer, the pH was lowered to 6.0 by addition of 0.5 M MES, pH 6.0, to 70 mM.

The coagulation activities of all four possible hybrid factor VIIIa molecules - [pA2/(hA1/A3-C1-C2)], [hA2/(pA1/A3-Cl-C2)], [pA2/(pA1/pA3-C1-C2)], and [hA2/(hA1/A3-C1-C2)] - were obtained by a two-stage clotting assay at various times.

The generation of activity following mixing the A2 domains and A1/A3-C1-C2 dimers was nearly complete by one hour and was stable for at least 24 hours at 37°C. Table IV shows the activity of reconstituted hybrid factor VIIIa molecules when assayed at 1 hour. The two-stage assay, by which the specific activities of factor VIIIa molecules were obtained, differs from the one-stage assay, and the values cannot be compared to activity values of factor VIII molecules obtained by a one-stage assay.

**TABLE IV:**

| **COMPARISON OF COAGULANT ACTIVITIES OF DOMAIN-SUBSTITUTED HYBRID HUMAN/PORCINE FACTOR VIIIa** | |
|---|---|
| Hybrid fVIIIa | Specific Activity (U/mg) |
| Porcine A2 + Human A1/A3-C1-C2 | 140,000 |
| | |
| Porcine A2 + Porcine A1/A3-C1-C2 | 70,000 |
| | |
| Human A2 + Porcine A1/A3-C1-C2 | 40,000 |
| | |
| Human A2 + Human A1/A3-C1-C2 | 40,000 |

Table IV shows that the greatest activity was exhibited by the porcine A2 domain/human A1/A3-C1-C2 dimer, followed by the porcine A2 domain/porcine A1/A3-C1-C2 dimer.

Thus, when the A2 domain of porcine factor VIIIa was mixed with the A1/A3-C1-C2 dimer of human factor VIIIa, coagulant activity was obtained. Further, when the A2 domain of human factor VIIIa was mixed with the A1/A3-C1-C2 dimer of porcine factor VIIIa, coagulant activity was obtained. By themselves, the A2, A1, and A3-C1-C2 regions have no coagulant activity.

### Example 6: Isolation and sequencing of the A2 domain of porcine factor VIII.

Only the nucleotide sequence encoding the B domain and part of the A2 domain of porcine factor VIII has been sequenced previously (Toole, J.J., *et al*., 83 Proc. Nat'l. Acad. Sci. U.S.A. 5939-5942 (1986)). The cDNA and predicted amino acid sequences (SEQ ID NOs:5 and 6, respectively) for the entire porcine factor VIII A2 domain are disclosed herein.

The porcine factor VIII A2 domain was cloned by reverse transcription of porcine spleen total RNA and PCR amplification; degenerate primers based on the known human factor VIII cDNA sequence and an exact porcine primer based on a part of the porcine factor VIII sequence were used. A 1 kb PCR product was isolated and amplified by insertion into a Bluescript™ (Stratagene) phagemid vector.

The porcine A2 domain was completely sequenced by dideoxy sequencing. The cDNA and predicted amino acid sequences are as described in SEQ ID NOs:5 and 6, respectively.

### Example 7: Preparation of recombinant hybrid human/animal factor VIII

The nucleotide and predicted amino acid sequences (SEQ ID NOs:1 and 2, respectively) of human factor VIII have been described in the literature (Toole, J.J., et *al.,* 312 Nature 342-347 (1984) (Genetics Institute); Gitschier, J., *et al*., 312 Nature 326-330 (1984) (Genentech); Wood, W.I., *et al*., 312 Nature 330-337 (1984) (Genentech); Vehar, G.A., et al., 312 Nature 337-342 (1984) (Genentech)).

Making recombinant hybrid human/animal factor VIII requires that a region of human factor VIII cDNA (Biogen Corp.) be removed and the animal cDNA sequence having sequence identity be inserted. Subsequently, the hybrid cDNA is expressed in an appropriate expression system. As an example, hybrid factor VIII cDNAs were cloned in which some or all of the porcine A2 domain was substituted for the corresponding human A2 sequences. Initially, the entire cDNA sequence corresponding to the A2 domain of human factor VIII and then a smaller part of the A2 domain was looped out by oligonucleotide-mediated mutagenesis, a method commonly known to those skilled in the art *(see, e*.*g*., Sambrook, J., E.F. Fritsch, and T. Maniatis, Molecular Cloning: A Laboratory Manual, Chapter 15, Cold Spring Harbor Press, Cold Spring Harbor, 1989). The steps were as follows.

### Materials.

Methoxycarbonyl-D-cyclohexylglycyl-glycl-arginine-p-nitroanilide (Spectrozyme™ Xa) and anti-factor VIII monoclonal antibodies ESH4 and ESH8 were purchased from American Diagnostica (Greenwich, CT). Unilamellar phosphatidylcholine/phosphatidylserine (75/25, w/w) vesicles were prepared according to the method of Barenholtz, Y., *et al*., 16 Biochemistry 2806-2810 (1977). Recombinant desulfatohirudin was obtained from Dr. R. B. Wallis, Ciba-Geigy Pharmaceuticals (Cerritos, CA). Porcine factors IXa, X, Xa, and thrombin were isolated according to the methods of Lollar, P., *et al.,* 63 Blood 1303-1306 (1984), and Duffy, E.J., and P. Lollar, 207 J. Biol. Chem. 7621-7827 (1992). Albumin-free pure recombinant human factor VIII was obtained from Baxter-Biotech (Deerfield, IL).

### Cloning of the porcine factor VIII A2 domain.

The cDNA encoding the porcine A2 domain was obtained following PCR of reverse-transcribed porcine spleen mRNA isolated as described by Chomczyneki, P., and Sacohi, N., 162 Anal. Biochem. 156-159 (1987). cDNA was prepared using the first-strand cDNA synthesis kit with random hexamers as primers (Pharmacia, Piscataway, N.J.). PCR was carried out using a 5'-terminal degenerate primer 5' AARCAYCCNAARACNTGGG 3' (SEQ ID NO:11), based on known limited porcine A2 amino acid sequence, and a 3'-terminal exact primer, 5' GCTCGCACTAGGGGGTCTTGAATTC 3' (SEQ ID NO:12), based on known porcine DNA sequence immediately 3' of the porcine A2 domain. These oligonucleotides correspond to nucleotides 1186-1203 and 2289-2313 in the human sequence (SEQ ID NO:1). Amplification was carried out for 35 cycles (1 minute 94°C, 2 minutes 50°C, 2 minutes 72°C) using *Taq* DNA polymerase (Promega Corp., Madison, WI). The 1.1-kilobase amplified fragment was cloned into pBluescript II KS-(Stratagene) at the *Eco*RV site using the T-vector procedure, as described by Murchuk, D., *et al*., 19 Nucl. Acids Res. 1154 (1991). *Escherichia coli* XL1-Blue-competent cella were transformed, and plasmid DNA was isolated. Sequencing was carried out in both directions using Sequenase™ version 2.0 (U.S. Biochemical Corp., a Division of Amersham LifeScience, Inc., Arlington Hts, IL). This sequence was confirmed by an identical sequence that was obtained by direct sequencing of the PCR product from an independent reverse transcription of spleen RNA from the same pig (CircumVent™, New England Biolabs, Beverly, MA). The region containing the epitope for autoantibody RC was identified as 373-536 in human factor VIII (SEQ ID NO:2).

### Construction and expression of a hybrid human/porcine factor VIII cDNA.

B-domainless human factor VIII (HB-, from Biogen, Inc. Cambridge, MA), which lacks sequences encoding for amino acid residues 741-1648 (SEQ ID NO:2), was used as the starting material for construction of a hybrid human/porcine factor VIII. HB- was cloned into the expression vector ReNeo. To facilitate manipulation, the cDNA for factor VIII was isolated as a *XhoI/HpaI* fragment from ReNeo⁻ and cloned into Xho1/*Eco*RV digested pBlueSsript II KS⁻. An oligonucleotide, 5' CCTTCCTTTATCCAAATACGTAGATCAAGAGGAAATTGAC 3' (SEQ ID NO:7), was used in a site-directed mutagenesis reaction using uracil-containing phage DNA, as described by Kunkel, T.A., *et al.,* 204 Meth. Enzymol. 125-139 (1991), to simultaneously loop-out the human A2 sequence (nucleotides 1169-2304 in SEQ ID NO:1) and introduce a SnaBI restriction site. The A2-domainless human factor VIII containing plasmid was digested with SnaBI followed by addition of *Cla*I linkers. The porcine A2 domain was then amplified by PCR using the phosphorylated 5' primer 5' GTAGCGTTGCCAAGAAGCACCCTAAGACG 3' (SEQ ID NO:8) and 3' primer 5' GAAGAGTAGTACGAGTTATTTCTCTGGGTTCAATGAC 3' (SEQ ID NO:9), respectively. *Cla*I linkers were added to the PCR product followed by ligation into the human factor VIII-containing vector. The A1/A2 and A2/A3 junctions were corrected to restore the precise thrombin cleavage and flanking sequences by site-directed mutagenesis using the oligonucleotide shown in SEQ ID NO:8 and nucleotides 1-22 (5' GAA ... TTC in SEQ ID NO:9) to correct the 5'- and 3'-terminal junctions, respectively. In the resulting construct, designated HP1, the human A2 domain was exactly substituted with the porcine A2 domain. A preliminary product contained an unwanted thymine at the A1-A2 junction as a result of the PCR amplification of the porcine A2 domain. This single base can be looped out by use of the mutagenic oligonucleotide 5' CCTTTATCCAAATACGTAGCGTTTGCCAAGAAG 3' (SEQ ID NO:10).

A region containing 63% of the porcine NH₂-terminal A2 domain, which encompasses the putative A2 epitope, was substituted for the homologous human sequence of B-domainless cDNA by exchanging *SpeI/Bam*HI fragments between the pBluescript plasmids containing human factor VIII and human/porcine A2 factor VIII cDNA. The sequence was confirmed by sequencing the A2 domain and splice sites. Finally, a *SpeI/ApaI* fragment, containing the entire A2 sequence, was substituted in place of the corresponding sequence in HB-, producing the HP2 construct.

Preliminary expression of HB⁻ and HP2 in COS-7 cells was tested after DEAE-dextran-mediated DNA transfection, as described by Seldon, R.F., in Current Protocols in Molecular Biology (Ausubel, F.M., *et al*, eds), pp. 9.21-9.26, Wiley Interscience, N.Y. After active factor VIII expression was confirmed and preliminary antibody inhibition studies were done, HB- and HP2 DNA were then stably transfected into baby hamster kidney cells using liposome-mediated transfection (Lipofectin®, Life Technologies, Inc., Gaithersburg, MD). Plasmid-containing clones were selected for G418 resistance in Dulbecco's modified Eagle's medium-F12, 10% fetal calf serum (DMEM-F12/10% fetal calf serum) containing 400 *µ*g/ml G418, followed by maintenance in DMEM-F12/10% fetal calf serum containing 100 *µ*g/ml G418. Colonies showing maximum expression of HB- and HP2 factor VIII activity were selected by ring cloning and expanded for further characterization.

HB- and HP2 factor VIII expression was compared by plasma-free factor VIII assay, one-stage clotting assay, and enzyme-linked immunosorbent assay using purified recombinant human factor VIII as a standard. Specific coagulant activities of 2600 and 2580 units/mg were obtained for HB- and HP2, respectively. HB- and HP2 produced 1.2 and 1.4 units/ml/48 hours/10⁷ cells, respectively. This is identical to that of the wild type construct (2,600 ± 200 units/mg). The specific activities of HB- and HP2 were indistinguishable in the plasma-free factor VIII assay.

### Construction and expression of hybrid human/non-porcine mammalian factor VIII.

Cloning of other animal A1, A3, C1, and C2 domains and part domains is feasible with the same strategy that was used for cloning the porcine A2 domain. Fragments of these domains can be cloned by the looping out mutagenesis technique. Excision of the corresponding domains in human factor VIII and any fragments thereof, including single amino acid eliminations, is feasible by looping out mutagenesis as described above. All possible domain replacements, fragments of domain replacements, or single amino acid residue replacements are possible by this approach.

The biological activity of recombinant hybrid human/animal factor VIII with A1, A2, A3, C1, and/or C2 domain substitutions can be evaluated initially by use of a COS-cell mammalian transient expression system. Hybrid human/animal cDNA can be transfected into COS cells, and supernatants can be analyzed for factor VIII activity by use of one-stage and two-stage coagulation assays as described above. Additionally, factor VIII activity can be measured by use of a chromogenic substrate assay, which is more sensitive and allows analysis of larger numbers of samples. Similar assays are standard in the assay of factor VIII activity (Wood, W.I., *et al*., 312 Nature 330-337, 1984; Toole, J.J., et al., 312 Nature 342-347, 1984). Expression of recombinant factor VIII in COS cells is also a standard procedure (Toole, J.J., *et al*., 312 Nature 342-347, 1984; Pittman, D.D., and R.J. Kaufman, 85 Proc. Nat'l. Acad. Sci. U.S.A. 2429-2433, 1988). The human factor VIII cDNA used as starting material for the recombinant molecules described herein has been expressed in COS cells yielding a product with biological activity. This material, as described above, can be used as a standard to compare hybrid human/animal factor VIII molecules. The activity in the assays is converted to a specific activity for proper comparison of the hybrid molecules. For this, a measurement of the mass of factor VIII produced by the cells is necessary and can be done by immunoassay with purified human and/or animal factor VIII as standards. Immunoassays for factor VIII are routine for those skilled in the art (*See, e.g.*, Lollar, P., et *al*., 71 Blood 137-143, 1988).

### Example 8. Determination of inhibitory activity in hybrid human/animal factor VIII.

Sequences of human and animal factor VIII likely to be involved as epitopes (i.e., as recognition sites for inhibitory antibodies that react with factor VIII) can be determined using routine procedures, for example through use of assay with antibodies to factor VIII combined with site directed mutagenesis techniques such as splicing by overlap extension methods (SOE), as shown below. Sequences of animal factor VIII that are not antigenic compared to corresponding antigenic human sequences can be identified, and substitutions can be made to insert animal sequences and delete human sequences according to standard recombinant DNA methods. Porcine factor VIII reacts less than human factor VIII with some inhibitory antibodies; this provides a basis for current therapy for patients with inhibitors. After the recombinant hybrids are made, they can be tested *in vitro* for reactivity with routine assays, including the Bethesda inhibitor assay. Those constructs that are less reactive than native human factor VIII and native animal factor VIII are candidates for replacement therapy.

The epitopes to which most, if not all, inhibitory antibodies reactive with human factor VIII are directed are thought to reside in two regions in the 2332 amino acid human factor VIII molecule, the A2 domain (amino acid residues 373-740) and the C2 domain (amino acid residues 2173-2332, both sequences shown in SEQ ID NO:2). The A2 epitope has been eliminated by making a recombinant hybrid human/porcine factor VIII molecule in which part of the human A2 domain is replaced by the porcine sequence having sequence identity to the replaced human amino acid sequence. This was accomplished, as described in Example 7, by cloning the porcine A2 domain by standard molecular biology techniques and then cutting and splicing within the A2 domain using restriction sites. In the resulting construct, designated HP2, residues 373-603 (SEQ ID NO:4) of porcine factor VIII were substituted into the human A2 domain. HP2 was assayed for immunoreactivity with anti-human factor VIII antibodies using the following methods.

### Factor VIII enzyme-linked immunosorbent assay.

Microtiter plate wells were coated with 0.15 ml of 6 *µ*g/ml ESH4, a human factor VIII light-chain antibody, and incubated overnight. After the plate was washed three times with H₂O, the wells were blocked for 1 hour with 0.15 M NaCl, 10 mM sodium phosphate, 0.05% Tween 20, 0.05% nonfat dry milk, 0.05% sodium azide, pH 7.4. To increase sensitivity, samples containing factor VIII were activated with 30 nM thrombin for 15 minutes. Recombinant desulfatohirudin then was added at 100 nM to inhibit thrombin. The plate was washed again and 0.1 ml of sample or pure recombinant human factor VIII (10-600 ng/ml), used as the standard, were added. Following a 2 hour incubation, the plate was washed and 0.1 ml of biotinylated ESH8, another factor VIII light-chain antibody, was added to each well. ESH8 was biotinylated using the Pierce sulfosuccinimidyl-6-(biotinamide)hexanoate biotinylation kit. After a 1 hour incubation, the plate was washed and 0.1 ml of strepavidin alkaline phosphatase was added to each well. The plate was developed using the Bio-Rad alkaline phosphatase substrate reagent kit, and the resulting absorbance at 405 nm for each well was determined by using a Vmax microtiter plate reader (Molecular Devices, Inc., Sunnyville, CA). Unknown factor VIII concentrations were determined from the linear portion of the factor VIII standard curve.

### Factor VIII assays.

HB- and HB2 factor VIII were measured in a one-stage clotting assay, which was performed as described above (Bowie, E.J.W., and C.A. Owen, in *Disorders of Hemostasis* (Ratnoff and Forbes, eds) pp. 43-72, Grunn & Stratton, Inc., Orlando, FL (1984)), or by a plasma-free assay as follows. HB- or HP2 factor VIII was activated by 40 nM thrombin in 0.15 M NaCl, 20 nM HEPES, 5 mM CaCl₂, 0.01% Tween 80, pH 7.4, in the presence of 10 nM factor IXa, 425 nM factor X, and 50 *µ*M unilamellar phosphatidylserine/phosphatidylcholine (25/75, *w/w*) vesicles. After 5 minutes, the reaction was stopped with 0.05 M EDTA and 100 nM recombinant desulfatohirudin, and the resultant factor Xa was measured by chromogenic substrate assay, according to the method of Hill-Eubanks, D.C., and P. Lollar, 265 J. Biol. Chem. 17854-17858 (1990). Under these conditions, the amount of factor Xa formed was linearly proportional to the starting factor VIII concentration as judged by using purified recombinant human factor VIII (Baxter Biotech, Deerfield, IL) as the standard.

Prior to clotting assay, HB- or HP2 factor VIII were concentrated from 48 hour conditioned medium to 10-15 units/ml by heparin-Sepharose™ chromatography. HB- or HP2 factor VIII were added to hemophilia A plasma (George King Biomedical) to a final concentration of 1 unit/ml. Inhibitor titers in RC or MR plasma or a stock solution of mAb 413 IgG (4 *µ*M) were measured by the Bethesda assay as described by Kasper, C.K., *et al*., 34 Thromb. Diath. Haemorrh. 869-872 (1975). Inhibitor IgG was prepared as described by Leyte, A., *et al*., 266 J. Biol. Chem. 740-746 (1991).

HP2 does not react with anti-A2 antibodies. Therefore, residues 373-603 must contain an epitope for anti-A2 antibodies.

### Preparation of hybrid human/porcine factor VIII and assay by splicing by overlap extension (SOE).

Several more hybrid human/porcine factor VIII molecules with porcine amino acid substitutions in the human A2 region have been prepared to further narrow the A2 epitope. Besides restriction site techniques, the "splicing by overlap extension" method (SOE) as described by Ho, S.N., et al., 77 Gene 51-59 (1989), has been used to substitute any arbitrary region of porcine factor VIII cDNA. In SOE, the splice site is defined by overlapping oligonucleotides that can be amplified to produce the desired cDNA by PCR. Eight cDNA constructs, designated HP4 through HP11, have been made. They were inserted into the ReNeo expression vector, stably transfected into baby hamster kidney cells, and expressed to high levels, as described in Example 7.

The hybrid human/porcine factor VIII constructs were assayed for reactivity with the anti-A2 inhibitor MAb413 using the Bethesda assay (Kasper, C.K., *et al*., 34 Thromb. Diath. Haemorrh. 869-872 (1975)). The Bethesda unit (BU) is the standard method for measuring inhibitor titers. The results are shown in Table V, and are compared to recombinant human factor VIII.

**TABLE V:**

| **COMPARISON OF IMMUNOREACTIVITY OF AMINO ACID-SUBSTITUTED HYBRID HUMAN/PORCINE FACTOR VIII** | | |
|---|---|---|
| Construct | Porcine Substitution | Inhibition MAb413 (BU/mg IgG) |
| Human fVIII | None | 1470 |
| HP4 | 373-540 | <0.7 |
| HP5 | 373-509 | <0.7 |
| HP6 | 373-444 | 1450 |
| HP7 | 445-509 | <0.7 |
| HP8 | 373-483 | 1250 |
| HP9 | 484-509 | <0.7 |
| HP10 | 373-403 | 1170 |
| HP11 | 404-509 | <0.7 |

As shown in Table V, if the Bethesda titer is not measurable (<0.7 BU/mg IgG), then an A2 epitope lies in the region of substituted porcine sequence. The epitope has been progressively narrowed to residues 484-509 (SEQ ID NO:2), consisting of only 26 residues, as exemplified by non-reactivity of MAb413 with HP9.

The region between 484-509 can be divided. If such division produces porcine sequences of, for example, residues 484-497 and 498-509, neither of which react with anti-A2 inhibitory antibodies, this will indicate that the epitope has been split, and that amino acids on both sides of the 497-498 splice site are necessary to produce the epitope.

The methods described in Examples 7 and 8 can be used to prepare other hybrid human/non-porcine mammalian factor VIII with amino acid substitution in the human A2 domain, hybrid human/animal factor VIII with amino acid substitution in any domain, or other hybrid factor VIII molecules or equivalents such hybrid factor VIII having reduced or absent immunoreactivity with anti-factor VIII antibodies.

## Claims

1. A purified hybrid factor VIII molecule comprising non-human mammalian and human amino acid sequences, wherein the molecule has procoagulant activity in an *in vitro* coagulation assay and wherein the molecule is selected from the group consisting of human or non-human mammalian factor VIII comprising at least one specific sequence shorter than a domain including one or more unique amino acids of the factor VIII of one species substituted for the corresponding sequence of the factor VIII of the other species, wherein the corresponding sequence includes at least a sequence of amino acids corresponding to positions 484-509 of human factor VIII as shown in SEQ ID No: 2, and wherein the hybrid factor VIII is less immunoreactive than human factor VIII with the inhibitory antibodies to the A2 domain of factor VIII.

2. The hybrid factor VIII molecule of claim 1, wherein the corresponding sequence of one or more amino acids of the factor VIII of one species substituted by the specific sequence including one or more unique amino acids of the factor VIII of the other species corresponds to human amino acid sequence as shown in SEQ ID NO: 2 selected from the group consisting of amino acids 373-540, 373-509, 445-509, and 404-509.

3. The hybrid factor VIII molecule of claim 2, wherein the molecule is a hybrid human/porcine molecule, the corresponding sequence to be substituted by the specific sequence is human, and the specific sequence is porcine.

4. The hybrid factor VIII molecule of claim 1, wherein the hybrid factor VIII is useful in treating human patients having antibodies to factor VIII that inhibit coagulation activity.

5. A composition containing a hybrid factor VIII molecule of claim 1, in combination with clotting factors selected from the group consisting of von Willebrand factor, vitamin K dependent clotting factors, and coagulant tissue factor.

6. The hybrid factor VIII molecule of claim 1, wherein the non-human mammalian domain substituted for the corresponding human domain is the porcine or murine A2 domain.

7. The hybrid factor VIII molecule of claim 1, wherein the non-human mammalian sequence substituted for the corresponding human sequence is porcine or murine, and the non-human mammalian specific sequence including one or more unique amino acids substituted for corresponding human amino acids 484-509 is murine or porcine.

8. The hybrid factor VIII molecule as claimed in any of claims 1-7, wherein the molecule lacks the B domain.

9. A method for manufacture of a medicament for treating human patients with factor VIII deficiency comprising preparing a hybrid factor VIII molecule as defined by any of claims 1-7.

10. A method of preparing purified procoagulant hybrid factor VIII, wherein the hybrid factor VIII comprises porcine or murine and human amino acid sequences, comprising substituting at least one specific sequence corresponding to positions 484-509 of human factor VIII as shown in SEQ ID NO: 2 for a corresponding sequence of amino acids of the factor VIII of the other species by site-directed mutagenesis of the encoding nucleic acid.

11. The method of claim 10 for preparing the hybrid factor VIII molecules of claims 1, 2 and 7.

12. The method of claim 10, wherein the corresponding sequence of one or more amino acids of the factor VIII of one species substituted by the specific sequence including one or more unique amino acids of the factor VIII of the other species corresponds to human amino acid sequence as shown in SEQ ID NO: 2 selected from the group consisting of amino acids 373-540, 373-509, 445-509, and 404-509.

13. The method of claim 10, wherein the specific sequence including one or more unique amino acids of the factor VIII of one species substituted for the corresponding sequence of one or more amino acids of the factor VIII of the other species includes a determinant of coagulant activity.

14. The method of claim 13, wherein the hybrid factor VIII has greater coagulant activity than that of human factor VIII when human plasma is used as the standard in a one-stage *in vitro* coagulation assay.

15. The method of claim 10, wherein the corresponding sequence of one or more amino acids of the factor VIII of one species to be substituted by the specific sequence including one or more unique amino acids of the factor VIII of the other species includes an antigenic site that reacts with antibodies to factor VIII that inhibit coagulant activity, and wherein the hybrid factor VIII is less immunoreactive than human factor VIII with the inhibitory antibodies to factor VIII.

16. The method as claimed in any of claims 10-15 further comprising the step of deleting the B domain to make a hybrid B-domainless factor VIII.

17. A method of preparing purified hybrid factor VIII according to claim 1, wherein the hybrid factor VIII comprises porcine and human amino acid sequences, comprising the steps of
expressing recombinant DNA encoding domains selected from the group consisting of A1, B, A3, C1 and C2 domains of porcine and human factor VIII,
further comprising substituting one or more domains of porcine and human factor VIII.

18. The method of claim 17, further comprising the step of deleting the B domain to make a hybrid B-domainless factor VIII.

19. A purified hybrid factor VIII equivalent molecule comprising human, non-human mammalian, or hybrid human/non-human mammalian factor VIII, wherein the molecule has procoagulant activity in an *in vitro* coagulation assay, comprising at least one sequence having no known sequence identity to factor VIII substituted for a specific amino acid sequence corresponding to positions 484-509 of human factor VIII as shown in SEQ ID NO: 2 in human, non-human mammalian, or hybrid human/non-human mammalian factor VIII.

20. The equivalent molecule of claim 19, having greater procoagulant activity than human factor VIII in a one-stage *in vitro* coagulation assay using human plasma as the standard.

21. The equivalent molecule of claim 19, wherein the specific amino acid sequence in human, non-human mammalian, or hybrid human/non-human mammalian factor VIII comprises an epitope having immunoreactivity with antibodies that inhibit the coagulant activity of factor VIII, and wherein the hybrid equivalent factor VIII has reduced immonoreactivity to the antibodies.

22. The equivalent molecule of claim 19 or 21, wherein the amino acid sequence having no known sequence identity to factor VIII comprises one or more alanine residues.

23. The equivalent molecule as claimed in any of claims 19 to 22 lacking the B domain.

24. A method of preparing a hybrid factor VIII equivalent molecule comprising human, non-human mammalian, or hybrid human/non-human mammalian factor VIII, wherein the equivalent molecule has procoagulant activity in an *in vitro* coagulation assay, comprising
substituting at least one sequence having no known sequence identity to factor VIII for a specific amino acid sequence corresponding to positions 484-509 of human factor VIII as shown in SEQ ID NO: 2 in the human, non-human mammalian, or hybrid human/non-human mammalian factor VIII.

25. The method of claim 24, wherein the amino acid sequence having no known sequence identity to factor VIII comprises one or more alanine residues.

26. The method of claim 24, further comprising substituting at least one specific sequence of one or more unique amino acids including a determinant of coagulant activity of the factor VIII of one species for a corresponding sequence of one or more amino acids of the factor VIII of another species, or for a corresponding sequence of one or more amino acids of the hybrid factor VIII, wherein the hybrid factor VIII equivalent molecule has greater coagulant activity than human factor VIII in a one-stage coagulation assay using human plasma as the standard.

27. The method of claim 24, wherein the specific amino acid sequence in human, non-human mammalian, or hybrid human/non-human mammalian factor VIII comprises an epitope having immunoreactivity with antibodies that inhibit the coagulant activity of factor VIII, and wherein the hybrid equivalent factor VIII has reduced immunoreactivity to the antibodies.

28. The method as claimed in any of claims 24-27, further comprising deleting the B domain.

29. A fusion protein comprising the hybrid factor VIII molecule of claims 1 or 19.

30. An isolated nucleic acid sequence encoding a hybrid human/non-human mammalian factor VIII molecule according to claim 1, wherein the hybrid factor VIII is less immunoreactive than human factor VIII with inhibitory antibodies to factor VIII.

31. The isolated nucleic acid sequence of claim 30, wherein the molecule has a specific activity greater than that of human factor VIII when human plasma is used as the standard in a one-stage coagulation assay.

32. The isolated nucleic acid sequence of claim 30, wherein the corresponding sequence of amino acids of the factor VIII of one species substituted by the specific sequence including unique amino acids of the factor VIII of the other species corresponds to human amino acid sequence as shown in SEQ ID NO: 2 selected from the group consisting of amino acids 373-540, 373-509, and 445-509.

33. The isolated nucleic acid sequence of claim 30, wherein the hybrid factor VIII is useful in treating human patients having antibodies to factor VIII that inhibit coagulation activity.

34. The isolated nucleic acid sequence as claimed in any of claims 30-33, wherein the molecule lacks the B domain.

35. An isolated nucleic acid sequence encoding a hybrid factor VIII equivalent molecule according to claim 19.

36. The isolated nucleic acid sequence of claim 35, said hybrid factor VIII equivalent molecule having greater procoagulant activity than human factor VIII in a one-stage *in vitro* coagulation assay using human plasma as the standard.

37. The isolated nucleic acid sequence of claim 35, wherein the specific amino acid sequence in human, non-human mammalian, or hybrid human/non-human mammalian factor VIII comprises an epitope having immunoreactivity with antibodies that inhibit the coagulant activity of factor VIII, and wherein the hybrid equivalent factor VIII has reduced immunoreactivity to the antibodies.

38. The isolated nucleic acid sequence of claim 35, wherein the amino acid sequence having no known sequence identity to factor VIII comprises one or more alanine residues.

39. The isolated nucleic acid sequence as claimed in any of claims 35-38, wherein the molecule lacks the B domain.

40. A hybrid factor VIII molecule as claimed in any of claims 1-8, 19-23 or 29 for use in medicine.

41. Use of a molecule as claimed in any of claims 1-8, 19-23 or 29 in the manufacture of a medicament for use in treating human factor VIII deficiency.

42. Use as claimed in claim 41, wherein the clotting activity of the hybrid human/non-human mammalian or hybrid equivalent molecule has reduced immunoreactivity with antibodies that inhibit factor VIII coagulant activity.

43. Use as claimed in claim 42, wherein the patients are characterized by the presence of antibodies to factor VIII.

## Patentansprüche

1. Gereinigtes Hybridfaktor VIII-Molekül, umfassend nicht-menschliche Säuger- und menschliche Aminosäuresequenzen, wobei das Molekül eine prokoagulierende Aktivität in einem *in vitro*-Koagulationstest aufweist, und wobei das Molekül ausgewählt ist aus der Gruppe, bestehend aus menschlichem oder nicht-menschlichem Säugerfaktor VIII, umfassend mindestens eine spezifische Sequenz, die kürzer ist als eine Domäne, beinhaltend eine oder mehrere einzelne Aminosäuren des Faktor VIII von einer Art, welche für die korrespondierende Sequenz des Faktor VIII der anderen Art substituiert worden ist oder sind, wobei die korrespondierende Sequenz mindestens eine Sequenz von Aminosäuren, entsprechend den Positionen 484-509 des humanen Faktors VIII, gezeigt in SEQ ID Nr. 2, einschließt, und wobei der Hybridfaktor VIII weniger immunreaktiv ist als humaner Faktor VIII mit den Inhibitionsantikörpern gegen die A2-Domäne von Faktor VIII.

2. Hybridfaktor VIII-Molekül nach Anspruch 1, wobei die korrespondierende Sequenz von einer oder mehreren Aminosäuren des Faktors VIII von einer Art, substituiert durch die spezifische Sequenz, welche ein oder mehrere einzelne Aminosäuren des Faktor VIII der anderen Art beinhaltet, der humanen Aminosäuresequenz, gezeigt in SEQ ID Nr. 2, ausgewählt aus der Gruppe, bestehend aus Aminosäuren 373-540, 373-509, 445-509 und 404-509, entspricht.

3. Hybridfaktor VIII-Molekül nach Anspruch 2, wobei das Molekül ein Human/Schwein-Hybridmolekül ist, die durch die spezifische Sequenz zu substituierende, korrespondierende Sequenz human ist und die spezifische Sequenz vom Schwein ist.

4. Hybridfaktor VIII-Molekül nach Anspruch 1, wobei der Hybridfaktor VIII zur Behandlung von humanen Patienten mit Antikörpern gegen Faktor VIII, welche die Koagulationsaktivität inhibieren, verwendbar ist.

5. Zusammensetzung, enthaltend ein Hybridfaktor VIII-Molekül nach Anspruch 1 in Kombination mit Gerinnungsfaktoren, die aus der Gruppe, bestehend aus Von-Willebrand-Faktor, Vitamin K-abhängigen Gerinnungsfaktoren und koagulierendem Gewebefaktor, ausgewählt sind.

6. Hybridfaktor VIII-Molekül nach Anspruch 1, wobei die nicht-humane Säugerdomäne, welche für die korrespondierende Humandomäne substituiert ist, die Schwein- oder Maus-A2-Domäne ist.

7. Hybridfaktor VIII-Molekül nach Anspruch 1, wobei die nicht-humane Säugersequenz, welche für die korrespondierende Humansequenz substituiert ist, von Schwein oder Maus ist und die spezifische nicht-humane Säugersequenz, welche eine oder mehrere einzelne Aminosäuren, substituiert für die korrespondierenden humanen Aminosäuren 484-509, beinhaltet, von Maus oder Schwein ist.

8. Hybridfaktor VIII-Molekül nach einem der Ansprüche 1 bis 7, wobei dem Molekül die B-Domäne fehlt.

9. Verfahren zur Herstellung eines Arzneimittels zur Behandlung von humanen Patienten mit Faktor VIII-Mangel, umfassend die Herstellung eines Hybridfaktor VIII-Moleküls, das wie in einem der Ansprüche 1 bis 7 definiert ist.

10. Verfahren zur Herstellung eines gereinigten prokoagulierenden Hybridfaktor VIII, wobei der Hybridfaktor VIII Schwein- oder Maus- und humane Aminosäuresequenzen umfaßt, welches das Substituieren von mindestens einer spezifischen Sequenz, welche den Positionen 484-509 des humanen Faktor VIII, gezeigt in SEQ ID Nr. 2, entspricht, für eine korrespondierende Sequenz von Aminosäuren des Faktors VIII der anderen Art durch gezielte Mutagenese der kodierenden Nukleinsäure umfaßt.

11. Verfahren nach Anspruch 10 zur Herstellung der Hybridfaktor VIII-Moleküle der Ansprüche 1, 2 und 7.

12. Verfahren nach Anspruch 10, wobei die korrespondierende Sequenz von einer oder mehreren Aminosäuren des Faktors VIII von einer Art, welche durch die spezifische Sequenz, beinhaltend eine oder mehrere einzelne Aminosäuren des Faktor VIII der anderen Art substituiert sind, der humanen Aminosäuresequenz, gezeigt in SEQ ID Nr. 2, ausgewählt aus der Gruppe, bestehend aus Aminosäuren 373-540, 373-509, 445-509 und 404-509, entspricht.

13. Verfahren nach Anspruch 10, wobei die spezifische Sequenz, welche eine oder mehrere einzelne Aminosäuren des Faktors VIII der einen Art, welche für die korrespondierende Sequenz von einer oder mehreren Aminosäuren des Faktor VIII der anderen Art substituiert ist oder sind, beinhaltet, eine Koagulationsaktivitätsdeterminante einschließt.

14. Verfahren nach Anspruch 13, wobei der Hybridfaktor VIII eine größere Koagulationsaktivität aufweist als der von humanem Faktor VIII, wenn humanes Plasma als Standard in einem einstufigen *in vitro*-Koagulationstest verwendet wird.

15. Verfahren nach Anspruch 10, wobei die korrespondierende Sequenz von einer oder mehreren Aminosäuren des Faktors VIII von einer Art, welche durch die spezifische Sequenz, beinhaltend eine oder mehrere einzelne Aminosäuren des Faktors VIII der anderen Spezies, zu substituieren ist oder sind, eine antigene Stelle einschließt, die mit Antikörpern gegen Faktor VIII, welche die Koagulationsaktivität inhibieren, reagiert, und wobei der Hybridfaktor VIII weniger immunreaktiv ist als der humane Faktor VIII mit den Inhibitionsantikörpern gegen Faktor VIII.

16. Verfahren nach einem der Ansprüche 10 bis 15, weiter umfassend den Schritt des Deletierens der B-Domäne zur Herstellung eines Hybridfaktors VIII ohne B-Domäne.

17. Verfahren zur Herstellung von gereinigtem Hybridfaktor VIII gemäß Anspruch 1, wobei der Hybridfaktor VIII Schwein- und humane Aminosäuresequenzen umfaßt, welches die Schritte
Exprimieren von rekombinanter DNA, welche für Domänen, ausgewählt aus der Gruppe, bestehend aus A1, B, A3, C1 und C2-Domänen von Schwein- und humanem Faktor VIII, kodiert, und weiter
Substituieren von einer oder mehreren Domänen des Schwein- und humanen Faktors VIII umfaßt.

18. Verfahren nach Anspruch 17, weiter umfassend den Schritt des Deletierens der B-Domäne zur Herstellung eines Hybridfaktors VIII ohne B-Domäne.

19. Gereinigtes Hybridfaktor VIII-äquivalentes Molekül, umfassend humanen, nicht-humanen Säuger- oder hybriden humanen/nicht-humanen Säugerfaktor VIII, wobei das Molekül Prokoagulationsaktivität in einem *in vitro*-Koagulationstest aufweist, umfassend mindestens eine Sequenz ohne bekannte Sequenzidentität zu Faktor VIII, substituiert für eine spezifische Aminosäuresequenz, korrespondierend zu den Positionen 484-509 des humanen Faktor VIII, gezeigt in SEQ ID Nr. 2, in humanem, nicht-humanem Säuger- oder hybridem humanem/nicht-humanem Säugerfaktor VIII.

20. Äquivalentes Molekül nach Anspruch 19 mit einer größeren Prokoagulationsaktivität als humaner Faktor VIII in einem einstufigen *in vitro*-Koagulationstest unter Verwendung von humanem Plasma als Standard.

21. Äquivalentes Molekül nach Anspruch 19, wobei die spezifische Aminosäuresequenz in humanem, nicht-humanem Säuger- oder hybridem humanem/nicht-humanem Säugerfaktor VIII ein Epitop mit Immunreaktivität mit Antikörpern umfaßt, welche die Koagulationsaktivität von Faktor VIII inhibieren, und wobei der äquivalente Hybridfaktor VIII eine verringerte Immunreaktivität gegen die Antikörper aufweist.

22. Äquivalentes Molekül nach Anspruch 19 oder 21, wobei die Aminosäuresequenz ohne bekannte Sequenzidentität zu Faktor VIII einen oder mehrere Alaninreste umfaßt.

23. Äquivalentes Molekül nach einem der Ansprüche 19 bis 22, welchem die B-Domäne fehlt.

24. Verfahren zur Herstellung eines äquivalenten Hybridfaktor VIII-Moleküls, umfassend humanen, nicht-humanen Säuger- oder hybriden humanen/nichthumanen Säugerfaktor VIII, wobei das äquivalente Molekül eine Prokoagulationsaktivität in einem *in vitro* Koagulationstest aufweist, umfassend das Substituieren von mindestens einer Sequenz ohne bekannte Sequenzidentität zu Faktor VIII für eine spezifische Aminosäuresequenz, korrespondierend zu den Positionen 484-509 des humanen Faktors VIII, gezeigt in SEQ ID Nr. 2, in humanem, nicht-humanem Säuger- oder hybridem humanem/nicht-humanem Säugerfaktor VIII.

25. Verfahren nach Anspruch 24, wobei die Aminosäuresequenz ohne bekannte Sequenzidentität zu Faktor VIII einen oder mehrere Alaninreste umfaßt.

26. Verfahren nach Anspruch 24, weiter umfassend das Substituieren von mindestens einer spezifischen Sequenz von einer oder mehreren einzelnen Aminosäuren, welche eine Koagulationsaktivitätdeterminante des Faktors VIII von einer Art beinhaltet, für eine korrespondierende Sequenz von einer oder mehreren Aminosäuren des Faktors VIII der anderen Art, oder für eine korrespondierende Sequenz von einer oder mehreren Aminosäuren des Hybridfaktors VIII, wobei das äquivalente Hybridfaktor VIII-Molekül eine größere Koagulationsaktivität aufweist als humaner Faktor VIII in einem einstufigen Koagulationstest, unter Verwendung von humanem Plasma als Standard.

27. Verfahren nach Anspruch 24, wobei die spezifische Aminosäuresequenz in humanem, nicht-humanem Säuger- oder hybridem humanem/nichthumanem Säugerfaktor VIII ein Epitop mit Immunreaktivität gegen Antikörper umfaßt, welche die Koagulationsaktivität des Faktors VIII inhibieren, und wobei der äquivalente Hybridfaktor VIII eine verringerte Immunreaktivität gegen diese Antikörper aufweist.

28. Verfahren nach einem der Ansprüche 24 bis 27, weiter umfassend das Deletieren der B-Domäne.

29. Fusionsprotein, umfassend das Hybridfaktor VIII-Molekül nach Anspruch 1 oder 19.

30. Isolierte Nukleinsäuresequenz, welche für ein humanes/nicht-humanes Säuger-Hybridfaktor VIII-Molekül gemäß Anspruch 1 kodiert, wobei der Hybridfaktor VIII weniger immunreaktiv ist als humaner Faktor VIII mit Inhibitionsantikörper gegen Faktor VIII.

31. Isolierte Nukleinsäuresequenz nach Anspruch 30, wobei das Molekül eine größere spezifische Aktivität aufweist als diejenige von humanem Faktor VIII, wenn humanes Plasma als Standard in einem einstufigen Koagulationstest verwendet wird.

32. Isolierte Nukleinsäuresequenz nach Anspruch 30, wobei die korrespondierende Sequenz von Aminosäuren des Faktors VIII von einer Art, substituiert durch die spezifische Sequenz, welche einzelne Aminosäuren des Faktors VIII der anderen Art beinhaltet, der humanen Aminosäuresequenz, gezeigt in SEQ ID Nr. 2, ausgewählt aus der Gruppe, bestehend aus Aminosäuren 373-540, 373-509 und 445-509, entspricht.

33. Isolierte Nukleinsäuresequenz nach Anspruch 30, wobei der Hybridfaktor VIII zur Behandlung von humanen Patienten mit Antikörpern gegen Faktor VIII, welche die Koagulationsaktivität inhibieren, verwendbar ist.

34. Isolierte Nukleinsäuresequenz nach einem der Ansprüche 30 bis 33, wobei dem Molekül die B-Domäne fehlt.

35. Isolierte Nukleinsäuresequenz, welche für ein äquivalentes Hybridfaktor VIII-Molekül gemäß Anspruch 19 kodiert.

36. Isolierte Nukleinsäuresequenz nach Anspruch 35, wobei das äquivalente Hybridfaktor VIII-Molekül eine größere Prokoagulationsaktivität aufweist als humaner Faktor VIII in einem einstufigen *in vitro*-Koagulationstest unter Verwendung von humanem Plasma als Standard.

37. Isolierte Nukleinsäuresequenz nach Anspruch 35, wobei die spezifische Aminosäuresequenz in humanem, nicht-humanem Säuger- oder hybridem humanem/nicht-humanem Säugerfaktor VIII ein Epitop mit Immunreaktivität gegen Antikörper umfaßt, welche die Koagulationsaktivität von Faktor VIII inhibieren, und wobei der äquivalente Hybridfaktor VIII eine verringerte Immunreaktivität gegen die Antikörper aufweist.

38. Isolierte Nukleinsäuresequenz nach Anspruch 35, wobei die Aminosäuresequenz ohne bekannte Sequenzidentität zu Faktor VIII einen oder mehrere Alaninreste umfaßt.

39. Isolierte Nukleinsäuresequenz nach einem der Ansprüche 35 bis 38, wobei dem Molekül die B-Domäne fehlt.

40. Hybridfaktor VIII-Molekül nach einem der Ansprüche 1 bis 8, 19 bis 23 oder 29 zur Verwendung in der Medizin.

41. Verwendung eines Moleküls nach einem der Ansprüche 1 bis 8, 19 bis 23 oder 29 zur Herstellung eines Arzneimittels für die Verwendung zur Behandlung von humanem Faktor VIII-Mangel.

42. Verwendung nach Anspruch 41, wobei die Gerinnungsaktivität des hybriden humanen/nicht-humanen Säuger- oder äquivalenten Hybridmoleküls eine verringerte Immunreaktivität gegen Antikörper aufweist, welche die Faktor VIII-Koagulationsaktivität inhibieren.

43. Verwendung nach Anspruch 42, wobei die Patienten durch Vorhandensein von Antikörpern gegen Faktor VIII gekennzeichnet sind.

## Revendications

1. Molécule de facteur VIII hybride purifiée comprenant des séquences d'acides aminés humaines et de mammifère non humain, où la molécule possède une activité procoagulante dans un essai de coagulation in vitro et où la molécule est choisie dans le groupe constitué par un facteur VIII humain ou de mammifère non humain comprenant au moins une séquence spécifique plus courte qu'un domaine, dans laquelle un ou plusieurs acides aminés uniques du facteur VIII d'une espèce remplacent la séquence correspondante du facteur VIII de l'autre espèce, où la séquence correspondante inclut au moins une séquence d'acides aminés correspondant aux positions 484-509 du facteur VIII humain comme indiqué dans SEQ ID N°:2, et où le facteur VIII hybride est moins immunoréactif que le facteur VIII humain avec les anticorps inhibiteurs dirigés contre le domaine A2 du facteur VIII.

2. Molécule de facteur VIII hybride selon la revendication 1, dans laquelle la séquence correspondante d'un ou de plusieurs acides aminés du facteur VIII d'une espèce, substituée par la séquence spécifique comportant un ou plusieurs acides aminés uniques du facteur VIII de l'autre espèce, correspond à une séquence d'acides aminés humaine présentée dans SEQ ID N°:2, choisie dans le groupe constitué par les acides aminés 373-540, 373-509, 445-509 et 404-509.

3. Molécule de facteur VIII hybride selon la revendication 2, dans laquelle la molécule est une molécule hybride humaine/porcine, la séquence correspondante à substituer par la séquence spécifique est une séquence humaine, et la séquence spécifique est une séquence porcine.

4. Molécule de facteur VIII hybride selon la revendication 1, dans laquelle le facteur VIII hybride est utile pour le traitement de patients humains ayant des anticorps dirigés contre le facteur VIII, qui inhibent l'activité de coagulation.

5. Composition contenant une molécule de facteur VIII hybride selon la revendication 1, en combinaison avec des facteurs de coagulation choisis dans le groupe constitué par le facteur de von Willebrand, les facteurs de coagulation dépendants de la vitamine K, et le facteur coagulant tissulaire.

6. Molécule de facteur VIII hybride selon la revendication 1, dans laquelle le domaine de mammifère non humain qui remplace le domaine humain correspondant est le domaine A2 porcin ou murin.

7. Molécule de facteur VIII hybride selon la revendication 1, dans laquelle la séquence de mammifère non humain qui remplace la séquence humaine correspondante est une séquence porcine ou murine, et la séquence spécifique de mammifère non humain comportant un ou plusieurs acides aminés uniques remplaçant les acides aminés humains 484-509 correspondants est une séquence murine ou porcine.

8. Molécule de facteur VIII hybride selon l'une quelconque des revendications 1-7, dans laquelle la molécule est dépourvue du domaine B.

9. Procédé de fabrication d'un médicament destiné au traitement de patients humains présentant une carence en facteur VIII, comprenant la préparation d'une molécule de facteur VIII hybride telle que définie selon l'une quelconque des revendications 1-7.

10. Procédé de préparation d'un facteur VIII hybride procoagulant purifié, dans lequel le facteur VIII hybride comprend des séquences d'acides aminés porcines ou murines et humaines, comprenant le remplacement par au moins une séquence spécifique correspondant aux positions 484-509 du facteur VIII humain, comme indiqué dans SEQ ID N°2, d'une séquence correspondante d'acides aminés du facteur VIII de l'autre espèce par mutagenèse dirigée de l'acide nucléique codant.

11. Procédé selon la revendication 10 pour préparer les molécules de facteur VIII hybrides des revendications 1, 2 et 7.

12. Procédé selon la revendication 10, dans lequel la séquence correspondante d'un ou de plusieurs acides aminés du facteur VIII d'une espèce, substituée par la séquence spécifique comportant un ou plusieurs acides aminés uniques du facteur VIII de l'autre espèce, correspond à une séquence d'acides aminés humaine présentée dans SEQ ID N°:2, choisie dans le groupe constitué par les acides aminés 373-540, 373-509, 445-509 et 404-509.

13. Procédé selon la revendication 10, dans lequel la séquence spécifique comportant un ou plusieurs acides aminés uniques du facteur VIII d'une espèce, qui remplace la séquence correspondante d'un ou de plusieurs acides aminés du facteur VIII de l'autre espèce, comprend un déterminant d'activité coagulante.

14. Procédé selon la revendication 13, dans lequel le facteur VIII hybride possède une activité coagulante supérieure à celle du facteur VIII humain lorsque du plasma humain est utilisé comme étalon dans un essai de coagulation en une étape in vitro.

15. Procédé selon la revendication 10, dans lequel la séquence correspondante d'un ou de plusieurs acides aminés du facteur VIII d'une espèce à substituer par la séquence spécifique comportant un ou plusieurs acides aminés uniques du facteur VIII de l'autre espèce comprend un site antigénique qui réagit avec des anticorps dirigés contre le facteur VIII, qui inhibent l'activité coagulante, et dans lequel le facteur VIII hybride est moins immunoréactif que le facteur VIII humain avec les anticorps inhibiteurs anti-facteur VIII.

16. Procédé selon l'une quelconque des revendications 10-15, comprenant en outre l'étape de délétion du domaine B pour produire un facteur VIII hybride sans domaine B.

17. Procédé de préparation d'un facteur VIII hybride purifié selon la revendication 1, dans lequel le facteur VIII hybride comprend des séquences d'acides aminés porcines et humaines, ledit procédé comprenant les étapes consistant à
exprimer un ADN recombinant codant pour des domaines choisis dans le groupe constitué par les domaines A1, B, A3, C1 et C2 du facteur VIII porcin et humain,
comprenant en outre la substitution d'un ou de plusieurs domaines du facteur VIII porcin et humain.

18. Procédé selon la revendication 17, comprenant en outre l'étape de délétion du domaine B pour produire un facteur VIII hybride sans domaine B.

19. Molécule équivalente de facteur VIII hybride purifiée comprenant un facteur VIII humain, de mammifère non humain, ou hybride humain/de mammifère non humain, dans laquelle la molécule possède une activité procoagulante dans un essai de coagulation in vitro, comprenant au moins une séquence n'ayant pas d'identité de séquence connue par rapport au facteur VIII, qui remplace une séquence d'acides aminés spécifique correspondant aux positions 484-509 du facteur VIII humain, comme indiqué dans SEQ ID N°:2 dans un facteur VIII humain, de mammifère non humain, ou hybride humain/de mammifère non humain.

20. Molécule équivalente selon la revendication 19, ayant une activité procoagulante supérieure à celle du facteur VIII humain dans un essai de coagulation en une étape in vitro, utilisant du plasma humain comme étalon.

21. Molécule équivalente selon la revendication 19, dans laquelle la séquence d'acides aminés spécifique dans le facteur VIII humain, de mammifère non humain, ou hybride humain/de mammifère non humain comprend un épitope présentant une immunoréactivité avec des anticorps qui inhibent l'activité coagulante du facteur VIII, et dans laquelle le facteur VIII équivalent hybride présente une immunoréactivité réduite vis-à-vis des anticorps.

22. Molécule équivalente selon la revendication 19 ou 21, dans laquelle la séquence d'acides aminés n'ayant pas d'identité de séquence connue par rapport au facteur VIII comprend un ou plusieurs résidus alanine.

23. Molécule équivalente selon l'une quelconque des revendications 19 à 22 qui est dépourvue du domaine B.

24. Procédé de préparation d'une molécule équivalente de facteur VIII hybride comprenant un facteur VIII humain, de mammifère non humain, ou hybride humain/de mammifère non humain, dans lequel la molécule équivalente possède une activité procoagulante dans un essai de coagulation in vitro, comprenant
le remplacement, par au moins une séquence n'ayant pas d'identité de séquence connue par rapport au facteur VIII, d'une séquence d'acides aminés spécifique correspondant aux positions 484-509 du facteur VIII humain, comme indiqué dans SEQ ID N°:2 dans le facteur VIII humain, de mammifère non humain, ou hybride humain/de mammifère non humain.

25. Procédé selon la revendication 24, dans lequel la séquence d'acides aminés n'ayant pas d'identité de séquence connue par rapport au facteur VIII comprend un ou plusieurs résidus alanine.

26. Procédé selon la revendication 24, comprenant en outre le remplacement, par au moins une séquence spécifique d'un ou de plusieurs acides aminés uniques comprenant un déterminant d'activité coagulante du facteur VIII d'une espèce, d'une séquence correspondante d'un ou de plusieurs acides aminés du facteur VIII d'une autre espèce, ou d'une séquence correspondante d'un ou de plusieurs acides aminés du facteur VIII hybride, dans lequel la molécule équivalente de facteur VIII hybride possède une activité coagulante supérieure à celle du facteur VIII humain dans un essai de coagulation en une étape utilisant du plasma humain comme étalon.

27. Procédé selon la revendication 24, dans lequel la séquence d'acides aminés spécifique dans le facteur VIII humain, de mammifère non humain, ou hybride humain/de mammifère non humain comprend un épitope présentant une immunoréactivité avec des anticorps qui inhibent l'activité coagulante du facteur VIII, et dans lequel le facteur VIII équivalent hybride présente une immunoréactivité réduite vis-à-vis des anticorps.

28. Procédé selon l'une quelconque des revendications 24-27, comprenant en outre la délétion du domaine B.

29. Protéine de fusion comprenant la molécule de facteur VIII hybride de la revendication 1 ou 19.

30. Séquence d'acides nucléiques isolée, codant pour une molécule de facteur VIII hybride humaine/de mammifère non humain selon la revendication 1, dans laquelle le facteur VIII hybride est moins immunoréactif que le facteur VIII humain avec des anticorps inhibiteurs anti-facteur VIII.

31. Séquence d'acides nucléiques isolée selon la revendication 30, dans laquelle la molécule possède une activité spécifique supérieure à celle du facteur VIII humain lorsqu'on utilise du plasma humain comme étalon dans un essai de coagulation en une étape.

32. Séquence d'acides nucléiques isolée selon la revendication 30, dans laquelle la séquence correspondante d'acides aminés du facteur VIII d'une espèce, substituée par la séquence spécifique comportant des acides aminés uniques du facteur VIII de l'autre espèce, correspond à une séquence d'acides aminés humaine comme indiqué dans SEQ ID N°:2, choisie dans le groupe constitué par les acides aminés 373-540, 373-509 et 445-509.

33. Séquence d'acides nucléiques isolée selon la revendication 30, dans laquelle le facteur VIII hybride est utile pour le traitement de patients humains ayant des anticorps dirigés contre le facteur VIII qui inhibent l'activité de coagulation.

34. Séquence d'acides nucléiques isolée selon l'une quelconque des revendications 30-33, dans laquelle la molécule est dépourvue du domaine B.

35. Séquence d'acides nucléiques isolée codant pour une molécule équivalente de facteur VIII hybride selon la revendication 19.

36. Séquence d'acides nucléiques isolée selon la revendication 35, ladite molécule équivalente de facteur VIII hybride ayant une activité procoagulante supérieure à celle du facteur VIII humain dans un essai de coagulation en une étape in vitro utilisant du plasma humain comme étalon.

37. Séquence d'acides nucléiques isolée selon la revendication 35, dans laquelle la séquence d'acides aminés spécifique dans le facteur VIII humain, de mammifère non humain, ou hybride humain/de mammifère non humain comprend un épitope ayant une immunoréactivité avec des anticorps qui inhibent l'activé coagulante du facteur VIII, et dans laquelle le facteur VIII équivalent hybride présente une immunoréactivité réduite vis-à-vis des anticorps.

38. Séquence d'acides nucléiques isolée selon la revendication 35, dans laquelle la séquence d'acides aminés n'ayant pas d'identité de séquence connue par rapport au facteur VIII comprend un ou plusieurs résidus alanine.

39. Séquence d'acides nucléiques isolée selon l'une quelconque des revendications 35-38, dans laquelle la molécule est dépourvue du domaine B.

40. Molécule de facteur VIII hybride selon l'une quelconque des revendications 1-8, 19-23 ou 29, à utiliser en médecine.

41. Utilisation d'une molécule selon l'une quelconque des revendications 1-8, 19-23 ou 29 dans la fabrication d'un médicament à utiliser dans le traitement d'une carence en facteur VIII humain.

42. Utilisation selon la revendication 41, dans laquelle l'activité de coagulation de la molécule hybride humaine/de mammifère non humain ou de la molécule équivalente hybride présente une immunoréactivité réduite avec des anticorps qui inhibent l'activité coagulante du facteur VIII.

43. Utilisation selon la revendication 42, dans laquelle les patients sont caractérisés par la présence d'anticorps anti-facteur VIII.
